(19) Europäisches Patentamt / European Patent Office / Office européen des brevets

(11) **EP 4 053 109 A1**

(12) **EUROPEAN PATENT APPLICATION**
published in accordance with Art. 153(4) EPC

(43) Date of publication:
**07.09.2022 Bulletin 2022/36**

(21) Application number: **20881503.5**

(22) Date of filing: **30.10.2020**

(51) International Patent Classification (IPC):
***C07D 235/04*** *(2006.01)* ***A61K 31/4184*** *(2006.01)*

(52) Cooperative Patent Classification (CPC):
**A61K 31/4184; C07D 235/04**

(86) International application number:
**PCT/CN2020/125125**

(87) International publication number:
**WO 2021/083311 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.10.2019 CN 201911049930**

(71) Applicants:
- **Jiangsu Hengrui Medicine Co., Ltd.**
  **Lianyungang, Jiangsu 222047 (CN)**
- **Shanghai Hengrui Pharmaceutical Co., Ltd**
  **Shanghai 200245 (CN)**

(72) Inventors:
- **ZHOU, Xianqiang**
  **Lianyungang, Jiangsu 222047 (CN)**
- **DU, Zhenxing**
  **Lianyungang, Jiangsu 222047 (CN)**
- **WANG, Jie**
  **Lianyungang, Jiangsu 222047 (CN)**
- **WANG, Lin**
  **Lianyungang, Jiangsu 222047 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) ACID ADDITION SALT OF ROR? REGULATOR

(57) An acid addition salt of a RORγ regulator. Specifically relating to the acid addition salt of the compound of formula II. More specifically relating to benzoate, oxalate, methanesulfonate, maleate, hydrobromate, hydrochloride salt, and acetate of the compound of formula II and the benzoate crystal form, benzoate amorphous form, oxalate crystal form, oxalate amorphous form, methanesulfonate amorphous form, maleate B crystal form, maleate C crystal form, maleate D crystal form, hydrobromate I crystal form, hydrochloride salt α crystal form, hydrochloride salt β crystal form, hydrochloride salt γ crystal form, and acetate crystal form of the compound of formula II.

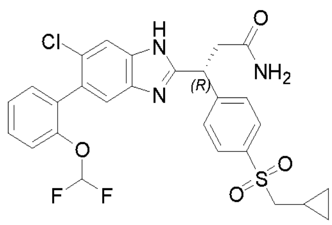

formula II

## Description

The present application claims priority to Chinese Patent Application No. CN201911049930.5 filed on Oct. 31, 2019, which is incorporated herein by reference in its entirety.

## TECHNICAL FIELD

The present disclosure relates to the field of pharmaceutical chemistry, and in particular, to an acid addition salt of a compound of formula II as an RORγ regulator.

## BACKGROUND

Nuclear receptors are ligand-regulated transcription factors that regulate development, immunity, and cellular metabolism, and are one of the major therapeutic target classes for human diseases. Retinoid-related orphan receptor gamma (RORγ) is a member of the nuclear receptor NR1 subfamily and has a typical nuclear receptor domain structure consisting of a DNA-binding domain, a ligand-binding domain, a hinge domain and an activation function 2 domain (Benoit G, et al., Pharmacological Reviews, 58(4):798-836, 2006; Zhang, Y, et al., Acta Pharmacogica Sinica, 36:71-87, 2015). In contrast to most other nuclear receptors that function as dimers, RORγ works as a monomer. It binds to specific DNA sequences usually consisting of TAAA/TNTAGGTCA, which are called ROR response elements (ROREs).

There are two RORγ subtypes, RORγ1 and RORy2 (also known as RORyt) that arise from the same RORC gene, and possibly differ in selection of promoters (Villey I et al, Eur. J. Immunol., 29(12):4072-80, 1999). Since the two RORγ subtypes (RORγ1 and RORyt) are derived from the same mRNA, they have identical ligand-binding domains, and are distinguished by N terminus only (Jetten, A. M., 2009; Ivanov, I. I. et al., 2006). Small molecule inhibitors generally bind to the ligand-binding domain to inhibit the function of the receptor. As such, they have no selectivity for the two RORγ subtypes, and are all referred to as RORγ small molecule inhibitors (or modulators) without subtyping.

The two RORγ subtypes have very different tissue distributions. RORyt is mainly expressed in thymus and several immune cells, while RORγ1 is expressed in many tissues, such as thymus, liver, muscle, testis, pancreas, prostate and heart. (Jetten, A. M., 2009; Zhang, Y. et al., 2015). It has been reported that one of the functions of RORγ1 is to regulate the human biochronometer, and particularly, to regulate the circadian rhythm (Jetten, A. M., 2009). T helper 17 (Th17) cells are the major source of autoimmune disease (Ivanov, I. I. et al., 2006). Both RORγ subtypes are expressed in Th17 cells, regulating T cell differentiation and inducing gene transcription in Th17 cells (Ruan, Q., et al., 2011). Cytokines IL-6 and TGF-β induce differentiation of undifferentiated CD4 T helper cells into Th17 cells. RORyt highly expressed in Th17 cells induces transcription of IL-23 receptor gene in undifferentiated CD4 T helper cells, IL23 receptors in turn promote and stabilize production of Th17 cells, forming part of a positive feedback loop (Ivanov, I. I. et al., 2006; Jetten, A. M., 2009). Meanwhile, RORyt can induce the gene transcription of proinflammatory cytokines such as IL-17A, IL-17F, IL-21 and IL-22, and enhance the inflammation process. Like RORyt, RORγ1 is also expressed in Th17 cells, and can also regulate differentiation and induce gene transcription in Th17 cells (Ruan, Q., et al., 2011). Pharmacological antagonism against RORγ has therapeutic potential for autoimmune diseases, making it an attractive target for small molecule inhibitors.

RORγ has been identified as a key mediator in the pathogenesis of several diseases, such as rheumatoid arthritis, psoriasis vulgaris, multiple sclerosis, inflammatory bowel disease, Crohn's disease, sicca syndrome and asthma. (Louten et al., J. Allergy Clin. Immunol., 123:1004-1011, 2009; Annuziato, F., et al., Nat. Rev. Rheumatol., 5(6):325-331, 2009; Lizuka, M., et al., J. Immunol., 194:56-67, 2014). Some other diseases, such as chronic xerophthalmia, Kawasaki's disease, mucosal leishmaniasis and Hashimoto's thyroiditis, are characterized by increased Th17 proportion and/or increased levels of Th17 marker cytokines, such as IL-17, IL-22 and IL-23. (Chen, Y. et al., Mucosal. Immunol., 7(1):38-45, 2014; Jia, S., et al., Clin. Exp. Immunol., 162:131-137, 2010; Boaventura, VS et al., Eur. J. Immunol., 40:2830-2836, 2010; Figueroa-Vega, N. et al., J. Clin. Endocrinol. Metab., 95:953-62, 2010). In each of the above examples, the inhibitory effect can be enhanced by inhibiting RORα. RORγt inhibitors are currently being developed for the treatment of autoimmune diseases such as psoriasis vulgaris and rheumatoid arthritis. See Jun R. Huh and Dan R. Littman, Eur. J. Immunol., 42(9):2232-2237 (2012), WO2012/027965, WO2013/029338 and US2015/291607.

Patent Application No. PCT/US19/30526 provides an RORγ regulator having a structure of formula II:

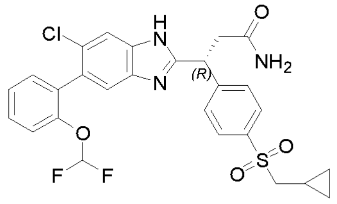

formula II

which is incorporated herein in its entirety.

## BRIEF SUMMARY

The present disclosure provides an acid addition salt of a compound of formula II or a pharmaceutically acceptable solvate of the acid addition salt, wherein the acid addition salt is an organic acid addition salt or an inorganic acid addition salt.

The present disclosure further relates to a method for preparing an acid addition salt of a compound of formula II or a pharmaceutically acceptable solvate of the acid addition salt, wherein the acid addition salt is an organic acid addition salt or an inorganic acid addition salt, and the method comprises mixing a certain amount of the compound of formula II with a proper amount of a solvent and the organic acid or the inorganic acid, and reacting for a period of time to obtain the salt of the compound of formula II with the corresponding acid, wherein the solvent is selected from the group consisting of one or more of a hydrocarbon solvent, an ether solvent, an alcohol solvent, an ester solvent, a ketone solvent, a nitrile solvent, a halogenated hydrocarbon solvent, a nitrogenous solvent, water and dimethyl sulfoxide.

In some embodiments, the organic acid addition salt is at least one selected from the group consisting of formate, acetate, propionate, butyrate, benzoate, malonate, succinate, pyruvate, methanesulfonate, ethanesulfonate, propanesulfonate, citrate, 4-nitrobenzoate, benzenesulfonate, *p*-toluenesulfonate, 1,2-ethanedisulfonate, β-naphthalenesulfonate, malate, propiolate, 2-butynoate, 2-hydroxy-ethanesulfonate, 3-butenoate, tartrate, fumarate, isethionate, maleate, lactate, lactobionate, pamoate, salicylate, galactarate, glucoheptonate, mandelate, 1,2-ethanedisulfonate, oxalate, trifluoroacetate, trifluoromethanesulfonate, adipate, suberate, sebacate, butyne-1,4-dioate, hexyne-1,6-dioate, glycolate, alginate, ascorbate, aspartate, glutamate, 2-phenoxybenzoate, 2-(4-hydroxybenzoyl)benzoate, acetoacetate, 2-hydroxyethanesulfonate, borate, chlorobenzoate, camphorate, itaconate, camphorsulfonate, methylbenzoate, dinitrobenzoate, sulfamate, galacturonate, cyclopentylpropionate, dodecylsulfate, acrylate, cyclopentanepropionate, glycerophosphate, methoxybenzoate, digluconate, gluconate, heptanoate, hexanoate, pivalate, glucuronate, laurate, phthalate, phenylacetate, laurylsulfate, 2-acetoxybenzoate, nicotinate, cinnamate, oleate, palmitate, pectate, *p*-phthalate, glutarate, hydroxymaleate, hydroxybenzoate, phenylacetate, 3-hydroxy-2-naphthoate, 3-phenylpropionate, isobutyrate, neopentanoate, picrate, stearate, 2,2-dichloroacetate, acylated amino acid salt, alginate, 4-acetamidobenzenesulfonate, caprate, cholate, caprylate, nonanoate, cyclamate, phthalate, cysteine hydrochloride salt, sorbate, glycine hydrochloride salt, 1,5-naphthalenedisulfonate, xylenesulfonate, cystine dihydrochloride salt, undecanoate, polyvinyl sulfonate salt, sulfosalicylate, phenylbutyrate, 4-hydroxybutyrate, polyvinyl sulfate salt, naphthalene-1-sulphonate and valerate.

In some embodiments, the inorganic acid addition salt is at least one selected from the group consisting of hydrochloride, sulfate, bisulfate, nitrate, hydrobromide, hydroiodide, carbonate, bicarbonate, sulfite, bisulfite, pyrosulfate, monohydrogen phosphate, dihydrogen phosphate, perchlorate, persulfate, hemisulfate, disulfate, thiocyanate, phosphate, pyrophosphate and metaphosphate.

In some embodiments, the organic acid addition salt may be at least one selected from the group consisting of benzoate, oxalate, methanesulfonate, maleate and acetate, and the inorganic acid addition salt may be selected from the group consisting of hydrochloride and hydrobromide. The present disclosure provides a crystalline form of benzoate, an amorphous form of benzoate, a crystalline form of oxalate, an amorphous form of oxalate, an amorphous form of methanesulfonate, a crystalline form B of maleate, a crystalline form C of maleate, a crystalline form D of maleate, a crystalline form I of hydrobromide, a crystalline form α of hydrochloride, a crystalline form β of hydrochloride, a crystalline form γ of hydrochloride and a crystalline form of acetate of a compound of formula II, and methods for preparing the same.

The present disclosure provides an amorphous form of a compound of formula II, having an XRPD pattern with no distinct sharp diffraction peaks; preferably, the amorphous form has an XRPD pattern as shown in FIG. 1.

The present disclosure provides a benzoate of a compound of formula II.

In some embodiments, the benzoate is an amorphous form having an XRPD pattern with no distinct sharp diffraction peaks; preferably, the amorphous form has an XRPD pattern as shown in FIG. 2.

In some embodiments, the benzoate is a crystalline form having an X-ray powder diffraction pattern with characteristic peaks at diffraction angles 2θ of 5.305 and 7.411.

Furthermore, the crystalline form of the benzoate has an X-ray powder diffraction pattern with characteristic

peaks at diffraction angles 2θ of 5.305, 7.411 and 22.031.

Furthermore, the crystalline form of the benzoate has an X-ray powder diffraction pattern with characteristic peaks at diffraction angles 2θ of 5.305, 7.411, 19.140 and 22.0314.

The present disclosure provides an oxalate of a compound of formula II.

In some embodiments, the oxalate is an amorphous form having an XRPD pattern with no distinct sharp diffraction peaks; preferably, the amorphous form has an XRPD pattern as shown in FIG. 5.

In some embodiments, the oxalate is a crystalline form having an X-ray powder diffraction pattern with characteristic peaks at diffraction angles 2θ of 14.378, 18.463 and 21.670. Furthermore, the crystalline form of the oxalate has an X-ray powder diffraction pattern with characteristic peaks at diffraction angles 2θ of 14.378, 18.463, 21.670 and 23.075. Furthermore, the crystalline form of the oxalate has an X-ray powder diffraction pattern with characteristic peaks at diffraction angles 2θ of 14.378, 18.463, 21.670, 23.075 and 28.127. The present disclosure provides a methanesulfonate of a compound of formula II.

In some embodiments, the methanesulfonate is an amorphous form having an XRPD pattern with no distinct sharp diffraction peaks; preferably, the amorphous form has an XRPD pattern as shown in FIG. 7.

The present disclosure provides a maleate of a compound of formula II.

In some embodiments, the maleate is a crystalline form B having an X-ray powder diffraction pattern with characteristic peaks at diffraction angles 2θ of 7.624, 9.659, 13.815, 15.844 and 17.391.

Furthermore, the crystalline form B of the maleate has an X-ray powder diffraction pattern with characteristic peaks at diffraction angles 2θ of 7.624, 9.659, 13.815, 15.844, 17.391 and 21.802. Furthermore, the crystalline form B of the maleate has an X-ray powder diffraction pattern with characteristic peaks at diffraction angles 2θ of 7.624, 9.659, 13.815, 15.844, 17.391, 18.619 and 21.802.

Furthermore, the crystalline form B of the maleate has an X-ray powder diffraction pattern with characteristic peaks at diffraction angles 2θ of 7.624, 9.659, 13.815, 15.844, 17.391, 18.619, 21.802, 23.667 and 26.441.

In some embodiments, the maleate is a crystalline form C having an X-ray powder diffraction pattern with characteristic peaks at diffraction angles 2θ of 7.325, 8.635, 9.809, 13.649, 16.133, 16.765 and 18.346.

Furthermore, the crystalline form C of the maleate has an X-ray powder diffraction pattern with characteristic peaks at diffraction angles 2θ of 7.325, 8.635, 9.809, 13.649, 16.133, 16.765, 18.346, 21.689 and 23.586.

Furthermore, the crystalline form C of the maleate has an X-ray powder diffraction pattern with characteristic peaks at diffraction angles 2θ of 7.325, 8.635, 9.809, 11.661, 13.649, 16.133, 16.765, 18.346, 21.689, 23.586 and 25.303.

In some embodiments, the maleate is a crystalline form D having an X-ray powder diffraction pattern with characteristic peaks at diffraction angles 2θ of 4.486, 7.288, 9.067, 10.001, 13.914, 18.229 and 18.940.

Furthermore, the crystalline form D of the maleate has an X-ray powder diffraction pattern with characteristic peaks at diffraction angles 2θ of 4.486, 5.998, 7.288, 9.067, 10.001, 13.914, 15.026, 16.227, 18.229 and 18.940.

Furthermore, the crystalline form D of the maleate has an X-ray powder diffraction pattern with characteristic peaks at diffraction angles 2θ of 4.486, 5.998, 7.288, 9.067, 10.001, 13.914, 15.026, 16.227, 18.229, 18.940, 23.076, 25.612 and 28.102.

The present disclosure provides a hydrobromide of a compound of formula II.

In some embodiments, the hydrobromide is a crystalline form I having an X-ray powder diffraction pattern with characteristic peaks at diffraction angles 2θ of 8.128, 12.579, 16.414, 17.075, 17.780 and 20.733.

Furthermore, the crystalline form I of the hydrobromide has an X-ray powder diffraction pattern with characteristic peaks at diffraction angles 2θ of 8.128, 12.579, 16.414, 17.075, 17.780, 19.675, 20.733, 21.262, 23.113, 23.906, 24.391, 26.550, 28.445, 28.930 and 29.547. Furthermore, the crystalline form I of the hydrobromide has an X-ray powder diffraction pattern with characteristic peaks at diffraction angles 2θ of 8.128, 11.918, 12.579, 16.414, 17.075, 17.780, 18.750, 19.675, 20.733, 21.262, 23.113, 23.906, 24.391, 26.550, 28.445, 28.930, 29.547, 30.958, 32.236, 33.382, 38.670, 39.640, 40.830, 42.064, 43.342, 46.824, 48.190, 48.983 and 50.746.

The present disclosure provides a hydrochloride of a compound of formula II.

In some embodiments, the hydrochloride is a crystalline form α having an X-ray powder diffraction pattern with characteristic peaks at diffraction angles 2θ of 7.931, 10.115, 13.920, 15.224, 17.425 and 18.309.

Furthermore, the crystalline form α of the hydrochloride has an X-ray powder diffraction pattern with characteristic peaks at diffraction angles 2θ of 7.931, 10.115, 12.166, 13.920, 15.224, 16.041, 16.315, 16.748, 17.425, 18.309, 22.340, 23.359 and 24.570.

Furthermore, the crystalline form α of the hydrochloride has an X-ray powder diffraction pattern with characteristic peaks at diffraction angles 2θ of 7.931, 10.115, 12.166, 13.920, 15.224, 16.041, 16.315, 16.748, 17.425, 18.309, 19.624, 20.235, 21.491, 22.340, 23.359, 23.905 and 24.570. Furthermore, the crystalline form α of the hydrochloride has an X-ray powder diffraction pattern with characteristic peaks at diffraction angles 2θ of 7.931, 10.115, 12.166, 13.920, 15.224, 16.041, 16.315, 16.748, 17.425, 18.309, 19.624, 20.235, 21.491, 22.340, 23.359, 23.905, 24.570, 25.320, 25.811, 26.096, 27.624, 28.213, 29.190, 29.760, 31.266, 31.795, 32.324, 35.906 and 37.291.

In some embodiments, the hydrochloride is a crystalline form β having an X-ray powder diffraction pattern with

characteristic peaks at diffraction angles 2θ of 5.386, 8.191, 12.688, 16.607 and 20.036.

Furthermore, the crystalline form β of the hydrochloride has an X-ray powder diffraction pattern with characteristic peaks at diffraction angles 2θ of 5.386, 8.191, 10.818, 12.688, 13.980, 14.915, 16.607, 20.036 and 21.372.

Furthermore, the crystalline form β of the hydrochloride has an X-ray powder diffraction pattern with characteristic peaks at diffraction angles 2θ of 5.386, 8.191, 10.818, 12.688, 13.980, 14.915, 16.607, 18.076, 19.056, 20.036, 21.372, 22.040, 23.465, 24.355, 25.869, 26.582, 27.383, 29.253, 29.832, 30.946, 31.480, 32.504 and 33.439.

In some embodiments, the hydrochloride is a crystalline form γ having an X-ray powder diffraction pattern with characteristic peaks at diffraction angles 2θ of 8.114, 11.997, 12.640, 13.772, 16.478, 17.897 and 20.337.

Furthermore, the crystalline form γ of the hydrochloride has an X-ray powder diffraction pattern with characteristic peaks at diffraction angles 2θ of 8.114, 11.997, 12.640, 13.772, 16.478, 17.897, 20.337, 21.422, 23.228 and 24.472.

Furthermore, the crystalline form γ of the hydrochloride has an X-ray powder diffraction pattern with characteristic peaks at diffraction angles 2θ of 8.114, 11.997, 12.640, 13.772, 16.478, 17.897, 19.671, 20.337, 21.422, 22.156, 23.228, 24.472, 25.882, 27.567, 28.277, 29.830, 31.160, 32.269 and 33.334.

The present disclosure provides an acetate of a compound of formula II.

In some embodiments, the acetate is a crystalline form having an X-ray powder diffraction pattern with characteristic peaks at diffraction angles 2θ of 11.651, 12.495, 15.636, 15.965, 18.075 and 20.935.

Furthermore, the crystalline form of the acetate has an X-ray powder diffraction pattern with characteristic peaks at diffraction angles 2θ of 11.651, 12.495, 14.323, 15.121, 15.636, 15.965, 18.075, 19.247, 19.903 and 20.935.

Furthermore, the crystalline form of the acetate has an X-ray powder diffraction pattern with characteristic peaks at diffraction angles 2θ of 11.651, 12.495, 14.323, 15.121, 15.636, 15.965, 18.075, 19.247, 19.903, 20.935, 22.107, 22.998, 23.842, 24.733, 25.530, 26.843, 28.719, 29.750, 30.829, 32.142, 35.143 and 39.973.

The present disclosure further relates to a method for preparing benzoate, oxalate, methanesulfonate, maleate, hydrobromide, hydrochloride or acetate of a compound of formula II, comprising: mixing a certain amount of the compound of formula II with a proper amount of a solvent and benzoic acid, oxalic acid, methanesulfonic acid, maleic acid, hydrobromic acid, hydrochloric acid or acetic acid for reaction to obtain the salt of the compound of formula II with the corresponding acid, wherein the solvent is selected from the group consisting of one or more of a hydrocarbon solvent, an ether solvent, an alcohol solvent, an ester solvent, a ketone solvent, a nitrile solvent, a halogenated hydrocarbon solvent, a nitrogenous solvent, water and dimethyl sulfoxide,

the hydrocarbon solvent includes, but is not limited to, *n*-butane, *n*-pentane, *n*-hexane or *n*-heptane;
the ether solvent includes, but is not limited to, tetrahydrofuran, diethyl ether, propylene glycol methyl ether, methyl *tert*-butyl ether, isopropyl ether or 1,4-dioxane;
the alcohol solvent includes, but is not limited to, methanol, ethanol, isopropanol, *n*-propanol, isoamyl alcohol or trifluoroethanol;
the ester solvent includes, but is not limited to, ethyl acetate, isopropyl acetate or butyl acetate; the ketone solvent includes, but is not limited to, acetone, acetophenone or 4-methyl-2-pentanone;
the nitrile solvent includes, but is not limited to, acetonitrile or propionitrile;
the halogenated hydrocarbon solvent includes, but is not limited to, chloromethane, dichloromethane, chloroform or carbon tetrachloride;
the nitrogenous solvent includes, but is not limited to, nitromethane, *N*,*N*-dimethylformamide or *N*,*N*-dimethylacetamide.

In some embodiments, a method for preparing an amorphous form of a compound of formula II, comprises: taking a certain amount of the compound of formula II, adding a proper amount of a solvent, precipitating a solid, filtering and drying to obtain the amorphous form of the compound of formula II. In certain embodiments, the solvent may be selected from the group consisting of isopropyl ether, toluene and isopropyl acetate/*n*-hexane (v:v = 1:3).

In some embodiments, a method for preparing an amorphous form of a compound of formula II, comprises: purifying a certain amount of the compound of formula II by high performance liquid chromatography with an elution system of ammonium bicarbonate/water/acetonitrile to obtain the amorphous form of the compound of formula II.

In some embodiments, a method for preparing an amorphous form of a compound of formula II, comprises: taking a certain amount of the compound of formula II, adding a proper amount of a solvent, precipitating a solid, filtering and drying to obtain the amorphous form of the compound of formula II, wherein the solvent is selected from the group consisting of isopropyl ether, toluene and a mixed solvent of isopropyl acetate and *n*-hexane (v:v = 1:3), and the method for precipitating the amorphous form is selected from the group consisting of precipitation at room temperature, precipitation by cooling and precipitation by volatilizing the solvent.

In some embodiments, a method for preparing an amorphous form of a compound of formula II, comprises: taking a certain amount of the compound of formula II, adding a proper amount of isopropyl ether, toluene or a mixed solvent of isopropyl acetate and *n*-hexane (v:v = 1:3), heating until complete or incomplete dissolution, cooling to room

temperature, stirring to precipitate a solid, filtering and drying to obtain the amorphous form of the compound of formula II.

The present disclosure further relates to a method for preparing an amorphous or crystalline form of a benzoate of a compound of formula II, comprising: taking a certain amount of the compound of formula II, adding a proper amount of a solvent and benzoic acid, precipitating a solid, filtering and drying to obtain the amorphous or crystalline form of the benzoate of the compound of formula II. In certain embodiments, the solvent is *n*-hexane or methyl *tert*-butyl ether.

The present disclosure further relates to a method for preparing an amorphous or crystalline form of an oxalate of a compound of formula II, comprising: taking a certain amount of the compound of formula II, adding a proper amount of a solvent and oxalic acid, precipitating a solid, filtering and drying to obtain the amorphous or crystalline form of the oxalate of the compound of formula II. In certain embodiments, the solvent is *n*-hexane or methyl *tert*-butyl ether.

The present disclosure further relates to a method for preparing an amorphous form of a methanesulfonate of a compound of formula II, comprising: taking a certain amount of the compound of formula II, adding a proper amount of a solvent and methanesulfonic acid, precipitating a solid, filtering and drying to obtain the amorphous form of the methanesulfonate of the compound of formula II. In certain embodiments, the solvent is methyl *tert*-butyl ether. The present disclosure further relates to a method for preparing crystalline forms B, C and D of a maleate of a compound of formula II, comprising: taking a certain amount of the compound of formula II, adding a proper amount of a solvent and maleic acid, precipitating a solid, filtering and drying to obtain the crystalline forms B, C and D of the maleate of the compound of formula II. In certain embodiments, the solvent is methyl *tert*-butyl ether.

The present disclosure further relates to a method for preparing a crystalline form I of a hydrobromide of a compound of formula II, comprising: precipitating a crystal by reacting the compound of formula II with hydrobromic acid in a proper amount of a solvent selected from the group consisting of one or more of a hydrocarbon solvent, an ether solvent, an alcohol solvent, an ester solvent, a ketone solvent, a nitrile solvent, a halogenated hydrocarbon solvent, a nitrogenous solvent, water and dimethyl sulfoxide, wherein

the hydrocarbon solvent includes, but is not limited to, *n*-butane, *n*-pentane, *n*-hexane or *n*-heptane;
the ether solvent includes, but is not limited to, tetrahydrofuran, diethyl ether, propylene glycol methyl ether, methyl *tert*-butyl ether, isopropyl ether or 1,4-dioxane;
the alcohol solvent includes, but is not limited to, methanol, ethanol, isopropanol, *n*-propanol, isoamyl alcohol or trifluoroethanol;
the ester solvent includes, but is not limited to, ethyl acetate, isopropyl acetate or butyl acetate; the ketone solvent includes, but is not limited to, acetone, acetophenone or 4-methyl-2-pentanone;
the nitrile solvent includes, but is not limited to, acetonitrile or propionitrile;
the halogenated hydrocarbon solvent includes, but is not limited to, chloromethane, dichloromethane, chloroform or carbon tetrachloride;
the nitrogenous solvent includes, but is not limited to, nitromethane, *N*,*N*-dimethylformamide or *N*,*N*-dimethylacetamide.

In some embodiments, for the method for preparing the crystalline form I of the hydrobromide of the compound of formula II, the solvent is methyl *tert*-butyl ether and ethanol.

The present disclosure further relates to a method for preparing a crystalline form I of a hydrobromide of a compound of formula II, comprising: mixing a certain amount of the compound of formula II with a proper amount of a solvent and hydrobromic acid, precipitating a solid, filtering and drying to obtain the crystalline form I of the hydrobromide of the compound of formula II. In certain embodiments, the solvent is methyl *tert*-butyl ether and ethanol.

The present disclosure further relates to a method for preparing crystalline forms $\alpha$, $\beta$ and $\gamma$ of a hydrochloride of a compound of formula II, comprising: taking a certain amount of the compound of formula II, adding a proper amount of a solvent and hydrochloric acid, precipitating a solid, filtering and drying to obtain the crystalline forms $\alpha$, $\beta$ and $\gamma$ of the hydrochloride of the compound of formula II. In certain embodiments, the solvent is methyl *tert*-butyl ether.

The present disclosure further relates to a method for preparing a crystalline form $\gamma$ of a hydrochloride of a compound of formula II, comprising: loading a certain amount of the crystalline form $\beta$ of the compound of formula II to a DVS system and running a process with parameters of dm/dt = 0.002, 50-95-0-95-50% RH, Max 360 min, 25 °C to obtain the crystalline form $\gamma$ of the hydrochloride of the compound of formula II.

The present disclosure further relates to a method for preparing a crystalline form of an acetate of a compound of formula II, comprising: taking a certain amount of the compound of formula II, adding a proper amount of a solvent and acetic acid, precipitating a solid, filtering and drying to obtain the crystalline form of the acetate of the compound of formula II. In certain embodiments, the solvent is water and ethanol.

In certain embodiments, for the preparation process of the amorphous form of the compound of formula II, the benzoate, oxalate, methanesulfonate, maleate, hydrobromide, hydrochloride and acetate of the compound of formula II, and the crystalline form of the benzoate, the amorphous form of the benzoate, the crystalline form of the oxalate, the amorphous form of the oxalate, the amorphous form of the methanesulfonate, the crystalline forms B, C and D of the

maleate, the crystalline form I of the hydrobromide, the crystalline forms α, β and γ of the hydrochloride and the crystalline form of the acetate, the solvent is selected from the group consisting of one or more of a hydrocarbon solvent, an ether solvent, an alcohol solvent, an ester solvent, a ketone solvent, a nitrile solvent, a halogenated hydrocarbon solvent, a nitrogenous solvent, water and dimethyl sulfoxide. The hydrocarbon solvent includes, but is not limited to, *n*-butane, *n*-pentane, *n*-hexane or *n*-heptane; the ether solvent includes, but is not limited to, diethyl ether, propylene glycol methyl ether, methyl *tert*-butyl ether, isopropyl ether or 1,4-dioxane; the alcohol solvent includes, but is not limited to, methanol, ethanol, isopropanol, *n*-propanol, isoamyl alcohol or trifluoroethanol; the ester solvent includes, but is not limited to, ethyl acetate, isopropyl acetate or butyl acetate; the ketone solvent includes, but is not limited to, acetone, acetophenone or 4-methyl-2-pentanone; the nitrile solvent includes, but is not limited to, acetonitrile or propionitrile; the halogenated hydrocarbon solvent includes, but is not limited to, chloromethane, dichloromethane, 1,2-dichloroethane, chloroform or carbon tetrachloride; and the nitrogenous solvent includes, but is not limited to, nitromethane, *N*,*N*-dimethylformamide or *N*,*N*-dimethylacetamide.

The method for precipitating a solid form of the amorphous form of the compound of formula II or the acid addition salt of the compound of formula II or the pharmaceutically acceptable solvate of the acid addition salt disclosed herein is selected from the group consisting of precipitation at room temperature, precipitation by cooling and precipitation by volatilizing the solvent.

In certain embodiments, the solid form of the acid addition salt of the compound of formula II or the pharmaceutically acceptable solvate of the acid addition salt disclosed herein refers to the crystalline form of the benzoate, the amorphous form of the benzoate, the crystalline form of the oxalate, the amorphous form of the oxalate, the amorphous form of the methanesulfonate, the crystalline forms B, C and D of the maleate, the crystalline form I of the hydrobromide, the crystalline forms α, β and γ of the hydrochloride and the crystalline form of the acetate of the compound of formula II.

The method for crystallizing the crystalline form of the compound disclosed herein is selected from the group consisting of crystallization at room temperature, crystallization by cooling, crystallization by volatilization or induction crystallization by adding seed crystal, and the crystalline form of the compound is selected from the group consisting of the crystalline form of the benzoate, the crystalline form of the oxalate, the crystalline forms B, C and D of the maleate, the crystalline form I of the hydrobromide, the crystalline forms α, β and γ of the hydrochloride and the crystalline form of the acetate of the compound of formula II.

The present disclosure further relates to a method for preparing an amorphous or crystalline form of a benzoate of a compound of formula II, comprising: taking a certain amount of the compound of formula II, adding a proper amount of *n*-hexane or methyl *tert-butyl* ether and benzoic acid, stirring at 50 °C overnight, filtering and drying to obtain the amorphous or crystalline form of the benzoate of the compound of formula II.

The present disclosure further relates to a method for preparing an amorphous or crystalline form of an oxalate of a compound of formula II, comprising: taking a certain amount of the compound of formula II, adding a proper amount of methyl *tert*-butyl ether or *n*-hexane and oxalic acid, stirring at 50 °C overnight, filtering and drying to obtain the amorphous or crystalline form of the oxalate of the compound of formula II.

The present disclosure further relates to a method for preparing an amorphous form of a methanesulfonate of a compound of formula II, comprising: taking a certain amount of the compound of formula II, adding a proper amount of methyl *tert-butyl* ether and methanesulfonic acid, stirring at 50 °C overnight, filtering and drying to obtain the amorphous form of the methanesulfonate of the compound of formula II.

The present disclosure further relates to a method for preparing a crystalline form B of a maleate of a compound of formula II, comprising: taking a certain amount of the compound of formula II, adding a proper amount of methyl *tert*-butyl ether and maleic acid, stirring at 50 °C at 600 rpm for 10 min to 10 h, filtering and drying to obtain the crystalline form B of the maleate of the compound of formula II.

The present disclosure further relates to a method for preparing a crystalline form B of a maleate of a compound of formula II, comprising: taking a certain amount of the compound of formula II, adding a proper amount of methyl *tert*-butyl ether and maleic acid, stirring at 50 °C at 600 rpm for 10 min, 20 min, 30 min, 1 h or 2 h, filtering and drying to obtain the crystalline form B of the maleate of the compound of formula II.

The present disclosure further relates to a method for preparing a crystalline form C of a maleate of a compound of formula II, comprising: taking a certain amount of the compound of formula II, adding a proper amount of methyl *tert*-butyl ether and maleic acid, stirring at 50 °C at 600 rpm for 12 h to 36 h, filtering and drying to obtain the crystalline form C of the maleate of the compound of formula II.

The present disclosure further relates to a method for preparing a crystalline form C of a maleate of a compound of formula II, comprising: taking a certain amount of the compound of formula II, adding a proper amount of methyl *tert*-butyl ether and maleic acid, stirring at 50 °C at 600 rpm for 1 d, filtering and drying to obtain the crystalline form C of the maleate of the compound of formula II.

The present disclosure further relates to a method for preparing a crystalline form D of a maleate of a compound of formula II, comprising: taking a certain amount of the compound of formula II, adding a proper amount of methyl *tert*-butyl ether and maleic acid, stirring at 50 °C at 600 rpm for 48 h to 72 h, filtering and drying to obtain the crystalline

form D of the maleate of the compound of formula II.

The present disclosure further relates to a method for preparing a crystalline form D of a maleate of a compound of formula II, comprising: taking a certain amount of the compound of formula II, adding a proper amount of methyl *tert*-butyl ether and maleic acid, stirring at 50 °C at 600 rpm for 3 d, filtering and drying to obtain the crystalline form D of the maleate of the compound of formula II.

The present disclosure further relates to a method for preparing a crystalline form I of a hydrobromide of a compound of formula II, comprising: taking a certain amount of the compound of formula II, adding a proper amount of methyl *tert-butyl* ether and hydrobromic acid or a mixture of hydrobromic acid and ethanol, stirring at 25 °C at 600 rpm for 12 h to 72 h, filtering and drying to obtain the crystalline form I of the hydrobromide of the compound of formula II.

The present disclosure further relates to a method for preparing a crystalline form I of a hydrobromide of a compound of formula II, comprising: taking a certain amount of the compound of formula II, adding a proper amount of methyl *tert-butyl* ether and hydrobromic acid, stirring at 25 °C at 600 rpm for 3 d, filtering and drying to obtain the crystalline form I of the hydrobromide of the compound of formula II.

The present disclosure further relates to a method for preparing a crystalline form I of a hydrobromide of a compound of formula II, comprising: taking a certain amount of the compound of formula II, adding a proper amount of methyl *tert-butyl* ether and a mixture of hydrobromic acid and ethanol (in a volume ratio selected from the group consisting of 1:1, 1:50 and 1:99), stirring at 25 °C at 600 rpm overnight and for 3 d, filtering and drying to obtain the crystalline form I of the hydrobromide of the compound of formula II.

The present disclosure further relates to a method for preparing a crystalline form α of a hydrochloride of a compound of formula II, comprising: taking a certain amount of the compound of formula II, adding a proper amount of methyl *tert*-butyl ether and concentrated hydrochloric acid, stirring at 50 °C for 12 h to 48 h, filtering and drying to obtain the crystalline form α of the hydrochloride of the compound of formula II.

The present disclosure further relates to a method for preparing a crystalline form α of a hydrochloride of a compound of formula II, comprising: taking a certain amount of the compound of formula II, adding a proper amount of methyl *tert*-butyl ether and concentrated hydrochloric acid, stirring at 50 °C for 2 d, filtering and drying to obtain the crystalline form α of the hydrochloride of the compound of formula II.

The present disclosure further relates to a method for preparing a crystalline form β of a hydrochloride of a compound of formula II, comprising: taking a certain amount of the compound of formula II, adding a proper amount of methyl *tert-butyl* ether and a solution of hydrochloric acid in ethanol, precipitating a solid, filtering and drying to obtain the crystalline form β of the hydrochloride of the compound of formula II.

The present disclosure further relates to a method for preparing a crystalline form β of a hydrochloride of a compound of formula II, comprising: taking a certain amount of the compound of formula II, adding a proper amount of methyl *tert-butyl* ether and a solution of hydrochloric acid in ethanol (concentrated hydrochloric acid:ethanol = 1:99, well mixed), stirring at 25 °C for 1 h and at 50 °C for 2 d, filtering and drying to obtain the crystalline form β of the hydrochloride of the compound of formula II.

The present disclosure further relates to a method for preparing a crystalline form of an acetate of a compound of formula II, comprising: taking a certain amount of the compound of formula II, adding a proper amount of water and a solution of acetic acid in ethanol, precipitating a solid, filtering and drying to obtain the crystalline form of the acetate of the compound of formula II. The present disclosure further relates to a method for preparing a crystalline form of an acetate of a compound of formula II, comprising: taking a certain amount of the compound of formula II, adding a proper amount of water and a solution of acetic acid in ethanol (precisely transferring 0.1 mL of acetic acid, adding 9.9 mL of ethanol, mixing well), stirring at 50 °C overnight, filtering and drying to obtain the crystalline form of the acetate of the compound of formula II. The present disclosure further relates to a pharmaceutical composition comprising an acid addition salt of a compound of formula II or a pharmaceutically acceptable solvate of the acid addition salt or an amorphous form of the compound of formula II, and one or more pharmaceutically acceptable carriers, diluents or excipients.

The present disclosure further relates to a pharmaceutical composition comprising a crystalline form I of a hydrobromide of a compound of formula II, and one or more pharmaceutically acceptable carriers, diluents or excipients.

The present disclosure further relates to a pharmaceutical composition prepared from an amorphous form of a compound of formula II, an acid addition salt of the compound of formula II or a pharmaceutically acceptable solvate of the acid addition salt, and one or more pharmaceutically acceptable carriers, diluents or excipients.

The present disclosure further relates to a pharmaceutical composition prepared from a crystalline form I of a hydrobromide of a compound of formula II and one or more pharmaceutically acceptable carriers, diluents or excipients.

The present disclosure further relates to a pharmaceutical composition comprising an amorphous form of a compound of formula II or an acid addition salt of the compound of formula II or a pharmaceutically acceptable solvate of the acid addition salt, and optionally one or more pharmaceutically acceptable carriers and/or diluents. The pharmaceutical composition can be formulated into any pharmaceutically acceptable dosage form, for example, into tablet, capsule, pill, granule, solution, suspension, syrup, injection (the formulation is prepared from the amorphous form of the compound of formula II or the acid addition salt of the compound of formula II or the pharmaceutically acceptable solvate

of the acid addition salt disclosed herein, or the injection itself comprises the amorphous form of the compound of formula II or the acid addition salt of the compound of formula II or the pharmaceutically acceptable solvate of the acid addition salt disclosed herein, including a solution for injection, a sterile powder for injection and a concentrated solution for injection), suppository, inhalant or spray.

The present disclosure further relates to a method for preparing a pharmaceutical composition, comprising: mixing the amorphous form of the compound of formula II or the acid addition salt of the compound of formula II or the pharmaceutically acceptable solvate of the acid addition salt disclosed herein with at least one pharmaceutically acceptable carrier, diluent or excipient. In addition, the pharmaceutical composition disclosed herein can be administered by any suitable route of administration, such as oral, parenteral, rectal, pulmonary or local administration, to a patient or subject in need. For oral administration, the pharmaceutical composition can be formulated into an oral formulation, for example, a solid oral formulation such as tablet, capsule, pill and granule, or a liquid oral formulation such as oral solution, oral suspension and syrup. When formulated into an oral formulation, the pharmaceutical formulation may further comprise a suitable filler, binder, disintegrant, lubricant and the like. For parenteral administration, the pharmaceutical composition can be formulated into an injection, including a solution for injection, a sterile powder for injection and a concentrated solution for injection. When formulated into an injection, the pharmaceutical composition may be manufactured by conventional methods in the prior art. When formulated into an injection, the pharmaceutical formulation may be free of additives, or contain proper additives according to the nature of the medicament. For rectal administration, the pharmaceutical formulation can be formulated into a suppository or the like. For pulmonary administration, the pharmaceutical formulation can be formulated into an inhalant or spray. In certain embodiments, the amorphous form of the compound of formula II or the acid addition salt of the compound of formula II or the pharmaceutically acceptable solvate of the acid addition salt disclosed herein is present in the pharmaceutical composition or medicament in a therapeutically and/or prophylactically effective amount. In certain embodiments, the amorphous form of the compound of formula II or the acid addition salt of the compound of formula II or the pharmaceutically acceptable solvate of the acid addition salt disclosed herein is present in the pharmaceutical composition or medicament in a form of unit dose.

The present disclosure further relates to use of an amorphous form of a compound of formula II or an acid addition salt of the compound of formula II or a pharmaceutically acceptable solvate of the acid addition salt, or a pharmaceutical composition comprising or prepared from the same, in preparing a medicament for treating a disease or condition mediated by RORγ. The disease or condition mediated by RORγ includes, but is not limited to, inflammatory and autoimmune diseases and cancers, wherein the inflammatory and autoimmune diseases include, but are not limited to, arthritis, rheumatoid arthritis, juvenile rheumatoid arthritis, psoriasis vulgaris, psoriatic arthritis, osteoarthritis, regional suppurative, ulcerative colitis, ankylosing spondylitis, autoimmune diabetes, type I diabetes, autoimmune ocular disease, autoimmune thyroid disease, type I immune hypersecretion syndrome, type II autoimmune polycyrine syndrome, multiple sclerosis, inflammatory bowel disease, inflammatory bowel syndrome, juvenile idiopathic arthritis, Sjögren syndrome, Crohn's disease, asthma, Kawasaki's disease, Hashimoto's thyroiditis, infectious disease, ankylosing spondylitis, chronic obstructive pulmonary disease (COPD), pulmonary disease, glomerulonephritis, myocarditis, thyroiditis, dry eye, uveitis, Behcet's disease, asthma, atopic dermatitis, contact dermatitis, allograft rejection, polymyositis, GVHD, acne, ulcerative colitis, systemic lupus erythematosus, scleroderma, bronchitis, dermatomyositis and allergic rhinitis; the cancers include, but are not limited to, non-Hodgkin's lymphoma, diffuse large B-cell lymphoma, follicular lymphoma, synovial sarcoma, breast cancer, cervical cancer, colon cancer, lung cancer, stomach cancer, rectal cancer, pancreatic cancer, brain cancer, skin cancer, mouth cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, papillary renal tumor, head and neck tumor, leukemia, lymphoma, myeloma and non-small cell lung cancer.

## SUMMARY

In the specification and claims of the present application, unless otherwise specified, the scientific and technological terms used herein have meanings generally understood by those skilled in the art. However, definitions and explanations for some of the related terms are provided below to better understand the present disclosure. In addition, if the definitions and explanations of the terms provided in the present application are not consistent with the meanings generally understood by those skilled in the art, the definitions and explanations of the terms provided in the present application shall prevail.

The "ether solvent" described herein includes, but is not limited to: tetrahydrofuran, diethyl ether, propylene glycol methyl ether, methyl *tert*-butyl ether, isopropyl ether or 1,4-dioxane.

Specific examples of "alcohol solvent" described herein include, but are not limited to: methanol, ethanol, isopropanol, *n*-propanol, isoamyl alcohol or trifluoroethanol.

The "ester solvent" described herein includes, but is not limited to: ethyl acetate, isopropyl acetate or butyl acetate.

Specific examples of "ketone solvent" described herein include, but are not limited to: acetone, acetophenone or 4-methyl-2-pentanone.

Specific examples of "nitrile solvent" described herein include, but are not limited to: acetonitrile or propionitrile.

Specific examples of "halogenated hydrocarbon solvent" described herein include, but are not limited to: chloromethane, dichloromethane, chloroform or carbon tetrachloride.

Specific examples of "hydrocarbon solvent" described herein include, but are not limited to: *n*-butane, *n*-pentane, *n*-hexane or *n*-heptane.

The "X-ray powder diffraction pattern or XRPD" described herein is obtained by Cu-K$\alpha$ ray diffraction.

The "differential scanning calorimetry or DSC" described herein refers to measurement of the temperature difference and heat flow difference between a sample and a reference substance during a process of increasing or holding the temperature of the sample to characterize all the physical changes and chemical changes related to the thermal effect, and to obtain the phase change information of the sample.

The "2$\theta$ or angle 2$\theta$" described herein refers to diffraction angle. $\theta$ is Bragg angle in unit ° or degree. The error range of 2$\theta$ may be $\pm$0.3, $\pm$0.2 or $\pm$0.1.

**Beneficial Effects**

The amorphous form of the compound of formula II, the benzoate, oxalate, methanesulfonate, maleate, hydrobromide, hydrochloride and acetate of the compound of formula II, and the crystalline form of the benzoate, the amorphous form of the benzoate, the crystalline form of the oxalate, the amorphous form of the oxalate, the amorphous form of the methanesulfonate, the crystalline forms B, C and D of the maleate, the crystalline form I of the hydrobromide, the crystalline forms $\alpha$, $\beta$ and $\gamma$ of the hydrochloride and the crystalline form of the acetate of the compound of formula II provided herein provide alternative solid forms of the compound of formula II that can be used as ROR$\gamma$ regulators in drug development processes.

## BRIEF DESCRIPTION OF THE DRAWINGS

FIG. 1 is an XRPD pattern of an amorphous form of a compound of formula II.
FIG. 2 is an XRPD pattern of an amorphous form of a benzoate of a compound of formula II.
FIG. 3 is an XRPD pattern of a crystalline form of a benzoate of a compound of formula II.
FIG. 4 is a DSC pattern of a crystalline form of a benzoate of a compound of formula II.
FIG. 5 is an XRPD pattern of an amorphous form of an oxalate of a compound of formula II.
FIG. 6 is an XRPD pattern of a crystalline form of an oxalate of a compound of formula II.
FIG. 7 is an XRPD pattern of an amorphous form of a methanesulfonate of a compound of formula II.
FIG. 8 is an XRPD pattern of a crystalline form B of a maleate of a compound of formula II.
FIG. 9 is a DSC pattern of a crystalline form B of a maleate of a compound of formula II.
FIG. 10 is a TGA pattern of a crystalline form B of a maleate of a compound of formula II.
FIG. 11 is a DVS vapor sorption plot of a crystalline form B of a maleate of a compound of formula II.
FIG. 12 is a comparison of XRPD patterns before and after DVS analysis of a crystalline form B of a maleate of a compound of formula II.
FIG. 13 is an XRPD pattern of a crystalline form C of a maleate of a compound of formula II.
FIG. 14 is a DSC pattern of a crystalline form C of a maleate of a compound of formula II.
FIG. 15 is a TGA pattern of a crystalline form C of a maleate of the compound of formula II.
FIG. 16 is an XRPD pattern of a crystalline form D of a maleate of a compound of formula II.
FIG. 17 is a DSC pattern of a crystalline form D of a maleate of a compound of formula II.
FIG. 18 is a TGA pattern of a crystalline form D of a maleate of a compound of formula II.
FIG. 19 is an XRPD pattern of a crystalline form I of a hydrobromide of a compound of formula II.
FIG. 20 is a DSC pattern of a crystalline form I of a hydrobromide of a compound of formula II.
FIG. 21 is a TGA pattern of a crystalline form I of a hydrobromide of a compound of formula II.
FIG. 22 is a DVS vapor sorption plot of a crystalline form I of a hydrobromide of a compound of formula II.
FIG. 23 is a comparison of XRPD patterns before and after DVS analysis of a crystalline form I of a hydrobromide of a compound of formula II.
FIG. 24 is an XRPD pattern of a crystalline form $\alpha$ of a hydrochloride of a compound of formula II.
FIG. 25 is a DSC pattern of a crystalline form $\alpha$ of a hydrochloride of a compound of formula II.
FIG. 26 is a TGA pattern of a crystalline form $\alpha$ of a hydrochloride of a compound of formula II.
FIG. 27 is a DVS vapor sorption plot of a crystalline form $\alpha$ of a hydrochloride of a compound of formula II.

FIG. 28 is a comparison of XRPD patterns before and after DVS analysis of a crystalline form α of a hydrochloride of a compound of formula II.
FIG. 29 is an XRPD pattern of a crystalline form β of a hydrochloride of a compound of formula II.
FIG. 30 is a DSC pattern of a crystalline form β of a hydrochloride of a compound of formula II.
FIG. 31 is a TGA pattern of a crystalline form β of a hydrochloride of a compound of formula II.
FIG. 32 is a DVS vapor sorption plot of a crystalline form β of a hydrochloride of a compound of formula II.
FIG. 33 is a comparison of XRPD patterns before and after DVS analysis of a crystalline form β of a hydrochloride of a compound of formula II.
FIG. 34 is an XRPD pattern of a crystalline form γ of a hydrochloride of a compound of formula II.
FIG. 35 is an XRPD pattern of a crystalline form of an acetate of a compound of formula II.
FIG. 36 is a DSC pattern of a crystalline form of an acetate of a compound of formula II.

## DETAILED DESCRIPTION

Hereinafter, the present disclosure will be explained in more details with reference to the examples. The examples are only used to illustrate the technical solutions of the present disclosure, rather than limit the essence and scope of the present disclosure.

Test conditions for the instruments used in the experiment:

The structure of the compounds was determined by nuclear magnetic resonance (NMR) analysis or/and mass spectrometry (MS). NMR shifts (δ) are given in a unit of $10^{-6}$ (ppm). NMR analysis was conducted with a Bruker AVANCE-400 system using deuterated dimethyl sulfoxide (DMSO-$d_6$), deuterated chloroform ($CDCl_3$) and deuterated methanol ($CD_3OD$) as solvents and tetramethylsilane (TMS) as internal standard.

MS was conducted with a FINNIGAN LCQAd (ESI) mass spectrometer (manufacturer: Thermo, model: Finnigan LCQ advantage MAX).

The HPLC was conducted with an Agilent 1200 DAD high pressure liquid chromatograph (Sunfire C18 150 × 4.6 mm chromatographic column) and a Waters 2695-2996 high pressure liquid chromatograph (Gimini C18 150 × 4.6 mm chromatographic column).

XRPD refers to X-ray powder diffraction detection: The measurement was conducted using a BRUKER D8 X-ray diffractometer with a Cu anode (40 kV, 40 mA) and Cu-Kα radiation (λ = 1.5418 Å). Scanning mode: θ/2θ, scanning range: 10-48°.

DSC refers to differential scanning calorimetry: The measurement was conducted using a METTLER TOLEDO DSC 3+ differential scanning calorimeter with a temperature ramping rate of 10 °C/min, specific temperature ranges shown in corresponding patterns (mostly 25-300 or 25-350 °C) and a nitrogen purging speed of 50 mL/min.

TGA refers to thermogravimetric analysis: The measurement was conducted using a METTLER TOLEDO TGA 2 thermogravimetric analyzer with a temperature ramping rate of 10 °C/min, specific temperature ranges shown in corresponding patterns (mostly 25-300 °C) and a nitrogen purging speed of 20 mL/min.

DVS refers to dynamic vapor sorption: The measurement was conducted with a Surface Measurement Systems advantage 2 at 25 °C, starting from 50% humidity in humidity range of 0%-95% with a step size of 10%. The judging criterion was that the mass change of each gradient dM/dT is less than 0.002 and TMAX is less than 360 min in two circles.

The monitoring of the reaction progress in the examples was conducted by thin layer chromatography (TLC). The developing solvent for reactions, the eluent system for column chromatography purification and the developing solvent system for thin layer chromatography included: A: *n*-hexane/ethyl acetate system. The volume ratio of the solvents was adjusted according to the polarity of the compound, or by adding a small amount of basic or acidic reagents such as triethylamine and acetic acid.

Comparative Example 1. Preparation example of compound of formula II (methods in Examples 152 and 153 of Patent Application No. PCT/US19/30526)

Preparation of (*S*)-3-(6-chloro-5-(2-(difluoromethoxy)phenyl)-1*H*-benzo[*d*]imidazol-2-yl)-3-(4-((cyclopropylmethyl)sulfonyl)phenyl)propanamide

Preparation of (*R*)-3-(6-chloro-5-(2-(difluoromethoxy)phenyl)-1*H*-benzo[*d*]imidazol-2-yl)-3-(4-((cyclopropylmethyl)sulfonyl)phenyl)propanamide

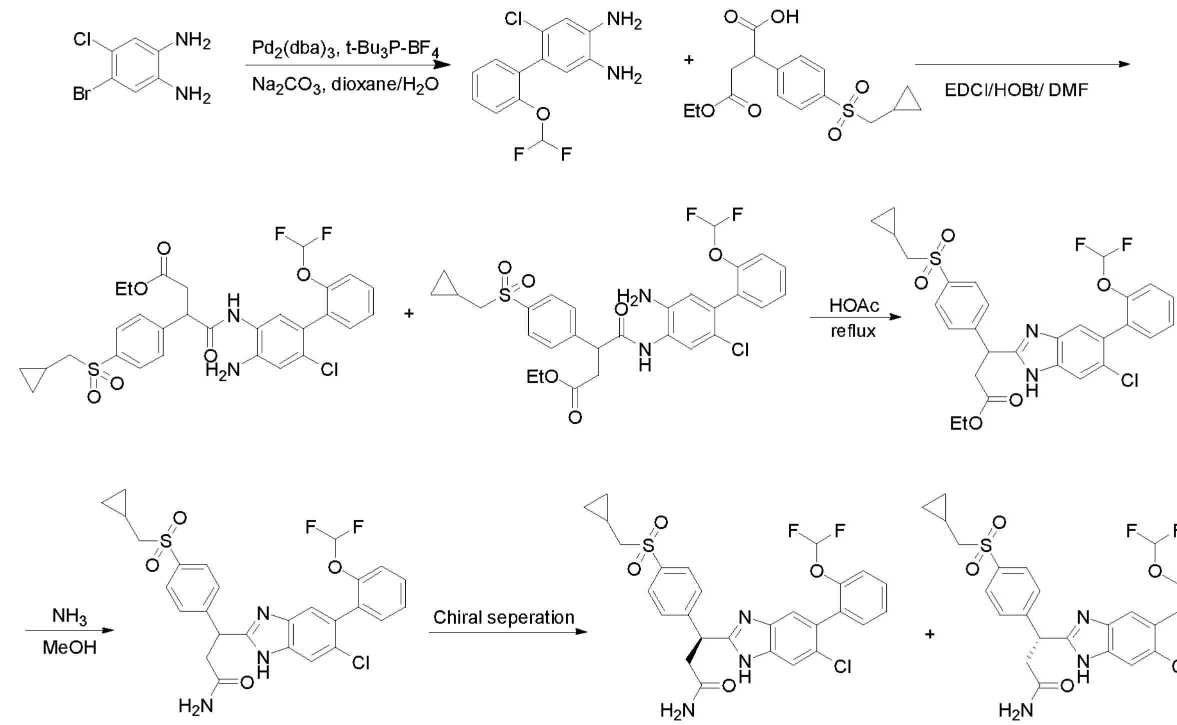


Step I. Preparation of 6-chloro-2'-(difluoromethoxy)-[1,1'-biphenyl]-3,4-diamine

A mixture of 4-bromo-5-chlorobenzene-1,2-diamine (1.5 g, 6.78 mmol), 2-(2-(difluoromethoxy)phenyl)-4,4,5,5-tetramethyl-1,3,2-dioxaborolane (2.2 g, 8.15 mmol), tris(dibenzylideneacetone)dipalladium (620 mg), tri-*tert*-butylphosphonium tetrafluoroborate (393 mg), sodium carbonate (1.7 g, 13.7 mmol), 1,4-dioxane (50 mL) and water (10 mL) was deoxygenated, heated to 90 °C, and stirred for 3 h. The reaction solution was concentrated at reduced pressure. The residue was directly loaded on an ISCO solid column, and eluted with a mixed solvent of *n*-hexane/ethyl acetate to give a white solid product (1.0 g, 51.9% yield). MS (+) ES: 285 $(M+H)^+$.

Step II. Preparation of ethyl 4-((4-amino-6-chloro-2'-(dichloromethoxy)-[1,1'-biphenyl]-3-yl)amino)-3-(4-((cyclopropylmethyl)sulfonyl)phenyl)-4-oxobutanoate

Preparation of ethyl 4-((5-amino-2-chloro-2'-(dichloromethoxy)-[1,1'-biphenyl]-4-yl)amino)-3-(4-((cyclopropylmethyl)sulfonyl)phenyl)-4-oxobutanoate

EDCI (560 mg, 2.93 mmol), HOBT (447 mg, 2.93 mmol) and DIPEA (380 mg, 2.94 mmol) were added to a solution of 6-chloro-2'-(difluoromethoxy)-[1,1'-biphenyl]-3,4-diamine (543 mg, 1.9 mmol), 2-(4-((cyclopropylmethyl)sulfonyl)phenyl)-4-ethoxy-4-oxobutanoic acid (500 mg, 1.47 mmol) and DMF (5 mL), and the reaction system was stirred at room temperature for 2 h. The reaction solution was adsorbed on 5 g of silica gel, loaded onto a silica gel column, and eluted with 45% ethyl acetate in *n*-hexane to obtain a mixture of ethyl 4-((4-amino-6-chloro-2'-(dichloromethoxy)-[1,1'-biphenyl]-3-yl)amino)-3-(4-((cyclopropylmethyl)sulfonyl)phenyl)-4-oxobutanoate and ethyl 4-((5-amino-2-chloro-2'-(dichloromethoxy)-[1,1-biphenyl]-4-yl)amino)-3-(4-((cyclopropylmethyl)sulfonyl)phenyl)-4-oxobutanoate as a white solid (600 mg, 62.3% yield). MS (ESI): 607 $(M+H)^+$.

Step III. Preparation of ethyl 3-(6-chloro-5-(2-(difluoromethoxy)phenyl)-1*H*-benzo[*d*]imidazol-2-yl)-3-(4-((cyclopropylmethyl)sulfonyl)phenyl)propionate

A solution of the mixture of ethyl 4-((4-amino-6-chloro-2'-(dichloromethoxy)-[1,1'-biphenyl]-3-yl)amino)-3-(4-((cyclopropylmethyl)sulfonyl)phenyl)-4-oxobutanoate and ethyl 4-((5-amino-2-chloro-2'-(dichloromethoxy)-[1,1'-biphenyl]-4-yl)amino)-3-(4-((cyclopropylmethyl)sulfonyl)phenyl)-4-oxobutanoate (800 mg) in acetic acid (15 mL) was heated to 80 °C and stirred for 2 h for reaction. The reaction solution was concentrated at reduced pressure, and the obtained residue was purified by column chromatography with an eluent of 60% ethyl acetate in *n*-hexane to obtain ethyl 3-(6-chloro-5-(2-(dichloromethoxy)phenyl)-1*H*-benzo[*d*]imidazol-2-yl)-3-(4-((cyclopropylmethyl)sulfonyl)phenyl)propionate as an off-white solid (600 mg, 77.3% yield). MS (ESI): 589 $(M+H)^+$.

Step IV. Preparation of (*S*)-3-(6-chloro-5-(2-(difluoromethoxy)phenyl)-1*H*-benzo[*d*]imidazol-2-yl)-3-(4-((cyclopropylmethyl)sulfonyl)phenyl)propanamide

Preparation of (*R*)-3-(6-chloro-5-(2-(difluoromethoxy)phenyl)-1*H*-benzo[*d*]imidazol-2-yl)-3-(4-((cyclopropylmethyl)sulfonyl)phenyl)propanamide

A 7 N solution of ammonia in methanol (4.8 mL, 33.9 mmol) was added to a solution of ethyl 3-(6-chloro-5-(2-(difluoromethoxy)phenyl)-1*H*-benzo[*d*]imidazol-2-yl)-3-(4-((cyclopropylmethyl)sulfonyl)phenyl)propionate (400 mg, 0.68 mmol) in methanol (5 mL). The reaction system was heated to 60 °C and stirred for 12 h for reaction. The reaction solution was concentrated at reduced pressure, and the resulting crude product was purified by column chromatography with a *n*-hexane/ethyl acetate eluent system to obtain 3-(6-chloro-5-(2-(difluoromethoxy)phenyl)-1*H*-benzo[*d*]imidazol-2-yl)-3-(4-((cyclopropylmethyl)sulfonyl)phenyl)propanamide (177 mg).

The resulting product was subjected to chiral resolution (conditions: CHIRALCEL OZ-H (OZH00CD-VC005), 0.46 cm I.D. × 15 cm L; mobile phase: 100% methanol; flow rate: 1.0 mL/min). The corresponding fractions were collected and concentrated at reduced pressure to obtain the target compound (67 mg, 60 mg).

Single-component compounds (shorter retention time)

MS (+) ES: 560 $(M+H)^+$

Chiral analysis methodology: retention time: 3.919 min, chiral purity: 100% (column: OD Phenomenex Lux Cellulose-1 150 × 4.6 mm, 5 μm; mobile phase: ethanol/*n*-hexane = 80:20 (v:v)).

1H NMR (400 mHz, $CD_3OD$): 7.92 (d, 8.0Hz, 2H), 7.51-7.49 (s, 1H), 7.65 (d, 8.0 Hz, 2 H), 7.56-7.54 (m, 1H), 7.48-7.44 (m, 1H), 7.33-7.32 (m, 2H), 7.28-7.26 (d, 8.0Hz, 1H), 6.86 (d, 8.0Hz, 1H), 4.96-4.92 (t, 8.0Hz, 1H), 3.41-3.35 (dd, 8.0Hz, 1H), 3.13-3.11 (d, 8.0Hz, 2H), 3.12-3.06 (dd, 8.0Hz, 1H), 0.93-0.91 (m, 1H), 0.52-0.50 (d, 8.0Hz, 2H), 0.13-0.11 (d, 8.0Hz, 2H).

Single-component compound (longer retention time, compound of formula II)

MS (+) ES: 560 $(M+H)^+$

Chiral analysis methodology: retention time: 8.942 min, chiral purity: 100% (column: OD Phenomenex Lux Cellulose-1 150 × 4.6 mm, 5 μm; mobile phase: ethanol/*n*-hexane = 80:20 (v:v)).

1H NMR (400 mHz, $CD_3OD$): 7.92 (d, 8.0Hz, 2H), 7.51-7.49 (s, 1H), 7.65 (d, 8.0 Hz, 2 H), 7.56-7.54 (m, 1H), 7.48-7.44 (m, 1H), 7.33-7.32 (m, 2H), 7.28-7.26 (d, 8.0Hz, 1H), 6.86 (d, 8.0Hz, 1H), 4.96-4.92 (t, 8.0Hz, 1H), 3.41-3.35 (dd, 8.0Hz, 1H), 3.13-3.11 (d, 8.0Hz, 2H), 3.12-3.06 (dd, 8.0Hz, 1H), 0.93-0.91 (m, 1H), 0.52-0.50 (d, 8.0Hz, 2H), 0.13-0.11 (d, 8.0Hz, 2H).

**Test Example 1**. Biochemical assay of LanthaScreen TR-FRET RORγ-LBD and co-activation peptide

**Materials and reagents**

1. RORγ LBD-GST tagged (Cat No. RORC-114H, Creative Biomart)
2. Fluorescein-D22 coactivator (Cat No. PV4386, Invitrogen)
3. LanthaScreen™ Tb anti-GST antibody (Cat No. PV3550, Invitrogen)
4. TR-FRET coregulatory buffer D (Cat No. PV4420, Invitrogen)
5. DTT (Cat No. P2325, Fisher)
6. 384 well assay plate (Cat No. 6008280, Perkin Elmer)
7. Tecan Infinite M1000 plate reader (Tecan)

**Procedures**

Complete TR-FRET Coregulator Buffer D was prepared by diluting 1 M DTT with TR-FRET Coregulator Buffer D to a final concentration of 5 mM DTT. The compounds were diluted in Complete TR-FRET Coregulator Buffer D. The solutions were serially 7-fold diluted from an initial concentration of 3 μM to the $7^{th}$ concentration. 10 μL of the dilutions was added to each well of the 384-well plate. For negative and positive controls, 10 μL of Complete TR-FRET Coregulator Buffer D was added.

A RORγ LBD solution was prepared using Complete TR-FRET Coregulator Buffer D. The final concentration

of the RORγ LBD solution in each reaction was 25 ng. Other than the negative wells receiving 5 μL of Complete TR-FRET Coregulator Buffer D, 5 μL of the RORγ LBD solution was added to the remaining wells of the 384-well assay plate.

Complete TR-FRET Coregulator Buffer D was used to prepare a solution containing 0.6 μM Fluorescein-D22 and 8 nM Tbanti-GST antibody, and 5 μL of the prepared solution was added to all wells of the 384-well assay plate.

The 384-well plate was mixed gently on a plate shaker and let stand at room temperature for 1 h away from light. The 384-well plate was sealed with a plastic film to avoid evaporation.

The plate was measured on a Tecan Infinite M1000 plate reader at wavelengths of 520 nm and 495 nm. $IC_{50}$ values were calculated using GraphPad Prism by plotting log compound concentration versus percentage inhibition. The $IC_{50}$ values for the compounds are shown in Table 1.

**Test Example 2.** Assay for inhibiting cytokine IL-17A production in human peripheral blood mononuclear cells

**Materials and instruments**

1. Human PBMC (Stemcell, Cat No. 70025.1)
2. Lymphocyte medium (Zenbio, Cat No. LYMPH-1)
3. TexMACS (Miltenyi Biotec, Cat No. 130-097-196)
4. Human Cytostim (Miltenyi Biotec, Cat No. 130-092-173)
5. Human IL-17 ELISA, human IL-17 enzyme-linked immunosorbent assay kit (R&D Systems, D1700)
6. 96-well cell culture plate (Fisher Scientific, Cat No. 07-200-80)
7. Tecan SPARK plate reader (Tecan)

**Procedures**

Frozen human peripheral blood mononuclear cells (PBMCs) were rapidly thawed in a prewarmed lymphocyte medium and centrifuged at 1000 rpm for 10 min. The cell culture supernatant was discarded, and the cells were gently suspended in the TexMACS medium and counted. T cell activating reagent cytostim (10 μL/mL) was added to the cell suspension in certain proportions, and then the cells were seeded in a 96-well cell culture plate at a density of $1 \times 10^5$ PBMCs/well. Test compounds were diluted in gradient using TexMACS medium and added to the treatment wells in 2-3 replicates. Negative control wells containing cells only without cytostim were prepared to obtain background readings. The cell culture plate was incubated in a 5% carbon dioxide/37 °C incubator for 3 days. Cell culture supernatant was collected 3 days after treatment and centrifuged to remove the suspended matter. IL-17A in the supernatant was then quantified using an IL-17A enzyme-linked immunosorbent assay kit. The log(inhibitor) vs. response -- Variable slope (four parameters) algorithm in GraphPad Prism 6.0 was used to plot the curve for calculating the $IC_{50}$ values of the compounds. The calculation equation of inhibition is as follows:

$$\text{Inhibition\%} = [100 - \frac{\text{OD(Compound)} - \text{OD(NC)}}{\text{OD(PC)} - \text{OD(NC)}} \times 100]/\text{-}1$$

In the calculation equation, inhibition% is inhibition rate; OD(NC) is the reading of cells in negative control groups with no cytostim and no compound; OD(PC) is the reading of cells in positive control groups with cytostim but no compound; OD(compound) is the reading of cells with cytostim and compound.

Table 1. Binding of compounds of formula II to RORγ and IL-17 production in human peripheral blood mononuclear cells

| Compound | RORγ coactivation assay ($IC_{50}$, μM) | IL-17 production ($IC_{50}$, μM) |
|---|---|---|
| Those with longer retention time in Examples 152 and 153 (compound of formula II) | 0.013 | 0.010 |

Example 1. Preparation of amorphous form of compound of formula II

The compound of formula II (30 mg, 53.57 μmol) was added to isopropyl ether (1.5 mL). The mixture was heated to 70 °C to obtain an opaque white suspension. The suspension was slowly cooled to room temperature, stirred for 16 h and filtered. The filter cake was collected and dried *in vacuo* to obtain a product (20 mg, 66% yield). The product

was in an amorphous form with XRPD pattern shown in FIG. 1.

Example 2. Preparation of amorphous form of compound of formula II

The compound of formula II (30 mg, 53.57 μmol) was added to toluene (1.5 mL). The mixture was heated to 70 °C and stirred to obtain a clarified solution. The solution was slowly cooled to room temperature and a solid was precipitated on the inner wall of the container. The mixture was stirred for 16 h at room temperature and filtered. The filter cake was collected and dried *in vacuo* to obtain a product (20 mg, 66% yield). The product was in an amorphous form as determined by X-ray powder diffraction.

Example 3. Preparation of amorphous form of compound of formula II

The compound of formula II (30 mg, 53.57 μmol) was added to a 1.5 mL mixture of isopropyl acetate/*n*-hexane (v:v = 1:3). The mixture was heated to 70 °C and a viscous solid was precipitated. The solution was slowly cooled to room temperature and a solid was precipitated. The mixture was stirred for 16 h at room temperature and filtered. The filter cake was collected and dried *in vacuo* to obtain a product (20 mg, 66% yield). The product was in an amorphous form as determined by X-ray powder diffraction.

Example 4. Preparation of amorphous form of compound of formula II

The compound of formula II (5 g, 8.93 mmol) was purified by high performance liquid chromatography (Waters-2767, eluent system: ammonium bicarbonate, water and acetonitrile) to obtain a product (2.5 g, 50% yield). The product was in an amorphous form as determined by X-ray powder diffraction.

Example 5. Influencing factor study of amorphous form of compound of formula II

A sample of the amorphous form of the compound of formula II (Example 4) was let stand open to examine the stability of the sample in conditions of heating (40 °C and 60 °C), illumination (4500 Lux) and high humidity (RH 75% and RH 90%) in a period of 30 days.

**Results:**

Table 2. Results of influencing factor study

| Conditions | Time (days) | Color and appearance | Amorphous form Purity (%) | Weight gain (%) | Chiral purity |
|---|---|---|---|---|---|
| Initial | 0 | White solid | 99.91 | / | 99.4 |
| | 5 | White solid | 99.91 | / | / |
| 4500 Lux | 10 | White solid | 99.91 | / | 99.3 |
| 40°C | 30 | White solid | 99.93 | / | 99.3 |
| | 5 | White solid | 99.93 | / | / |
| | 10 | White solid | 99.90 | / | 99.4 |
| | 30 | White solid | 99.92 | / | 99.5 |
| 60°C | 5 | White solid | 99.95 | / | / |
| | 10 | White solid | 99.95 | / | 99.4 |
| | 30 | White solid | 99.95 | / | 99.4 |
| RH 75% | 5 | White solid | 99.92 | 10.42 | / |
| | 10 | White solid | 99.90 | 13.2 | 99.3 |
| | 30 | White solid | 99.90 | 23.24 | 99.4 |
| RH 90% | 5 | White solid | 99.91 | 21.67 | / |
| | 10 | White solid | 99.92 | 26.49 | 99.4 |
| | 30 | White solid | 99.89 | 37.24 | 99.4 |

**Conclusion:**

The results of influencing factor study in Table 2 showed that: the amorphous form of the compound of the formula II has good chemical stability after standing for 30 days in conditions of illumination, high temperature of 40 °C, high temperature of 60 °C, high humidity of 75% and high humidity of 90%.

Example 6. Long-term/accelerated stability study of amorphous form of compound of formula II

The amorphous form of the compound of formula II (Example 4) was subjected to a long-term (25 °C, 60% RH)/accelerated (40 °C, 75% RH) stability study with a period of 3 months.

**Results**

Table 3. Result of long-term/accelerated stability study of amorphous form of compound of formula II

| Sample | Condition of standing | Purity/chiral purity Initial | Purity (%) Month 1 | Purity (%) Month 2 | Purity (%) Month 3 | Chiral purity (%) Month 3 |
|---|---|---|---|---|---|---|
| Amorphous form | 25°C, 60%RH | 99.87/99.2 | 99.86 | 99.86 | 99.85 | 99.2 |
| | 40°C, 75%RH | 99.87/99.2 | 99.87 | 99.86 | 99.88 | 99.1 |
| | 5°C | 99.87/99.2 | 99.86 | 99.86 | 99.85 | 99.1 |

The results of the long-term/accelerated stability study in Table 3 showed that: the amorphous form of the compound of formula II has good chemical stability in conditions of a long term (25 °C, 60% RH) and acceleration (40 °C, 75% RH) in a period of 3 months.

Example 7. Preparation of amorphous form of benzoate of compound of formula II

0.5 mL of *n*-hexane was added to 10 mg of the compound of formula II before 2.3 mg of benzoic acid was added. The mixture was stirred overnight at 50 °C and filtered *in vacuo.* The residue was dried for 1 h at 40 °C to obtain a product. The product was identified as an amorphous form of the benzoate of the compound of formula II by X-ray powder diffraction, with an XRPD pattern shown in FIG. 2.

Example 8. Preparation of crystalline form of benzoate of compound of formula II

0.5 mL of methyl *tert*-butyl ether was added to 10 mg of the compound of formula II before 2.3 mg of benzoic acid was added. The mixture was stirred overnight at 50 °C and filtered *in vacuo.* The residue was dried for 1 h at 40 °C to obtain a product. The product was identified as a crystalline form of the benzoate of the compound of formula II by X-ray powder diffraction, with an XRPD pattern shown in FIG. 3. The DSC pattern is shown in FIG. 4, with a first endothermic peak value at 178.46 °C and a second endothermic peak value at 237.42 °C.

Table 4. Characteristic peaks of crystalline form of benzoate of compound of formula II

| No. | 2-Theta | d(A) | I% |
|---|---|---|---|
| Peak 1 | 5.305 | 16.64444 | 100.0 |
| Peak 2 | 7.411 | 11.91828 | 43.6 |
| Peak 3 | 19.140 | 4.63321 | 1.3 |
| Peak 4 | 22.031 | 4.03134 | 23.9 |

Example 9. Preparation of amorphous form of oxalate of compound of formula II

0.5 mL of methyl *tert*-butyl ether was added to 10 mg of the compound of formula II before 2 mg of oxalic acid was added. The mixture was stirred overnight at 50 °C and filtered *in vacuo.* The residue was dried for 1 h at 40 °C to obtain a product. The product was identified as an amorphous form of the oxalate of the compound of formula II by X-ray powder diffraction, with an XRPD pattern shown in FIG. 5.

Example 10. Preparation of crystalline form of oxalate of compound of formula II 0.5 mL of *n*-hexane was added to 10 mg of the compound of formula II before 2 mg of oxalic acid was added. The mixture was stirred overnight at 50 °C and filtered *in vacuo.* The residue was dried for 1 h at 40 °C to obtain a product. The product was identified as a crystalline form of the oxalate of the compound of formula II by X-ray powder diffraction, with an XRPD pattern shown in FIG. 6.

Table 5. Characteristic peaks of crystalline form of oxalate of compound of formula II

| No. | 2-Theta | d(A) | I% |
|---|---|---|---|
| Peak 1 | 14.378 | 6.15525 | 100.0 |
| Peak 2 | 18.463 | 4.80158 | 51.5 |
| Peak 3 | 21.670 | 4.09779 | 70.9 |
| Peak 4 | 23.075 | 3.85126 | 50.6 |
| Peak 5 | 28.127 | 3.17003 | 21.6 |

Example 11. Preparation of amorphous form of methanesulfonate of compound of formula II

0.5 mL of methyl *tert*-butyl ether was added to 10 mg of the compound of formula II before 1.8 μL of methanesulfonic acid was added. The mixture was stirred overnight at 50 °C and filtered *in vacuo.* The residue was dried for 1 h at 40 °C to obtain a product. The product was identified as an amorphous form of the methanesulfonate of the compound of formula II by X-ray powder diffraction, with an XRPD pattern shown in FIG. 7.

Example 12. Preparation of crystalline form B of maleate of compound of formula II

100 mg of the compound of formula II and 22 mg of maleic acid were added to 5 mL of methyl tert-butyl ether. The mixture was stirred at 600 rpm at 50 °C for 30 min to obtain a suspension. The suspension was filtered *in vacuo* and the residue was dried for 1 h at 40 °C to obtain a product. The product was identified as a crystalline form B of the maleate of the compound of formula II by X-ray powder diffraction, with an XRPD pattern shown in FIG. 8. The DSC pattern is shown in FIG. 9, with a first endothermic peak value at 138.04 °C; the TGA pattern is shown in FIG. 10.

DVS characterization: the vapor sorption of the crystalline form B of the maleate at 25 °C increased along with the increase of humidity in a range of 20.0% RH to 80.0% RH, with a weight change of 1.731%, less than 2% but not less than 0.2%, indicating that the sample is slightly hygroscopic. In a normal storage condition (i.e., 60% humidity/25 °C), the vapor sorption was about 1.438%; in an accelerated test condition (i.e., 70% humidity), the vapor sorption was about 1.809%; in an extreme condition (i.e., 90% humidity), the vapor sorption was about 3.077%.

The comparison of X-ray powder diffraction patterns before and after DVS showed that crystalline form did not change during DVS. The DVS pattern is shown in FIG. 11, and the comparison of X-ray powder diffraction patterns before and after DVS is shown in FIG. 12.

Table 6. Characteristic peaks of crystalline form B of maleate of compound of formula II

| No. | 2-Theta | d(A) | I% |
|---|---|---|---|
| Peak 1 | 7.624 | 11.58707 | 74.9 |
| Peak 2 | 9.659 | 9.14922 | 40.7 |
| Peak 3 | 13.815 | 6.40503 | 41.2 |
| Peak 4 | 15.844 | 5.58882 | 56.9 |
| Peak 5 | 17.391 | 5.09522 | 100.0 |
| Peak 6 | 18.619 | 4.76186 | 31.5 |
| Peak 7 | 21.802 | 4.07319 | 76.4 |
| Peak 8 | 23.667 | 3.75633 | 49.8 |
| Peak 9 | 26.441 | 3.36816 | 13.2 |

Example 13. Preparation of crystalline form B of maleate of compound of formula II

100 mg of the compound of formula II and 22 mg of maleic acid were added to 5 mL of methyl *tert*-butyl ether. The mixture was stirred at 600 rpm at 50 °C for 10 min to obtain a suspension. The suspension was filtered *in vacuo* and the residue was dried for 1 h at 40 °C to obtain a product. The product was a crystalline form B of the maleate of the compound of formula II as determined by X-ray powder diffraction.

Example 14. Preparation of crystalline form B of maleate of compound of formula II

100 mg of the compound of formula II and 22 mg of maleic acid were added to 5 mL of methyl *tert*-butyl ether. The mixture was stirred at 600 rpm at 50 °C for 20 min to obtain a suspension. The suspension was filtered *in vacuo* and the residue was dried for 1 h at 40 °C to obtain a product. The product was a crystalline form B of the maleate of the compound of formula II as determined by X-ray powder diffraction.

Example 15. Preparation of crystalline form B of maleate of compound of formula II

100 mg of the compound of formula II and 22 mg of maleic acid were added to 5 mL of methyl *tert*-butyl ether. The mixture was stirred at 600 rpm at 50 °C for 1 h to obtain a suspension. The suspension was filtered *in vacuo* and the residue was dried for 1 h at 40 °C to obtain a product. The product was a crystalline form B of the maleate of the compound of formula II as determined by X-ray powder diffraction.

Example 16. Preparation of crystalline form B of maleate of compound of formula II

100 mg of the compound of formula II and 22 mg of maleic acid were added to 5 mL of methyl *tert*-butyl ether. The mixture was stirred at 600 rpm at 50 °C for 2 h to obtain a suspension. The suspension was filtered *in vacuo* and the residue was dried for 1 h at 40 °C to obtain a product. The product was a crystalline form B of the maleate of the compound of formula II as determined by X-ray powder diffraction.

Example 17. Preparation of crystalline form C of maleate of compound of formula II

100 mg of the compound of formula II and 22 mg of maleic acid were added to 5 mL of methyl *tert*-butyl ether. The mixture was stirred at 600 rpm at 50 °C for 1 d to obtain a suspension. The suspension was filtered *in vacuo* and the residue was dried for 1 h at 40 °C to obtain a product. The product was identified as a crystalline form C of the maleate of the compound of formula II by X-ray powder diffraction, with an XRPD pattern shown in FIG. 13. The DSC pattern is shown in FIG. 14, with a first endothermic peak value at 154.58 °C; the TGA pattern is shown in FIG. 15.

Table 7. Characteristic peaks of crystalline form C of maleate of compound of formula II

| No. | 2-Theta | d(A) | I% |
|---|---|---|---|
| Peak 1 | 7.325 | 12.05902 | 24.9 |
| Peak 2 | 8.635 | 10.23231 | 46.0 |
| Peak 3 | 9.809 | 9.00970 | 38.0 |
| Peak 4 | 11.661 | 7.58265 | 14.6 |
| Peak 5 | 13.649 | 6.48261 | 27.1 |
| Peak 6 | 16.133 | 5.48950 | 23.5 |
| Peak 7 | 16.765 | 5.28383 | 27.8 |
| Peak 8 | 18.346 | 4.83192 | 100.0 |
| Peak 9 | 21.689 | 4.09421 | 37.3 |
| Peak 10 | 23.586 | 3.76901 | 31.9 |
| Peak 11 | 25.303 | 3.51708 | 15.5 |

Example 18. Preparation of crystalline form D of maleate of compound of formula II

100 mg of the compound of formula II and 22 mg of maleic acid were added to 5 mL of methyl *tert*-butyl ether. The mixture was stirred at 600 rpm at 50 °C for 3 d to obtain a suspension. The suspension was filtered *in vacuo* and the residue was dried for 1 h at 40 °C to obtain a product. The product was identified as a crystalline form D of the maleate of the compound of formula II by X-ray powder diffraction, with an XRPD pattern shown in FIG. 16. The DSC pattern is shown in FIG. 17, with a first endothermic peak value at 159.27 °C; the TGA pattern is shown in FIG. 18.

Table 8. Characteristic peaks of crystalline form D of maleate of compound of formula II

| No. | 2-Theta | d(A) | I% |
|---|---|---|---|
| Peak 1 | 4.486 | 19.68368 | 65.1 |
| Peak 2 | 5.998 | 14.72391 | 35.9 |
| Peak 3 | 7.288 | 12.12061 | 48.6 |
| Peak 4 | 9.067 | 9.74591 | 49.9 |
| Peak 5 | 10.001 | 8.83770 | 50.9 |
| Peak 6 | 13.914 | 6.35938 | 59.0 |
| Peak 7 | 15.026 | 5.89121 | 34.9 |
| Peak 8 | 16.227 | 5.45786 | 27.0 |
| Peak 9 | 18.229 | 4.86287 | 100.0 |
| Peak 10 | 18.940 | 4.68174 | 84.8 |
| Peak 11 | 23.076 | 3.85108 | 58.0 |
| Peak 12 | 25.612 | 3.47535 | 60.6 |
| Peak 13 | 28.102 | 3.17274 | 12.9 |

Example 19. Preparation of crystalline form I of hydrobromide of compound of formula II

5 mL of methyl *tert*-butyl ether was added to 100 mg of the compound of formula II before 20 μL of hydrobromic acid was added. The mixture was stirred at 600 rpm at 25 °C for 3 days and filtered *in vacuo*. The residue was dried for 1 h at 40 °C to obtain a product. The product was identified as a crystalline form I of the hydrobromide of the compound of formula II by X-ray powder diffraction, with an XRPD pattern shown in FIG. 19. The DSC pattern is shown in FIG. 20, with a first endothermic peak value at 201.73 °C; the TGA pattern is shown in FIG. 21. DVS characterization: the vapor sorption of the crystalline form I of the hydrobromide at 25 °C increased along with the increase of humidity in a range of 20.0% RH to 80.0% RH, with a weight change of 0.636%, less than 2% but not less than 0.2%, indicating that the sample is slightly hygroscopic. In a normal storage condition (i.e., 60% humidity/25 °C), the vapor sorption was about 0.589%; in an accelerated test condition (i.e., 70% humidity), the vapor sorption was about 0.702%; in an extreme condition (i.e., 90% humidity), the vapor sorption was about 1.094%.

The desorption process and the sorption process of the sample basically overlapped in the process of 0-95% humidity change; the comparison of X-ray powder diffraction patterns before and after DVS showed that crystalline form did not change during DVS. The DVS pattern is shown in FIG. 22, and the comparison of X-ray powder diffraction patterns before and after DVS is shown in FIG. 23.

Table 9. Characteristic peaks of crystalline form I of hydrobromide of compound of formula II

| No. | 2-Theta | d(A) | I% |
|---|---|---|---|
| Peak 1 | 8.128 | 10.86905 | 68.5 |
| Peak 2 | 11.918 | 7.41964 | 7.5 |
| Peak 3 | 12.579 | 7.03116 | 19.0 |
| Peak 4 | 16.414 | 5.39626 | 54.1 |
| Peak 5 | 17.075 | 5.18879 | 15.6 |

(continued)

| No. | 2-Theta | d(A) | I% |
|---|---|---|---|
| Peak 6 | 17.780 | 4.98456 | 39.3 |
| Peak 7 | 18.750 | 4.72892 | 7.8 |
| Peak 8 | 19.675 | 4.50851 | 9.9 |
| Peak 9 | 20.733 | 4.28082 | 27.1 |
| Peak 10 | 21.262 | 4.17552 | 100.0 |
| Peak 11 | 23.113 | 3.84512 | 36.8 |
| Peak 12 | 23.906 | 3.71929 | 15.0 |
| Peak 13 | 24.391 | 3.64645 | 67.5 |
| Peak 14 | 26.550 | 3.35455 | 14.5 |
| Peak 15 | 28.445 | 3.13522 | 29.4 |
| Peak 16 | 28.930 | 3.08378 | 26.0 |
| Peak 17 | 29.547 | 3.02077 | 55.8 |
| Peak 18 | 30.958 | 2.88630 | 27.7 |
| Peak 19 | 32.236 | 2.77471 | 19.3 |
| Peak 20 | 33.382 | 2.68204 | 1.4 |
| Peak 21 | 38.670 | 2.32653 | 2.4 |
| Peak 22 | 39.640 | 2.27183 | 3.3 |
| Peak 23 | 40.830 | 2.20832 | 12.6 |
| Peak 24 | 42.064 | 2.14635 | 5.4 |
| Peak 25 | 43.342 | 2.08597 | 5.2 |
| Peak 26 | 46.824 | 1.93865 | 4.0 |
| Peak 27 | 48.190 | 1.88683 | 6.2 |
| Peak 28 | 48.983 | 1.85811 | 4.1 |
| Peak 29 | 50.746 | 1.79762 | 9.3 |

Example 20. Preparation of crystalline form I of hydrobromide of compound of formula II

5 mL of methyl *tert*-butyl ether was added to 100 mg of the compound of formula II before a 500 μL mixture of hydrobromic acid/ethanol (v:v = 1:50) was added. The mixture was stirred at 600 rpm at 25 °C for 3 days and filtered *in vacuo.* The residue was dried for 1 h at 40 °C to obtain a product. The product was a crystalline form I of the hydrobromide of the compound of formula II as determined by X-ray powder diffraction.

Example 21. Preparation of crystalline form I of hydrobromide of compound of formula II

5 mL of methyl tert-butyl ether was added to 100 mg of the compound of formula II before a 40 μL mixture of hydrobromic acid/ethanol (v:v = 1:1) was added. The mixture was stirred at 600 rpm at 25 °C for 3 days and filtered *in vacuo.* The residue was dried for 1 h at 40 °C to obtain a product. The product was a crystalline form I of the hydrobromide of the compound of formula II as determined by X-ray powder diffraction.

Example 22. Preparation of crystalline form I of hydrobromide of compound of formula II

15 mL of methyl *tert-butyl* ether was added to 100 mg of the compound of formula II before a 1000 μL mixture of hydrobromic acid/ethanol (v:v = 1:99) was added. The mixture was stirred at 600 rpm at 25 °C overnight and filtered *in vacuo.* The residue was dried for 1 h at 40 °C to obtain a product. The product was a crystalline form I of the hydrobromide

of the compound of formula II as determined by X-ray powder diffraction.

Example 23. Preparation of crystalline form I of hydrobromide of compound of formula II

5 mL of methyl *tert*-butyl ether was added to 100 mg of the compound of formula II before a 1000 μL mixture of hydrobromic acid/ethanol (v:v = 1:99) was added. The mixture was stirred at 600 rpm at 25 °C overnight and filtered *in vacuo*. The residue was dried for 1 h at 40 °C to obtain a product. The product was a crystalline form I of the hydrobromide of the compound of formula II as determined by X-ray powder diffraction.

Example 24. Preparation of crystalline form α of hydrochloride of compound of formula II

5 mL of methyl *tert*-butyl ether and 15.6 μL of concentrated hydrochloric acid were added to 100 mg of the compound of formula II. The mixture was stirred at 50 °C for 2 d and dried for 1 h at 40 °C to obtain a product. The product was identified as a crystalline form α of the hydrochloride of the compound of formula II by X-ray powder diffraction, with an XRPD pattern shown in FIG. 24. The DSC pattern is shown in FIG. 25, with a first endothermic peak value at 192.13 °C; the TGA pattern is shown in FIG. 26.

DVS characterization: the vapor sorption of the sample at 25 °C increased along with the increase of humidity in a range of 20.0% RH to 80.0% RH, with a weight change of 0.549%, less than 2% but not less than 0.2%, indicating that the sample is slightly hygroscopic. In a normal storage condition (i.e., 60% humidity/25 °C), the vapor sorption was about 0.463%; in an accelerated test condition (i.e., 70% humidity), the vapor sorption was about 0.574%; in an extreme condition (i.e., 90% humidity), the vapor sorption was about 1.040%.

The desorption process and the sorption process of the sample basically overlapped in the process of 0-95% humidity change; the comparison of X-ray powder diffraction patterns before and after DVS showed that crystalline form did not change during DVS. The DVS pattern is shown in FIG. 27, and the comparison of X-ray powder diffraction patterns before and after DVS is shown in FIG. 28.

Table 10. Characteristic peaks of crystalline form α of hydrochloride of compound of formula II

| No. | 2-Theta | d(A) | I% |
|---|---|---|---|
| Peak 1 | 7.931 | 11.13829 | 53.4 |
| Peak 2 | 10.115 | 8.73832 | 19.9 |
| Peak 3 | 12.166 | 7.26910 | 15.4 |
| Peak 4 | 13.920 | 6.35673 | 24.2 |
| Peak 5 | 15.224 | 5.81523 | 33.9 |
| Peak 6 | 16.041 | 5.52078 | 13.9 |
| Peak 7 | 16.315 | 5.42854 | 12.6 |
| Peak 8 | 16.748 | 5.28930 | 12.2 |
| Peak 9 | 17.425 | 5.08526 | 21.3 |
| Peak 10 | 18.309 | 4.84177 | 64.7 |
| Peak 11 | 19.624 | 4.52003 | 15.9 |
| Peak 12 | 20.235 | 4.38496 | 19.6 |
| Peak 13 | 21.491 | 4.13138 | 36.6 |
| Peak 14 | 22.340 | 3.97642 | 67.3 |
| Peak 15 | 23.359 | 3.80507 | 65.0 |
| Peak 16 | 23.905 | 3.71950 | 48.0 |
| Peak 17 | 24.570 | 3.62032 | 100.0 |
| Peak 18 | 25.320 | 3.51464 | 20.1 |
| Peak 19 | 25.811 | 3.44896 | 13.9 |
| Peak 20 | 26.096 | 3.41194 | 10.5 |

(continued)

| No. | 2-Theta | d(A) | I% |
|---|---|---|---|
| Peak 21 | 27.624 | 3.22652 | 24.8 |
| Peak 22 | 28.213 | 3.16057 | 24.6 |
| Peak 23 | 29.190 | 3.05697 | 5.1 |
| Peak 24 | 29.760 | 2.99971 | 2.4 |
| Peak 25 | 31.266 | 2.85855 | 10.1 |
| Peak 26 | 31.795 | 2.81217 | 5.5 |
| Peak 27 | 32.324 | 2.76732 | 8.1 |
| Peak 28 | 35.906 | 2.49902 | 3.7 |
| Peak 29 | 37.291 | 2.40939 | 8.3 |

Example 25. Preparation of crystalline form β of hydrochloride of compound of formula II

5 mL of methyl *tert-butyl* ether and a 0.6 mL solution of concentrated hydrochloric acid in ethanol (0.1 mL of concentrated hydrochloric acid was added to 9.9 mL of ethanol and the mixture was well mixed) were added to 100 mg of the compound of formula II. The mixture was stirred at 25 °C for 1 h and at 50 °C for 2 d, and filtered *in vacuo.* The residue was dried for 2 h at 40 °C to obtain a product. The product was identified as a crystalline form β of the hydrochloride of the compound of formula II by X-ray powder diffraction, with an XRPD pattern shown in FIG. 29. The DSC pattern is shown in FIG. 30, with a first endothermic peak value at 194.04 °C; the TGA pattern is shown in FIG. 31.

DVS characterization: the vapor sorption of the sample at 25 °C increased along with the increase of humidity in a range of 20.0% RH to 80.0% RH, with a weight change of 1.235%, less than 2% but not less than 0.2%, indicating that the sample is slightly hygroscopic. In a normal storage condition (i.e., 60% humidity/25 °C), the vapor sorption was about 1.755%; in an accelerated test condition (i.e., 70% humidity), the vapor sorption was about 1.954%; in an extreme condition (i.e., 90% humidity), the vapor sorption was about 2.534%.

The desorption process and the sorption process of the sample basically overlapped in individual processes of 0-95% humidity change, but the desorption process and the sorption process of the first and second cycles could not overlap; the comparison of X-ray powder diffraction patterns before and after DVS showed that crystalline form changed during DVS. The DVS pattern is shown in FIG. 32, and the comparison of X-ray powder diffraction patterns before and after DVS is shown in FIG. 33.

Table 11. Characteristic peaks of crystalline form β of hydrochloride of compound of formula II

| No. | 2-Theta | d(A) | I% |
|---|---|---|---|
| Peak 1 | 5.386 | 16.39621 | 39.0 |
| Peak 2 | 8.191 | 10.78576 | 77.6 |
| Peak 3 | 10.818 | 8.17156 | 29.1 |
| Peak 4 | 12.688 | 6.97098 | 42.8 |
| Peak 5 | 13.980 | 6.32982 | 20.9 |
| Peak 6 | 14.915 | 5.93499 | 26.6 |
| Peak 7 | 16.607 | 5.33388 | 19.7 |
| Peak 8 | 18.076 | 4.90345 | 12.8 |
| Peak 9 | 19.056 | 4.65352 | 12.9 |
| Peak 10 | 20.036 | 4.42814 | 20.8 |
| Peak 11 | 21.372 | 4.15427 | 100.0 |
| Peak 12 | 22.040 | 4.02986 | 35.2 |
| Peak 13 | 23.465 | 3.78826 | 25.0 |

(continued)

| No. | 2-Theta | d(A) | I% |
|---|---|---|---|
| Peak 14 | 24.355 | 3.65171 | 28.5 |
| Peak 15 | 25.869 | 3.44132 | 36.7 |
| Peak 16 | 26.582 | 3.35068 | 21.8 |
| Peak 17 | 27.383 | 3.25440 | 12.0 |
| Peak 18 | 29.253 | 3.05045 | 29.5 |
| Peak 19 | 29.832 | 2.99256 | 39.9 |
| Peak 20 | 30.946 | 2.88740 | 22.9 |
| Peak 21 | 31.480 | 2.83959 | 41.4 |
| Peak 22 | 32.504 | 2.75242 | 7.6 |
| Peak 23 | 33.439 | 2.67755 | 8.5 |

Example 26. Preparation of crystalline form γ of hydrochloride of compound of formula II

A small amount of the crystalline form β of the hydrochloride of the compound of formula II was loaded on a DVS system and subjected to a detection with parameters of dm/dt=0.002, 50-95-0-95-50% RH, Max 360 min, 25 °C. The product was identified as a crystalline form β of the hydrochloride of the compound of formula II by X-ray powder diffraction, with an XRPD pattern shown in FIG. 34.

Table 12. Characteristic peaks of crystalline form γ of hydrochloride of compound of formula II

| No. | 2-Theta | d(A) | I% |
|---|---|---|---|
| Peak 1 | 8.114 | 10.88737 | 89.0 |
| Peak 2 | 11.997 | 7.37097 | 42.5 |
| Peak 3 | 12.640 | 6.99748 | 77.7 |
| Peak 4 | 13.772 | 6.42501 | 45.7 |
| Peak 5 | 16.478 | 5.37549 | 25.8 |
| Peak 6 | 17.897 | 4.95221 | 45.6 |
| Peak 7 | 19.671 | 4.50933 | 9.7 |
| Peak 8 | 20.337 | 4.36327 | 52.8 |
| Peak 9 | 21.422 | 4.14464 | 79.1 |
| Peak 10 | 22.156 | 4.00903 | 62.3 |
| Peak 11 | 23.228 | 3.82632 | 79.1 |
| Peak 12 | 24.472 | 3.63450 | 100.0 |
| Peak 13 | 25.882 | 3.43968 | 26.9 |
| Peak 14 | 27.567 | 3.23307 | 27.4 |
| Peak 15 | 28.277 | 3.15351 | 14.1 |
| Peak 16 | 29.830 | 2.99281 | 38.1 |
| Peak 17 | 31.160 | 2.86797 | 15.9 |
| Peak 18 | 32.269 | 2.77189 | 17.0 |
| Peak 19 | 33.334 | 2.68576 | 13.6 |

Example 27. Preparation of crystalline form of acetate of compound of formula II

1 mL of water was added to 20 mg of the compound of formula II before a solution of acetic acid in ethanol (0.1 mL of acetic acid was added to 9.9 mL of ethanol and the mixture was well mixed) was added in a molar ratio of 1:1. The mixture was stirred overnight at 50 °C and filtered *in vacuo*. The residue was dried for 1 h at 40 °C to obtain a product. The product was identified as a crystalline form of the acetate of the compound of formula II by X-ray powder diffraction, with an XRPD pattern shown in FIG. 35. The DSC pattern is shown in FIG. 36, with a first endothermic peak value at 206.96 °C, a first exothermic peak value at 213.49 °C and a second endothermic peak value at 246.30 °C.

Table 13. Characteristic peaks of crystalline form of acetate of compound of formula II

| No. | 2-Theta | d(A) | I% |
|---|---|---|---|
| Peak 1 | 11.651 | 7.58948 | 96.9 |
| Peak 2 | 12.495 | 7.07863 | 38.2 |
| Peak 3 | 14.323 | 6.17872 | 29.8 |
| Peak 4 | 15.121 | 5.85472 | 28.9 |
| Peak 5 | 15.636 | 5.66274 | 100.0 |
| Peak 6 | 15.965 | 5.54704 | 66.5 |
| Peak 7 | 18.075 | 4.90394 | 58.9 |
| Peak 8 | 19.247 | 4.60782 | 20.4 |
| Peak 9 | 19.903 | 4.45730 | 25.1 |
| Peak 10 | 20.935 | 4.23993 | 38.5 |
| Peak 11 | 22.107 | 4.01768 | 29.1 |
| Peak 12 | 22.998 | 3.86402 | 47.4 |
| Peak 13 | 23.842 | 3.72910 | 52.3 |
| Peak 14 | 24.733 | 3.59676 | 59.9 |
| Peak 15 | 25.530 | 3.48623 | 35.2 |
| Peak 16 | 26.843 | 3.31862 | 11.7 |
| Peak 17 | 28.719 | 3.10600 | 12.9 |
| Peak 18 | 29.750 | 3.00061 | 6.1 |
| Peak 19 | 30.829 | 2.89805 | 18.2 |
| Peak 20 | 32.142 | 2.78260 | 14.6 |
| Peak 21 | 35.143 | 2.55155 | 3.6 |
| Peak 22 | 39.973 | 2.25369 | 2.6 |

Example 28. Influencing factor study of crystalline form α of hydrochloride and crystalline form I of hydrobromide of compound of formula II

Sample of the crystalline form α of the hydrochloride and the crystalline form I of the hydrobromide of the compound of formula II were let stand open to examine the stability of the samples in conditions of illumination (4500 Lux), high temperature (40 °C and 60 °C) and high humidity (RH 75% and RH 92.5%) in a period of 30 days.

Table 14. Stability data of the influencing factor study

| Conditions | Time (days) | Crystalline form α of hydrochloride of compound of formula II | | | | |
|---|---|---|---|---|---|---|
| | | Color and appearance | Main peak purity (%) | Chiral purity (%) | Chloride ion content (%) | Crystalline form |
| Initial | 0 | White solid | 96.925 | 99.14 | 5.75 | Crystalline form α |
| 40°C | 7 | White solid | 96.915 | 99.131 | / | Not changed |
| | 14 | White solid | 96.921 | 99.115 | / | Not changed |
| | 30 | White solid | 96.824 | 99.207 | 5.75 | Not changed |
| 60°C | 7 | White solid | 96.884 | 99.136 | / | Not changed |
| | 14 | White solid | 96.907 | 99.106 | / | Not changed |
| | 30 | White solid | 96.814 | 99.232 | 5.65 | Not changed |
| 75% RH | 7 | White solid | 96.916 | 99.138 | / | Not changed |
| | 14 | White solid | 96.908 | 99.130 | / | Not changed |
| | 30 | White solid | 96.903 | 99.220 | 5.68 | Not changed |
| 92.5% RH | 7 | White solid | 96.912 | 99.132 | / | Not changed |
| | 14 | White solid | 96.908 | 99.111 | / | Not changed |
| | 30 | White solid | 96.880 | 99.211 | 5.67 | Not changed |
| 4500 Lux | 7 | White solid | 96.911 | 99.137 | / | Not changed |
| | 14 | White solid | 96.911 | 99.139 | / | Not changed |
| | 30 | White solid | 96.810 | 99.216 | 5.71 | Not changed |
| Conditions | Time (days) | Crystalline form I of hydrobromide of compound of formula II | | | | |
| | | Color and appearance | Main peak purity (%) | Chiral purity (%) | Bromide ion content (%) | Crystalline form |
| Initial | 0 | White solid | 97.689 | 99.298 | 11.75 | Crystalline form I |
| 40°C | 7 | White solid | 97.716 | 99.293 | / | Not changed |
| | 14 | White solid | 97.727 | 99.165 | / | Not changed |
| | 30 | White solid | 97.644 | 99.251 | 11.73 | Not changed |
| 60°C | 7 | White solid | 97.645 | 99.299 | / | Not changed |
| | 14 | White solid | 97.671 | 99.187 | / | Not changed |
| | 30 | White solid | 97.630 | 99.263 | 11.50 | Not changed |
| 75% RH | 7 | White solid | 97.687 | 99.293 | / | Not changed |
| | 14 | White solid | 97.705 | 99.179 | / | Not changed |
| | 30 | White solid | 97.676 | 99.274 | 11.73 | Not changed |
| 92.5% RH | 7 | White solid | 97.706 | 99.294 | / | Not changed |
| | 14 | White solid | 97.701 | 99.175 | / | Not changed |
| | 30 | White solid | 97.653 | 99.268 | 11.55 | Not changed |
| 4500 Lux | 7 | White solid | 97.683 | 99.297 | / | Not changed |
| | 14 | White solid | 97.660 | 99.152 | / | Not changed |
| | 30 | White solid | 97.626 | 99.243 | 11.18 | Not changed |

Conclusions: the influencing factor study showed that: the crystalline form α of the hydrochloride and the crystalline form I of the hydrobromide of the compound of formula II have good physical and chemical stabilities in conditions of illumination, high temperatures of 40 °C and 60 °C and high humidities of 75% and 92.5%.

Example 29. Long-term/accelerated stability study of crystalline form α of hydrochloride and crystalline form I of hydrobromide of compound of formula II

The crystalline form α of the hydrochloride of the compound of formula II was let stand in conditions of 25 °C/60% RH and 40 °C/75% RH to examine its stability.

Table 15. Long term/accelerated stability study data for the crystalline form α of the hydrochloride of the compound of formula II

| Conditions | Time | Crystalline form α of hydrochloride of compound of formula II | | | | |
|---|---|---|---|---|---|---|
| | | Color and appearance | Main peak purity (%) | Chiral purity (%) | Chloride ion content (%) | Crystalline form |
| Initial | 0 | White solid | 96.925 | 99.14 | 5.75 | Crystalline form α |
| 25 °C, 60%RH | Day 7 | White solid | 96.915 | 99.131 | / | Not changed |
| | Day 14 | White solid | 96.831 | 99.111 | / | Not changed |
| | Month 1 | White solid | 96.906 | 99.225 | 5.75 | Not changed |
| | Month 2 | White solid | 96.883 | 99.332 | 5.74 | Not changed |
| | Month 3 | White solid | 96.749 | 99.239 | / | Not changed |
| | Month 6 | White solid | 96.170 | 99.187 | 5.85 | Not changed |
| 40°C, 75%RH | Day 7 | White solid | 96.906 | 99.137 | / | Not changed |
| | Day 14 | White solid | 96.902 | 99.104 | / | Not changed |
| | Month 1 | White solid | 96.846 | 99.206 | 5.44 | Not changed |
| | Month 2 | White solid | 96.780 | 99.292 | 5.86 | Not changed |
| | Month 3 | White solid | 96.664 | 99.149 | / | Not changed |
| | Month 6 | White solid | 95.990 | 98.972 | 5.98 | Not changed |

The long-term/accelerated stability study showed that: the crystalline form α of the hydrochloride of the compound of formula II has good physical and chemical stability in conditions of a long term and acceleration in a period of 6 months.

The crystalline form I of the hydrobromide of the compound of formula II was let stand in conditions of 25 °C/60% RH and 40 °C/75% RH to examine its stability.

Table 16. Long term/accelerated stability study data for the crystalline form I of the hydrobromide of the compound of formula II

| Conditions | Time | Crystalline form I of hydrobromide of compound of formula II | | | | |
|---|---|---|---|---|---|---|
| | | Color and appearance | Main peak purity (%) | Chiral purity (%) | Bromide ion content (%) | Crystalline form |
| Initial | 0 | White solid | 97.689 | 99.298 | 11.75 | Crystalline form I |
| 25 °C, 60%RH | Day 7 | White solid | 97.689 | 99.297 | / | Not changed |
| | Day 14 | White solid | 97.697 | 99.198 | / | Not changed |
| | Month 1 | White solid | 97.649 | 99.255 | 11.20 | Not changed |
| | Month 2 | White solid | 97.661 | 99.363 | 11.05 | Not changed |
| | Month 3 | White solid | 97.552 | 99.283 | / | Not changed |
| | Month 6 | White solid | 96.981 | 99.228 | 11.82 | Not changed |

(continued)

| Conditions | Time | Crystalline form I of hydrobromide of compound of formula II | | | | |
|---|---|---|---|---|---|---|
| | | Color and appearance | Main peak purity (%) | Chiral purity (%) | Bromide ion content (%) | Crystalline form |
| 40°C, 75%RH | Day 7 | White solid | 97.661 | 99.295 | / | Not changed |
| | Day 14 | White solid | 97.660 | 99.174 | / | Not changed |
| | Month 1 | White solid | 97.558 | 99.262 | 11.49 | Not changed |
| | Month 2 | White solid | 97.561 | 99.328 | 10.93 | Not changed |
| | Month 3 | White solid | 97.259 | 99.275 | / | Not changed |
| | Month 6 | White solid | 96.412 | 99.199 | 11.91 | Not changed |

The long-term/accelerated stability study showed that: the crystalline form I of the hydrobromide of the compound of formula II has good physical and chemical stability in conditions of a long term and acceleration in a period of 6 months.

## Claims

1. An acid addition salt of a compound of formula II or a pharmaceutically acceptable solvate of the acid addition salt, wherein the acid addition salt is an organic acid addition salt or an inorganic acid addition salt,

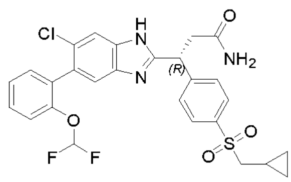

formula II.

2. The acid addition salt of the compound of formula II or the pharmaceutically acceptable solvate of the acid addition salt according to claim 1, wherein the organic acid addition salt is selected from the group consisting of benzoate, oxalate, methanesulfonate, maleate and acetate, and the inorganic acid addition salt is selected from the group consisting of hydrobromide and hydrochloride.

3. The acid addition salt of the compound of formula II or the pharmaceutically acceptable solvate of the acid addition salt according to claim 2, wherein the benzoate, oxalate and methanesulfonate are amorphous.

4. The acid addition salt of the compound of formula II or the pharmaceutically acceptable solvate of the acid addition salt according to claim 2, wherein the benzoate is a crystalline form having an X-ray powder diffraction pattern with characteristic peaks at diffraction angles 2θ of 5.305 and 7.411.

5. The acid addition salt of the compound of formula II or the pharmaceutically acceptable solvate of the acid addition salt according to claim 2, wherein the oxalate is a crystalline form having an X-ray powder diffraction pattern with characteristic peaks at diffraction angles 2θ of 14.378, 18.463 and 21.670.

6. The acid addition salt of the compound of formula II or the pharmaceutically acceptable solvate of the acid addition salt according to claim 2, wherein the maleate is a crystalline form B having an X-ray powder diffraction pattern with characteristic peaks at diffraction angles 2θ of 7.624, 9.659, 13.815, 15.844 and 17.391.

7. The acid addition salt of the compound of formula II or the pharmaceutically acceptable solvate of the acid addition salt according to claim 2, wherein the maleate is a crystalline form C having an X-ray powder diffraction pattern with characteristic peaks at diffraction angles 2θ of 7.325, 8.635, 9.809, 13.649, 16.133, 16.765 and 18.346.

8. The acid addition salt of the compound of formula II or the pharmaceutically acceptable solvate of the acid addition salt according to claim 2, wherein the maleate is a crystalline form D having an X-ray powder diffraction pattern with characteristic peaks at diffraction angles 2θ of 4.486, 7.288, 9.067, 10.001, 13.914, 18.229 and 18.940.

9. The acid addition salt of the compound of formula II or the pharmaceutically acceptable solvate of the acid addition salt according to claim 2, wherein the hydrobromide is a crystalline form I having an X-ray powder diffraction pattern with characteristic peaks at diffraction angles 2θ of 8.128, 12.579, 16.414, 17.075, 17.780 and 20.733.

10. The acid addition salt of the compound of formula II or the pharmaceutically acceptable solvate of the acid addition salt according to claim 2, wherein the hydrobromide is a crystalline form I having an X-ray powder diffraction pattern with characteristic peaks at diffraction angles 2θ of 8.128, 12.579, 16.414, 17.075, 17.780, 19.675, 20.733, 21.262, 23.113, 23.906, 24.391, 26.550, 28.445, 28.930 and 29.547.

11. The acid addition salt of the compound of formula II or the pharmaceutically acceptable solvate of the acid addition salt according to claim 2, wherein the hydrobromide is a crystalline form I having an X-ray powder diffraction pattern with characteristic peaks at diffraction angles 2θ of 8.128, 11.918, 12.579, 16.414, 17.075, 17.780, 18.750, 19.675, 20.733, 21.262, 23.113, 23.906, 24.391, 26.550, 28.445, 28.930, 29.547, 30.958, 32.236, 33.382, 38.670, 39.640, 40.830, 42.064, 43.342, 46.824, 48.190, 48.983 and 50.746.

12. The acid addition salt of the compound of formula II or the pharmaceutically acceptable solvate of the acid addition salt according to claim 2, wherein the hydrochloride is a crystalline form α having an X-ray powder diffraction pattern with characteristic peaks at diffraction angles 2θ of 7.931, 10.115, 13.920, 15.224, 17.425 and 18.309.

13. The acid addition salt of the compound of formula II or the pharmaceutically acceptable solvate of the acid addition salt according to claim 2, wherein the hydrochloride is a crystalline form β having an X-ray powder diffraction pattern with characteristic peaks at diffraction angles 2θ of 5.386, 8.191, 12.688, 16.607 and 20.036.

14. The acid addition salt of the compound of formula II or the pharmaceutically acceptable solvate of the acid addition salt according to claim 2, wherein the hydrochloride is a crystalline form γ having an X-ray powder diffraction pattern with characteristic peaks at diffraction angles 2θ of 8.114, 11.997, 12.640, 13.772, 16.478, 17.897 and 20.337.

15. The acid addition salt of the compound of formula II or the pharmaceutically acceptable solvate of the acid addition salt according to claim 2, wherein the acetate is a crystalline form having an X-ray powder diffraction pattern with characteristic peaks at diffraction angles 2θ of 11.651, 12.495, 15.636, 15.965, 18.075 and 20.935.

16. The acid addition salt of the compound of formula II or the pharmaceutically acceptable solvate of the acid addition salt according to any of claims 4 to 15, wherein the 2θ values of the crystalline forms have an error range of ±0.2.

17. A method for preparing the crystalline form I of the hydrobromide of the compound of formula II according to any of claims 9 to 11, comprising: precipitating a crystal by reacting the compound of formula II with hydrobromic acid in a proper amount of a solvent selected from the group consisting of one or more of a hydrocarbon solvent, an ether solvent, an alcohol solvent, an ester solvent, a ketone solvent, a nitrile solvent, a halogenated hydrocarbon solvent, a nitrogenous solvent, water and dimethyl sulfoxide, wherein

the hydrocarbon solvent includes, but is not limited to, *n*-butane, *n*-pentane, *n*-hexane or *n*-heptane;
the ether solvent includes, but is not limited to, tetrahydrofuran, diethyl ether, propylene glycol methyl ether, methyl *tert*-butyl ether, isopropyl ether or 1,4-dioxane;
the alcohol solvent includes, but is not limited to, methanol, ethanol, isopropanol, *n*-propanol, isoamyl alcohol or trifluoroethanol;
the ester solvent includes, but is not limited to, ethyl acetate, isopropyl acetate or butyl acetate; the ketone solvent includes, but is not limited to, acetone, acetophenone or 4-methyl-2-pentanone;
the nitrile solvent includes, but is not limited to, acetonitrile or propionitrile;
the halogenated hydrocarbon solvent includes, but is not limited to, chloromethane, dichloromethane, chloroform or carbon tetrachloride;

the nitrogenous solvent includes, but is not limited to, nitromethane, *N*,*N*-dimethylformamide or *N*,*N*-dimethylacetamide.

**18.** The method according to claim 17, wherein the solvent is methyl *tert*-butyl ether and ethanol.

**19.** A pharmaceutical composition comprising the acid addition salt or the pharmaceutically acceptable solvate of the acid addition salt according to any of claims 1 to 16, and one or more pharmaceutically acceptable carriers, diluents or excipients.

**20.** A pharmaceutical composition comprising an amorphous form of a compound of formula II, and one or more pharmaceutically acceptable carriers, diluents or excipients,

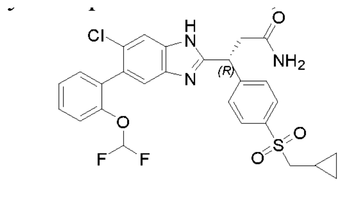

formula II.

**21.** A pharmaceutical composition comprising the crystalline form I of the hydrobromide according to any of claims 9 to 11, and one or more pharmaceutically acceptable carriers, diluents or excipients.

**22.** A pharmaceutical composition prepared from the acid addition salt of the compound of formula II or the pharmaceutically acceptable solvate of the acid addition salt according to any of claims 1 to 16 and one or more pharmaceutically acceptable carriers, diluents or excipients.

**23.** A pharmaceutical composition prepared from an amorphous form of a compound of formula II and one or more pharmaceutically acceptable carriers, diluents or excipients,

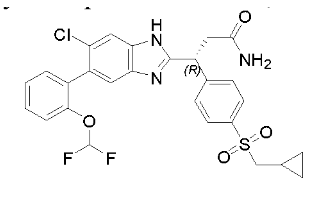

formula II.

**24.** A pharmaceutical composition prepared from the crystalline form I of the hydrobromide according to any of claims 9 to 11 and one or more pharmaceutically acceptable carriers, diluents or excipients.

**25.** A method for preparing a pharmaceutical composition, comprising: mixing the acid addition salt of the compound of formula II or the pharmaceutically acceptable solvate of the acid addition salt according to any of claims 1 to 16 with at least one pharmaceutically acceptable carrier, diluent or excipient.

**26.** A method for preparing a pharmaceutical composition, comprising: mixing an amorphous form of a compound of formula II with at least one pharmaceutically acceptable carrier, diluent or excipient,

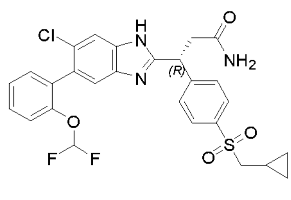

formula II.

**27.** Use of the acid addition salt of the compound of formula II or the pharmaceutically acceptable solvate of the acid addition salt according to any of claims 1 to 16 or the pharmaceutical composition according to any of claims 19 to 24 in preparing a medicament for treating a disease or disorder mediated by RORγ.

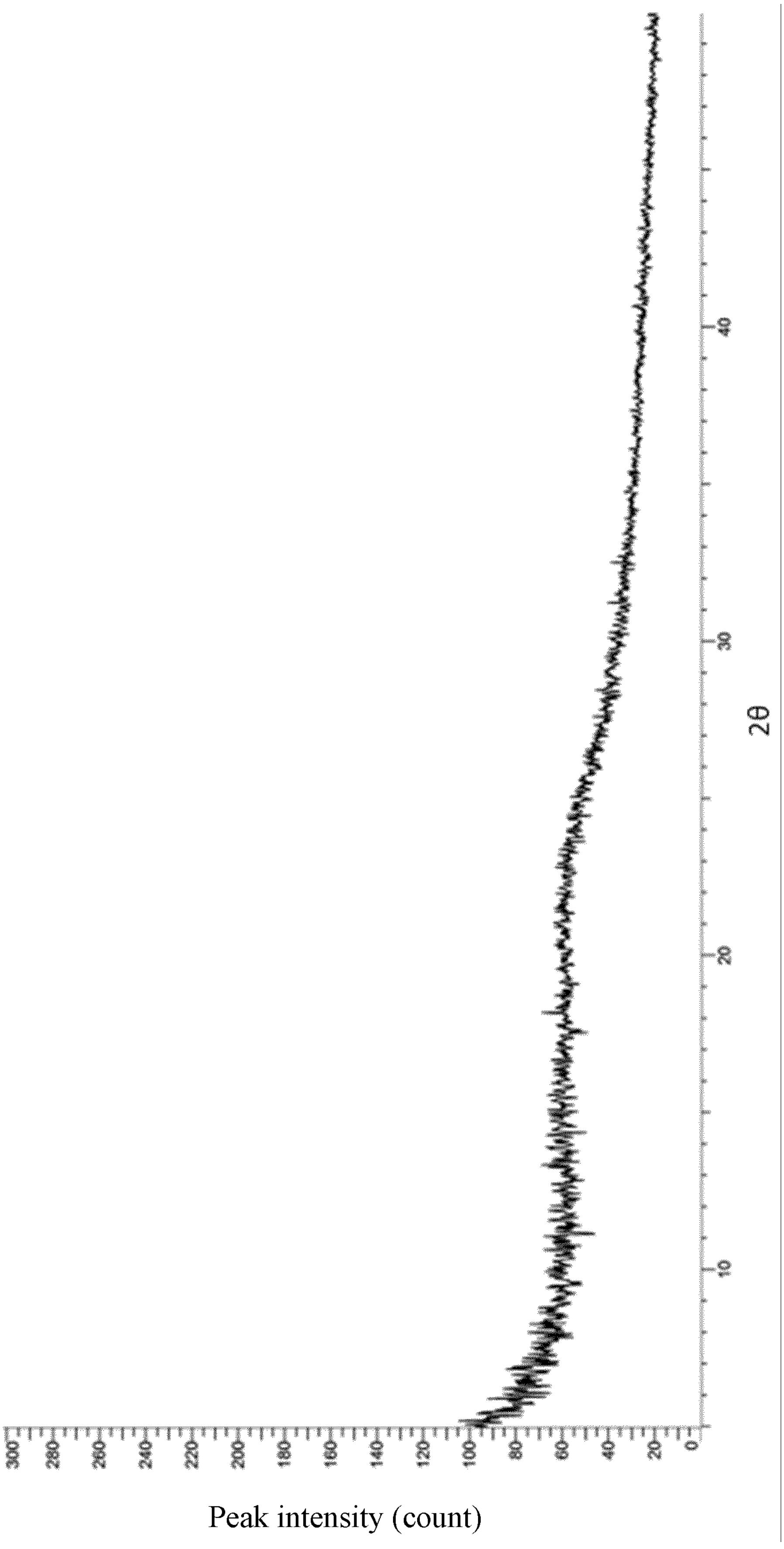
Peak intensity (count)
2θ
300
280
260
240
220
200
180
160
140
120
100
80
60
40
20
0
10
20
30
40

FIG. 1

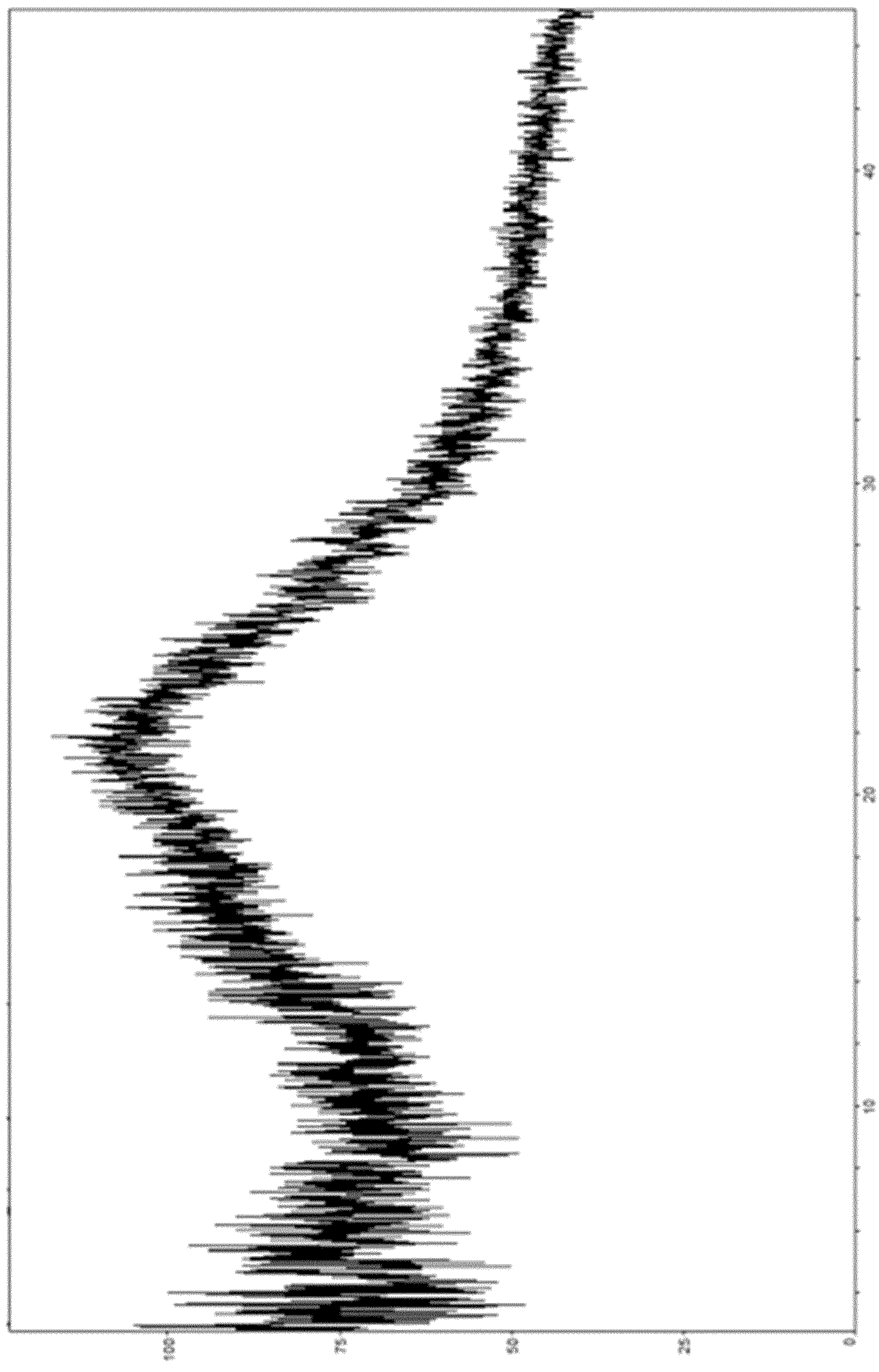


FIG. 2

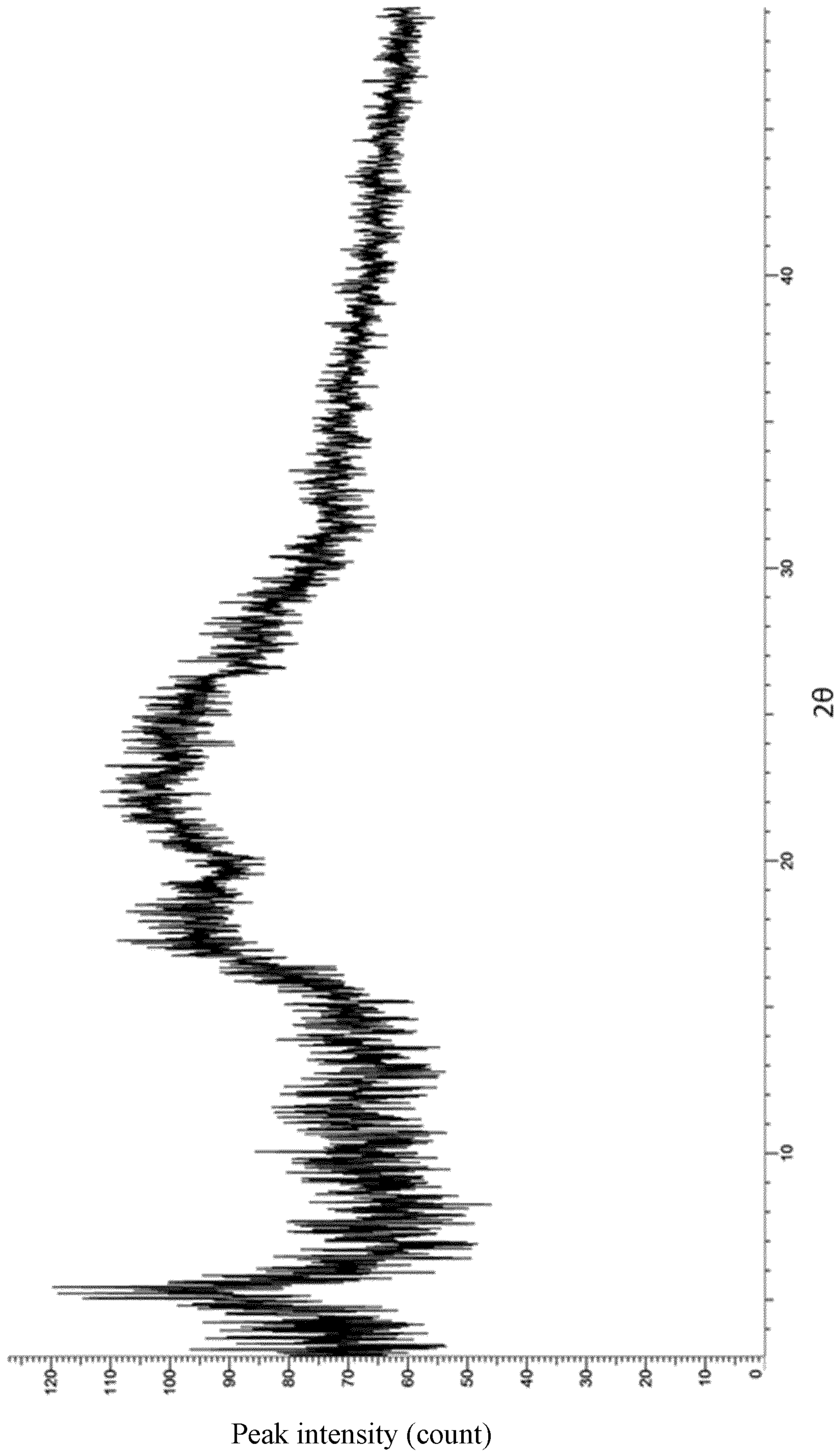
0
10
20
30
40
50
60
70
80
90
100
110
120
Peak intensity (count)
10
20
30
40
2θ

FIG. 3

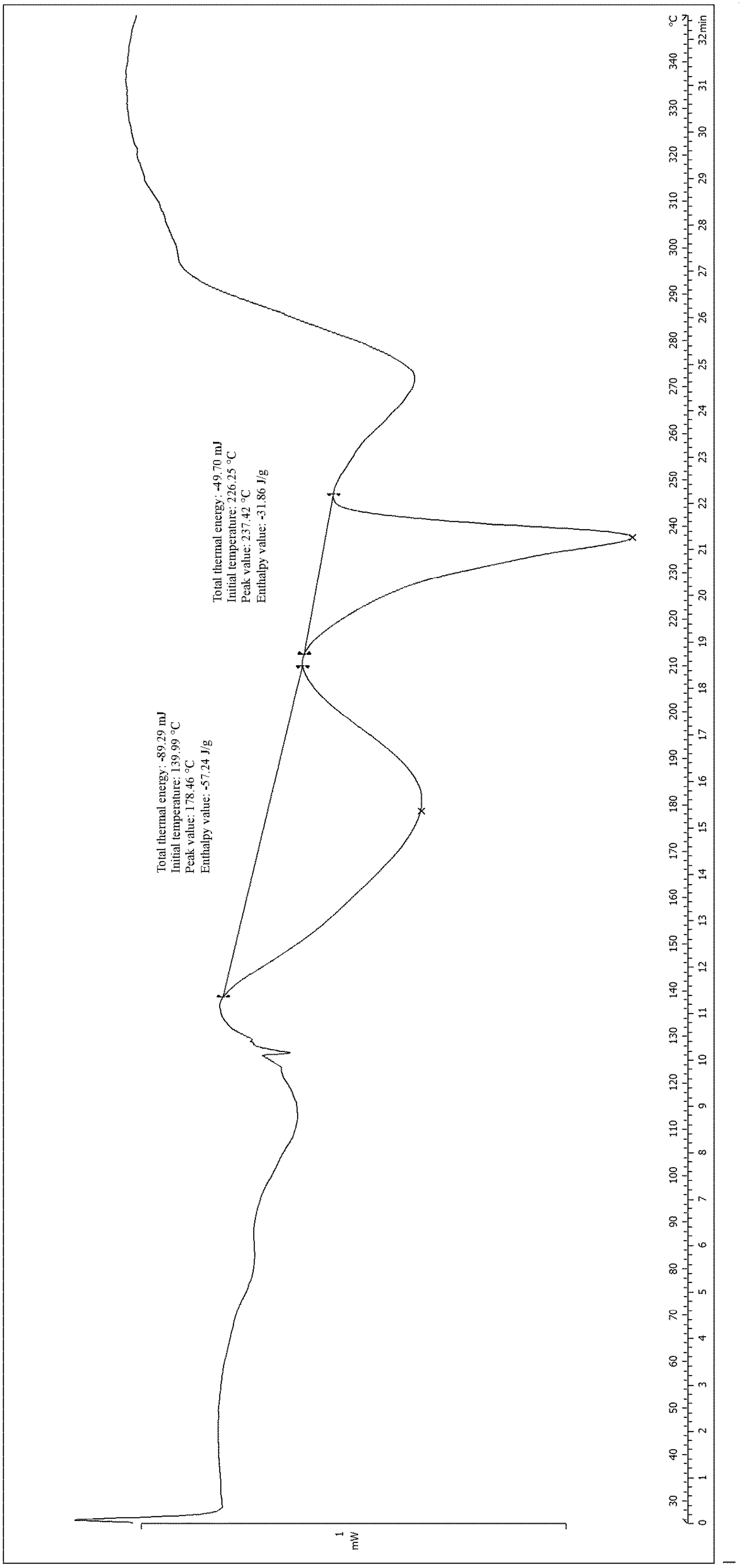
Total thermal energy: -49.70 mJ
Initial temperature: 226.25 °C
Peak value: 237.42 °C
Enthalpy value: -31.86 J/g
Total thermal energy: -89.29 mJ
Initial temperature: 139.99 °C
Peak value: 178.46 °C
Enthalpy value: -57.24 J/g
1 mW
°C
32 min

FIG. 4

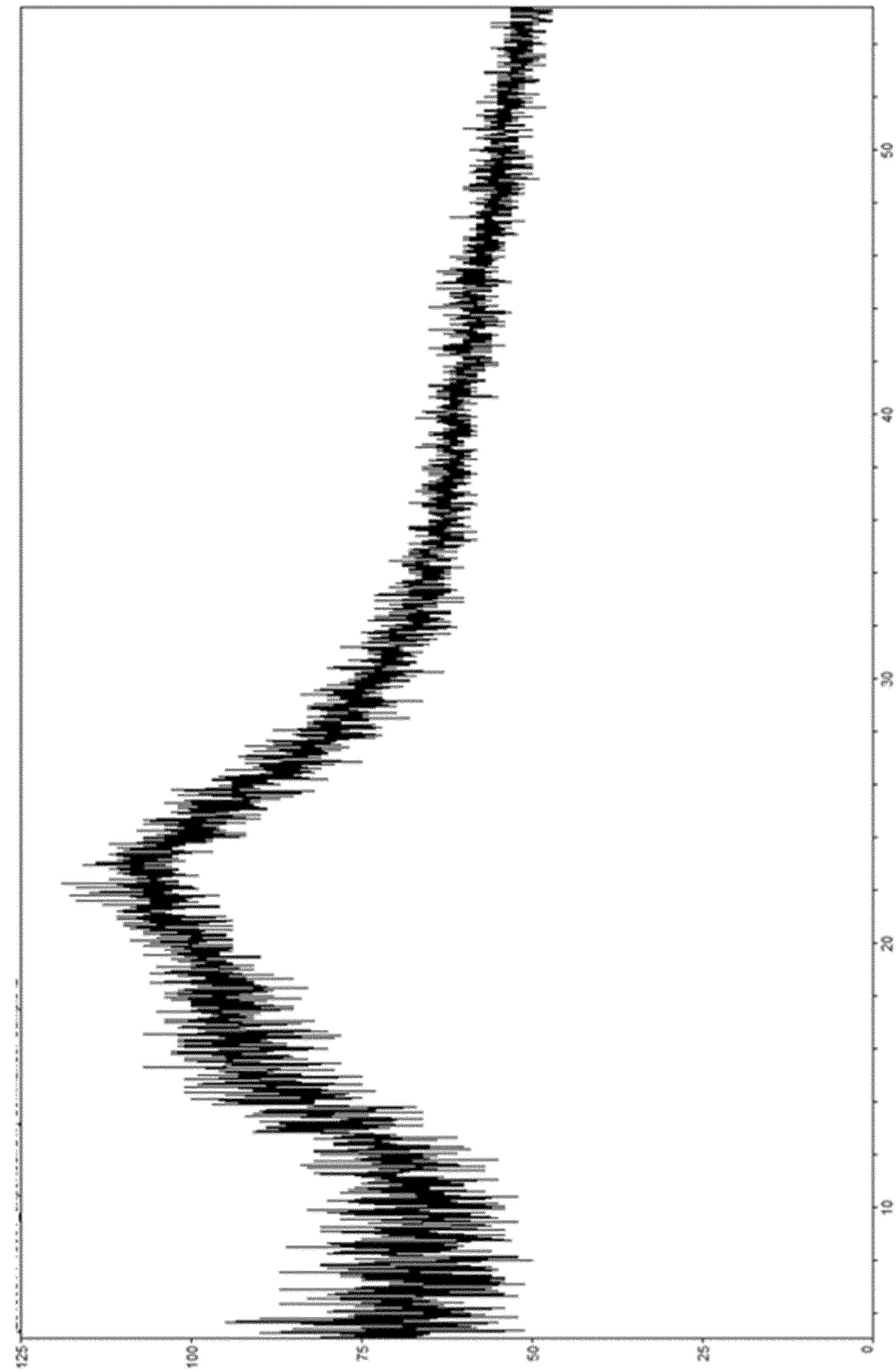


FIG. 5

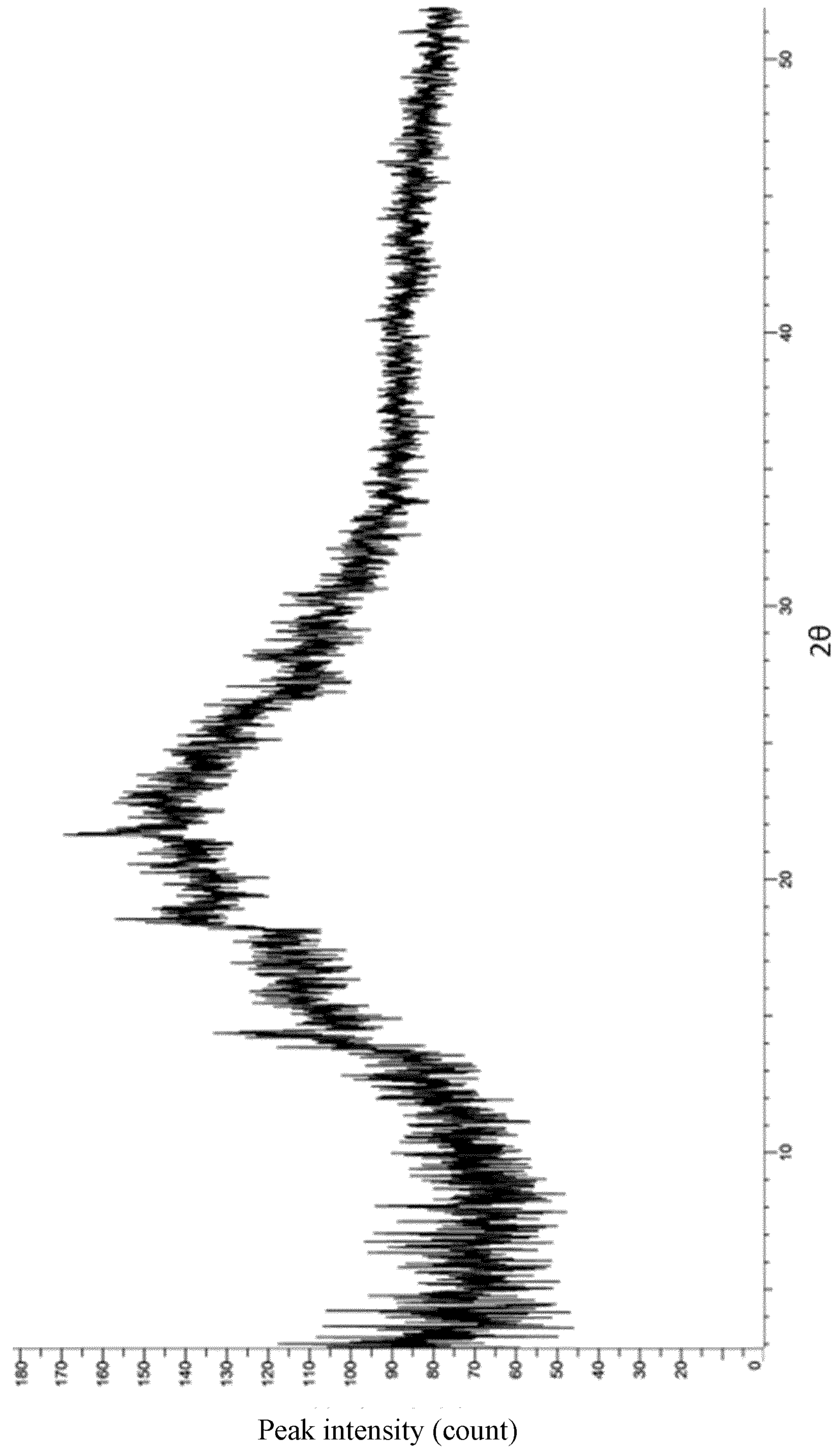
Peak intensity (count)
180
170
160
150
140
130
120
110
100
90
80
70
60
50
40
30
20
0
10
20
30
40
50
2θ

FIG. 6

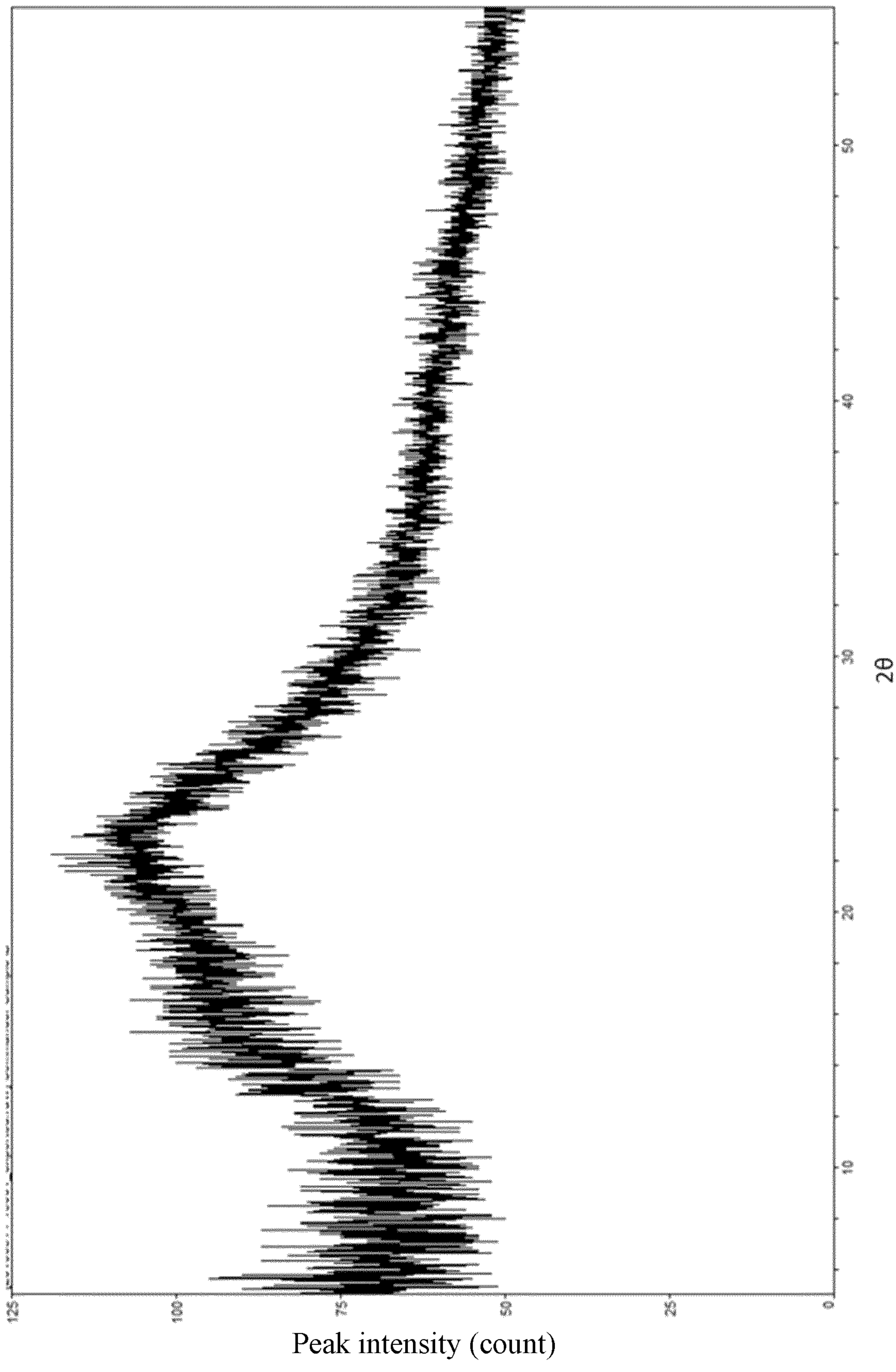


FIG. 7

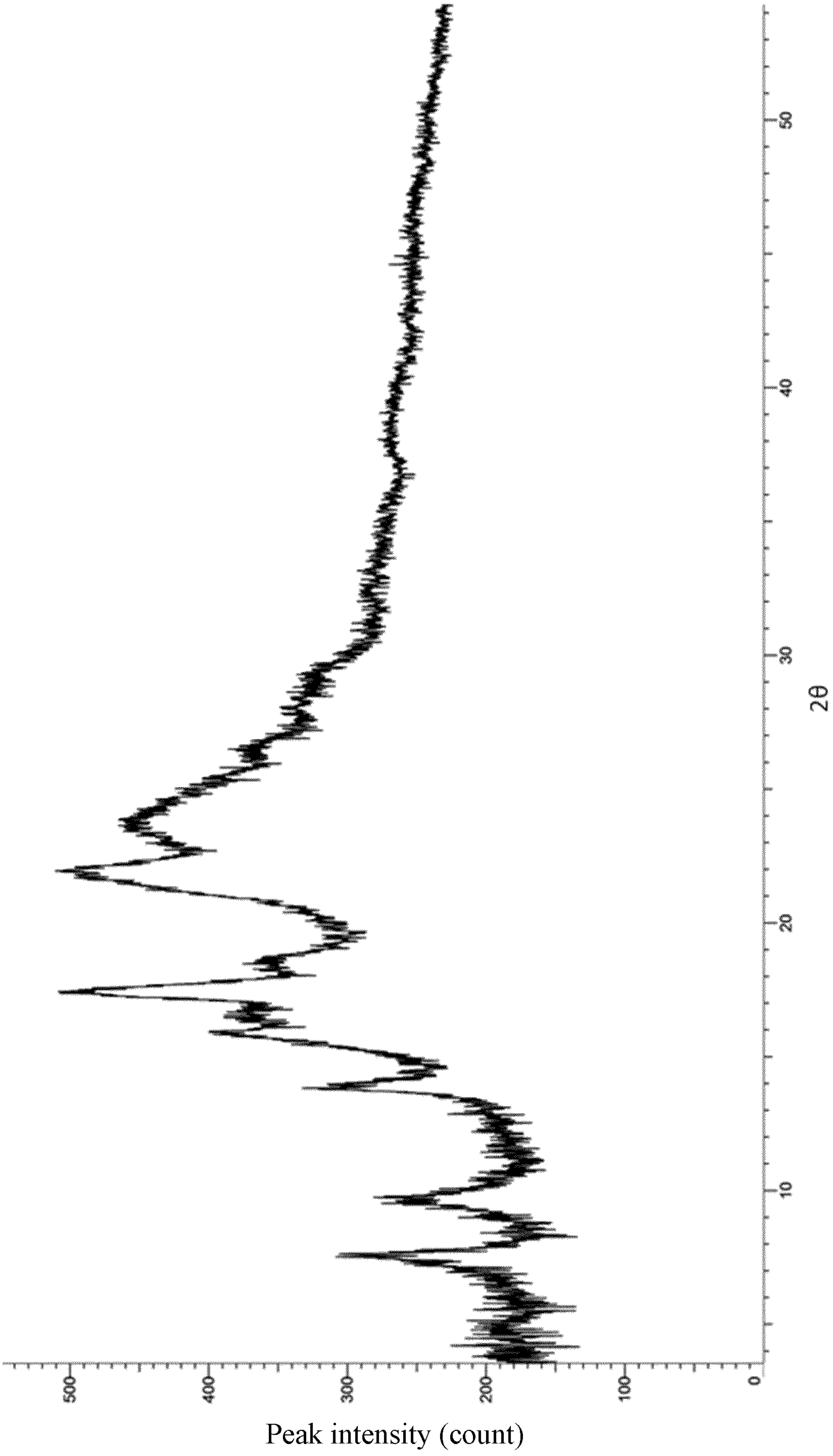


FIG. 8

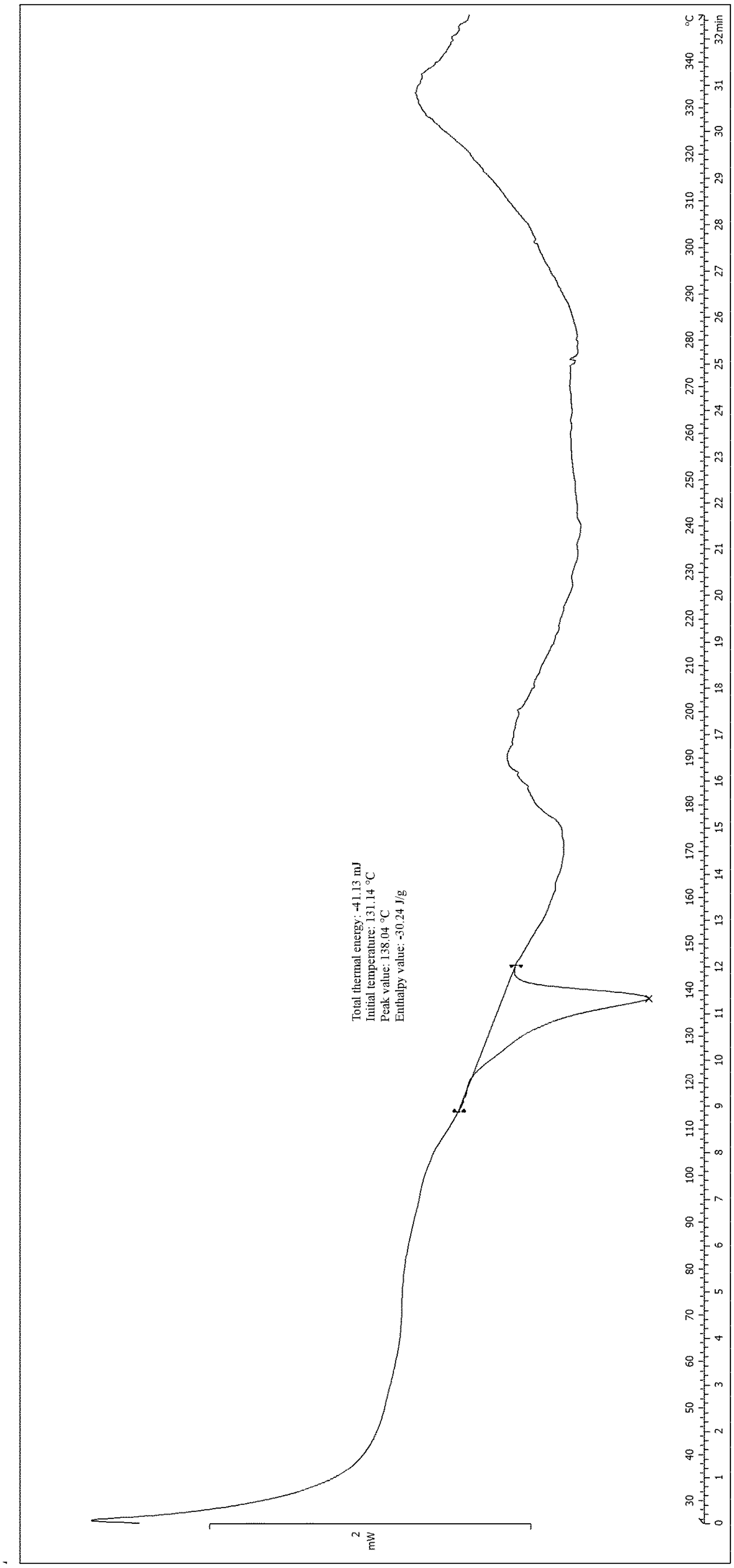
Total thermal energy: -41.13 mJ
Initial temperature: 131.14 °C
Peak value: 138.04 °C
Enthalpy value: -30.24 J/g
2
mW
°C
min

FIG. 9

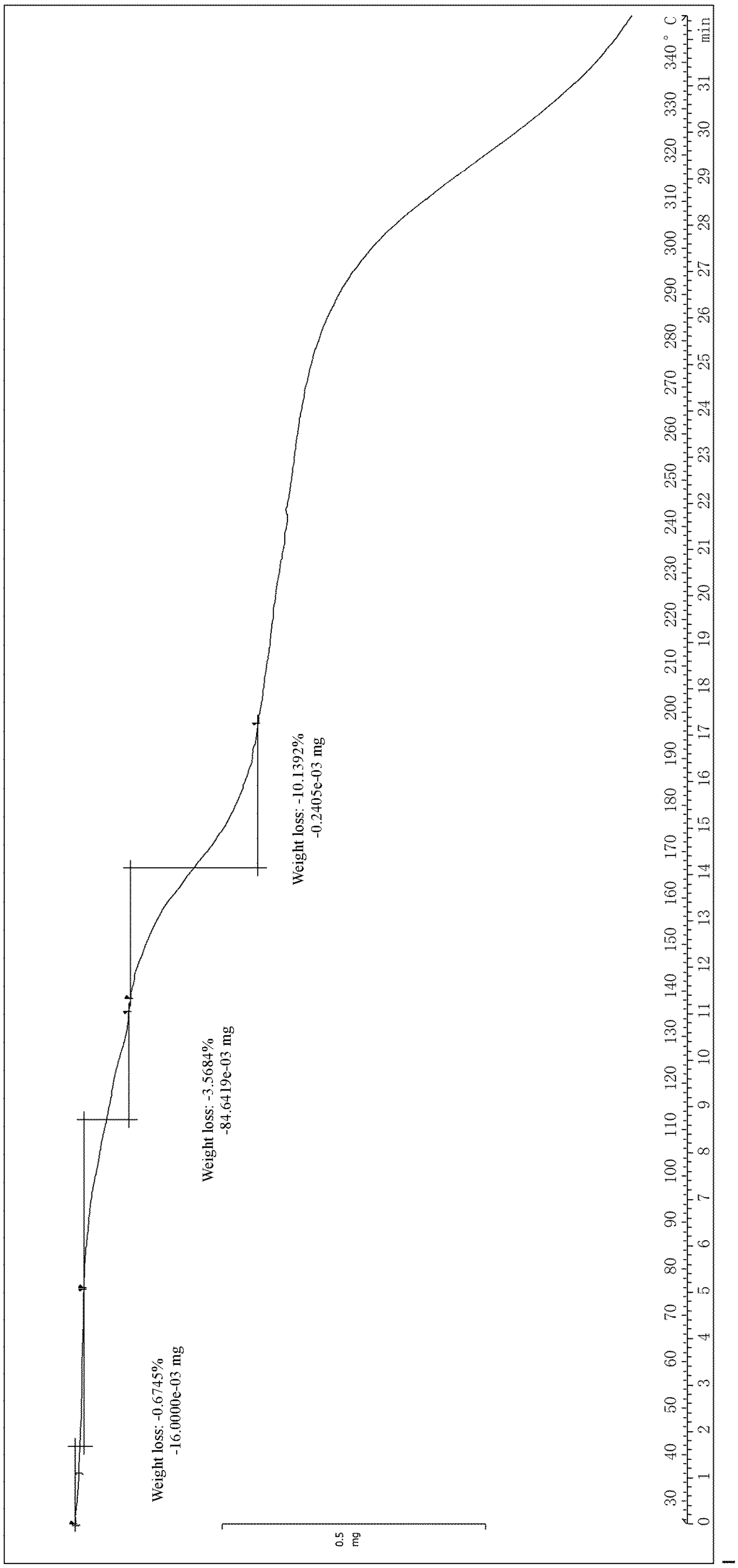
Weight loss: -0.6745%
-16.0000e-03 mg
Weight loss: -3.5684%
-84.6419e-03 mg
Weight loss: -10.1392%
-0.2405e-03 mg
0.5
mg
30 40 50 60 70 80 90 100 110 120 130 140 150 160 170 180 190 200 210 220 230 240 250 260 270 280 290 300 310 320 330 340 ° C
0 1 2 3 4 5 6 7 8 9 10 11 12 13 14 15 16 17 18 19 20 21 22 23 24 25 26 27 28 29 30 31 min

FIG. 10

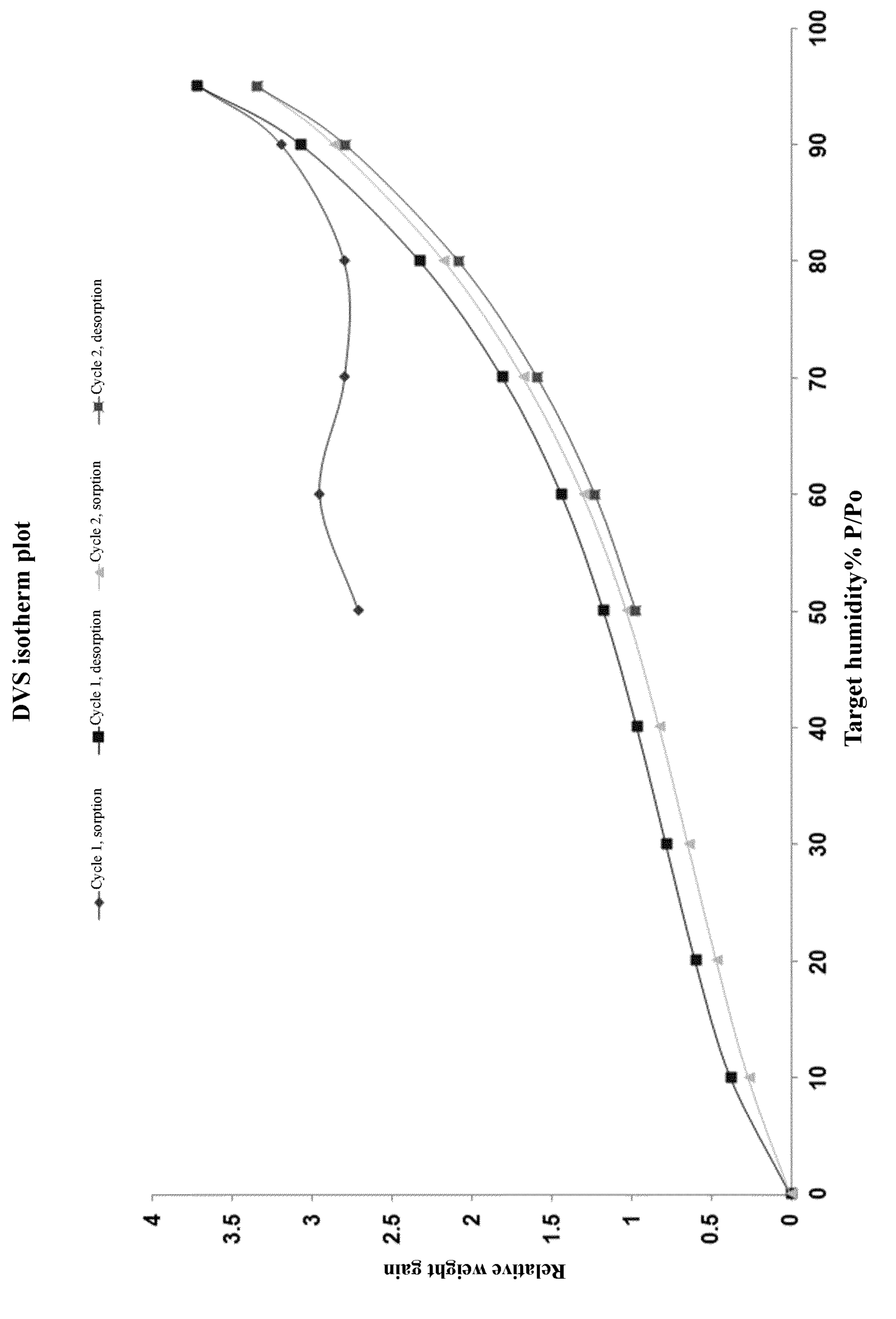
DVS isotherm plot
Cycle 1, sorption
Cycle 1, desorption
Cycle 2, sorption
Cycle 2, desorption
Relative weight gain
0
0.5
1
1.5
2
2.5
3
3.5
4
0
10
20
30
40
50
60
70
80
90
100
Target humidity% P/Po

FIG. 11

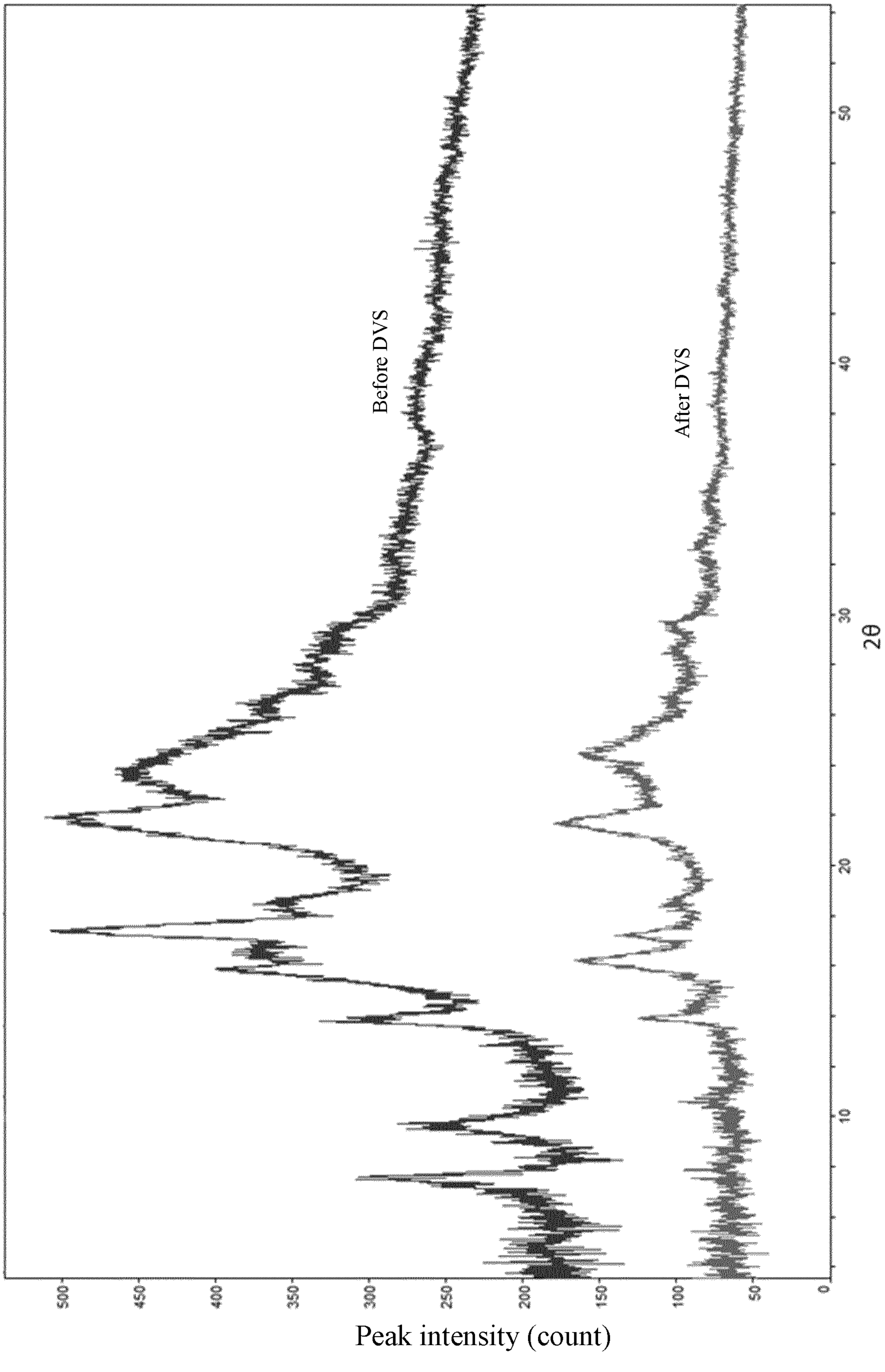
Before DVS
After DVS
2θ
Peak intensity (count)
0
50
100
150
200
250
300
350
400
450
500
10
20
30
40
50

FIG. 12

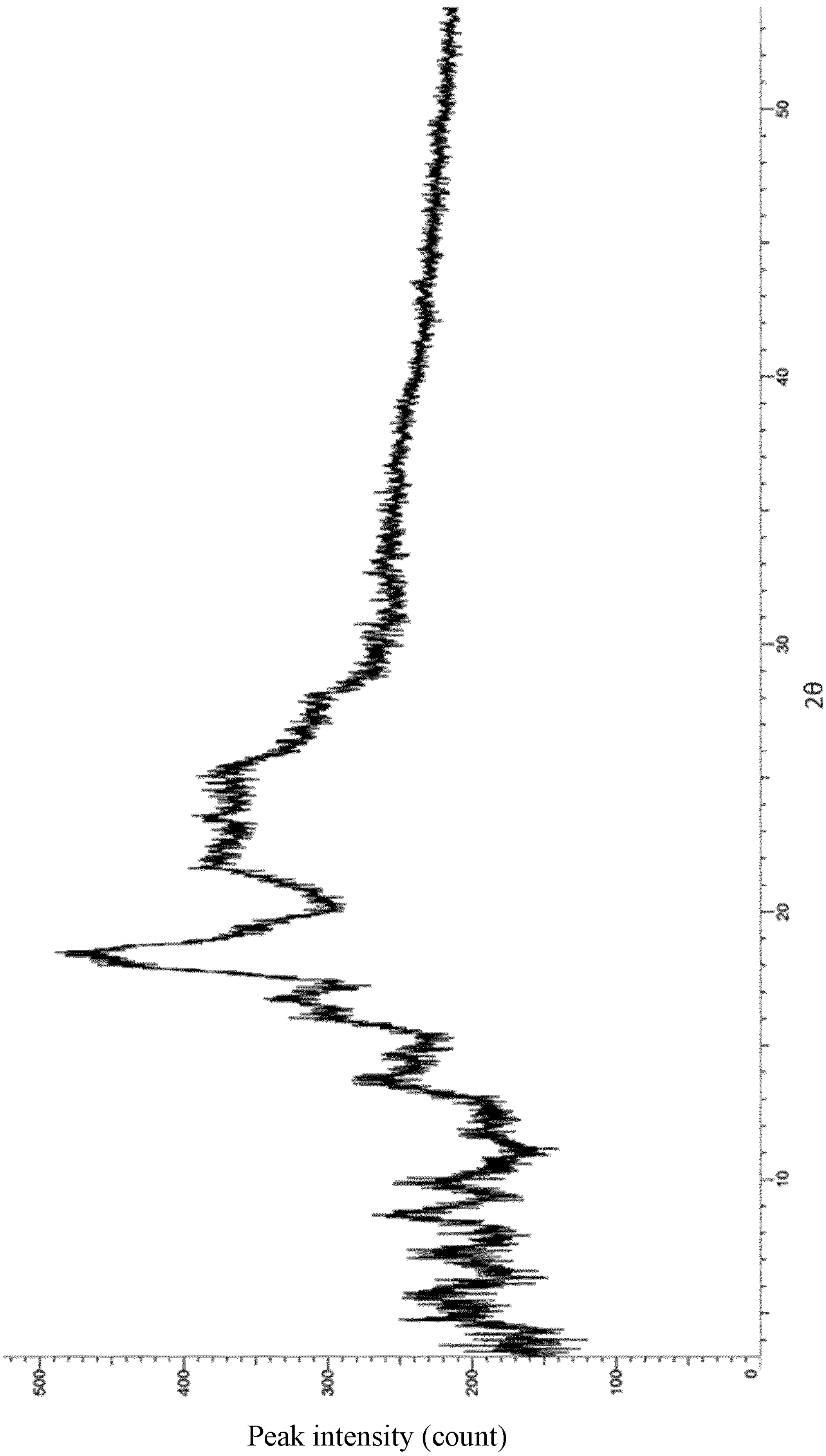


FIG. 13

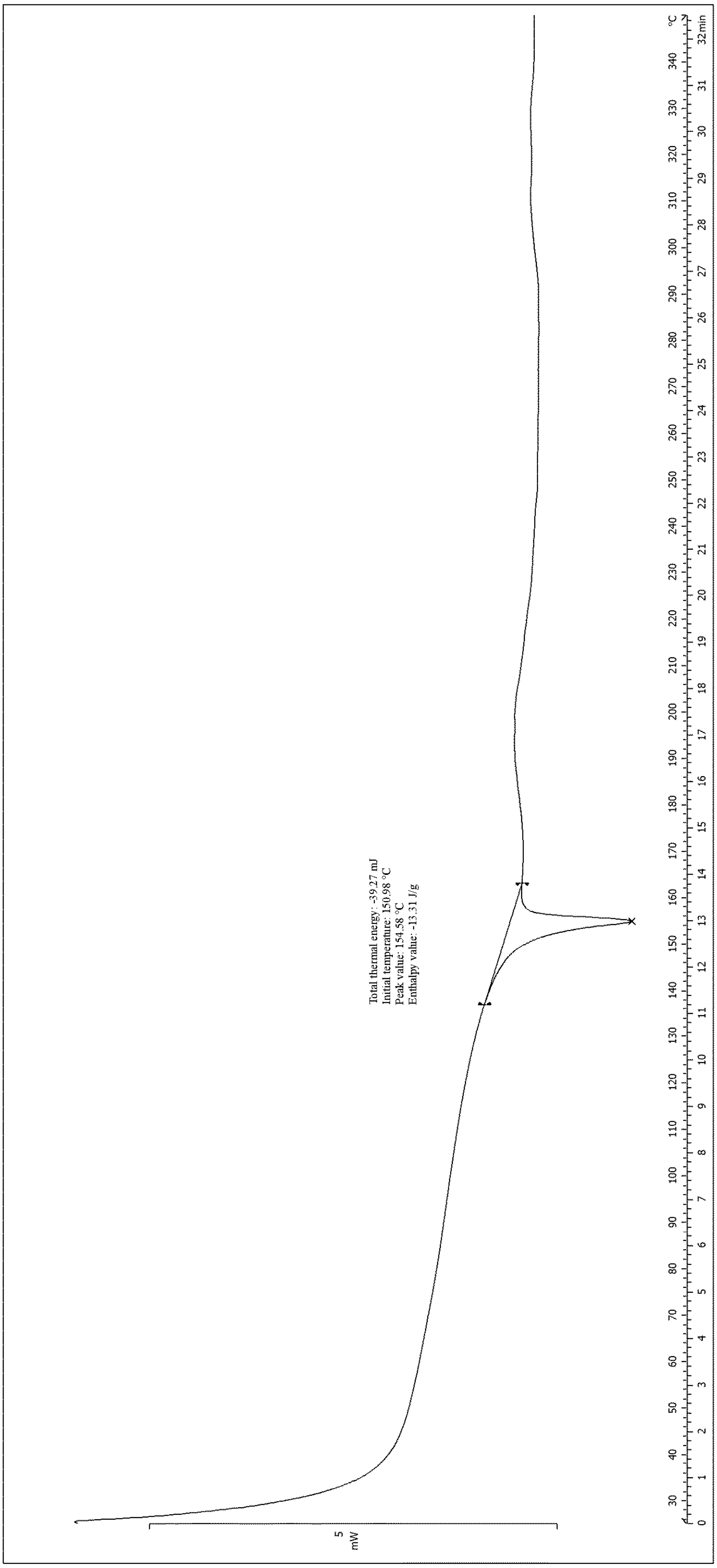
Total thermal energy: -39.27 mJ
Initial temperature: 150.98 °C
Peak value: 154.58 °C
Enthalpy value: -13.31 J/g
5
mW
°C
min

FIG. 14

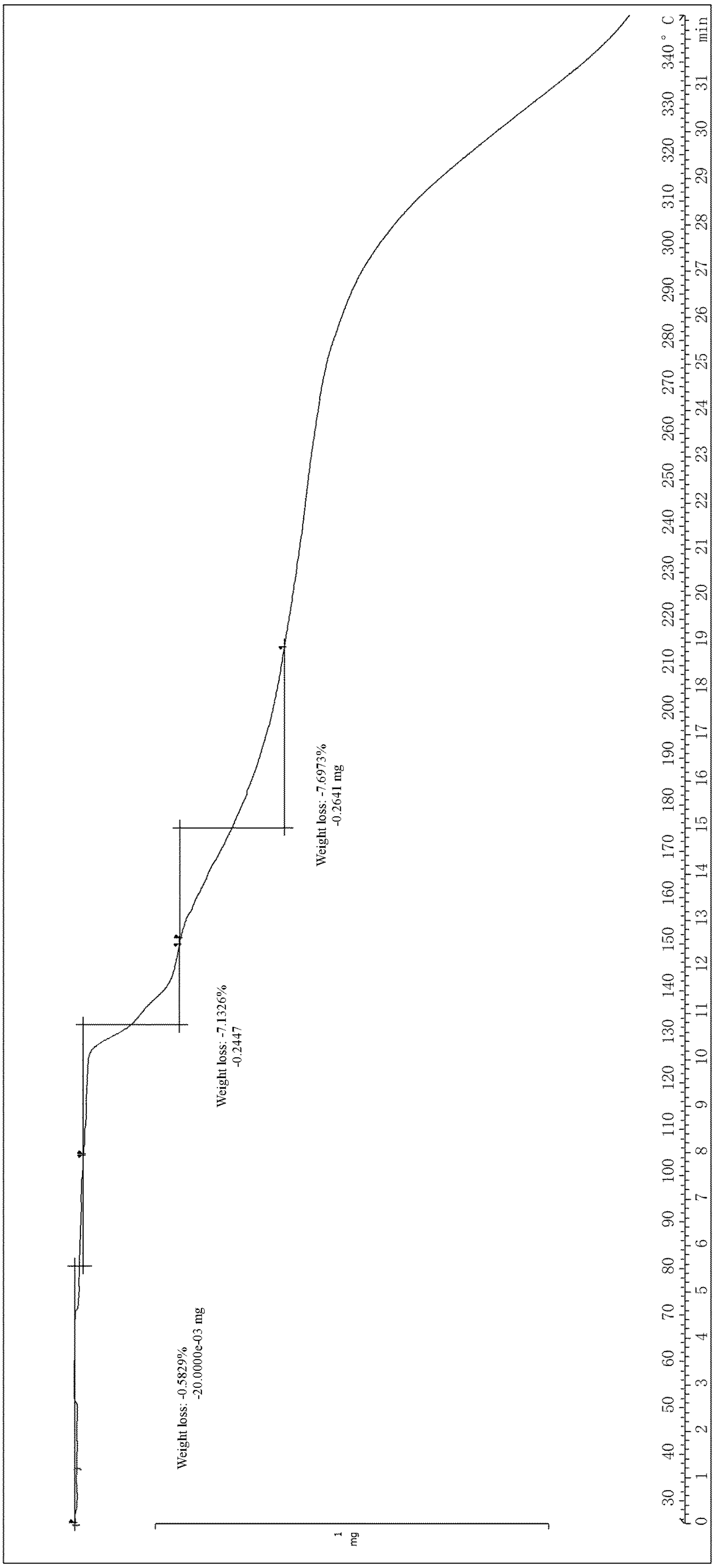
Weight loss: -0.5829%
-20.0000e-03 mg
Weight loss: -7.1326%
-0.2447
Weight loss: -7.6973%
-0.2641 mg
1
mg
30 40 50 60 70 80 90 100 110 120 130 140 150 160 170 180 190 200 210 220 230 240 250 260 270 280 290 300 310 320 330 340 ° C
0 1 2 3 4 5 6 7 8 9 10 11 12 13 14 15 16 17 18 19 20 21 22 23 24 25 26 27 28 29 30 31 min

FIG. 15

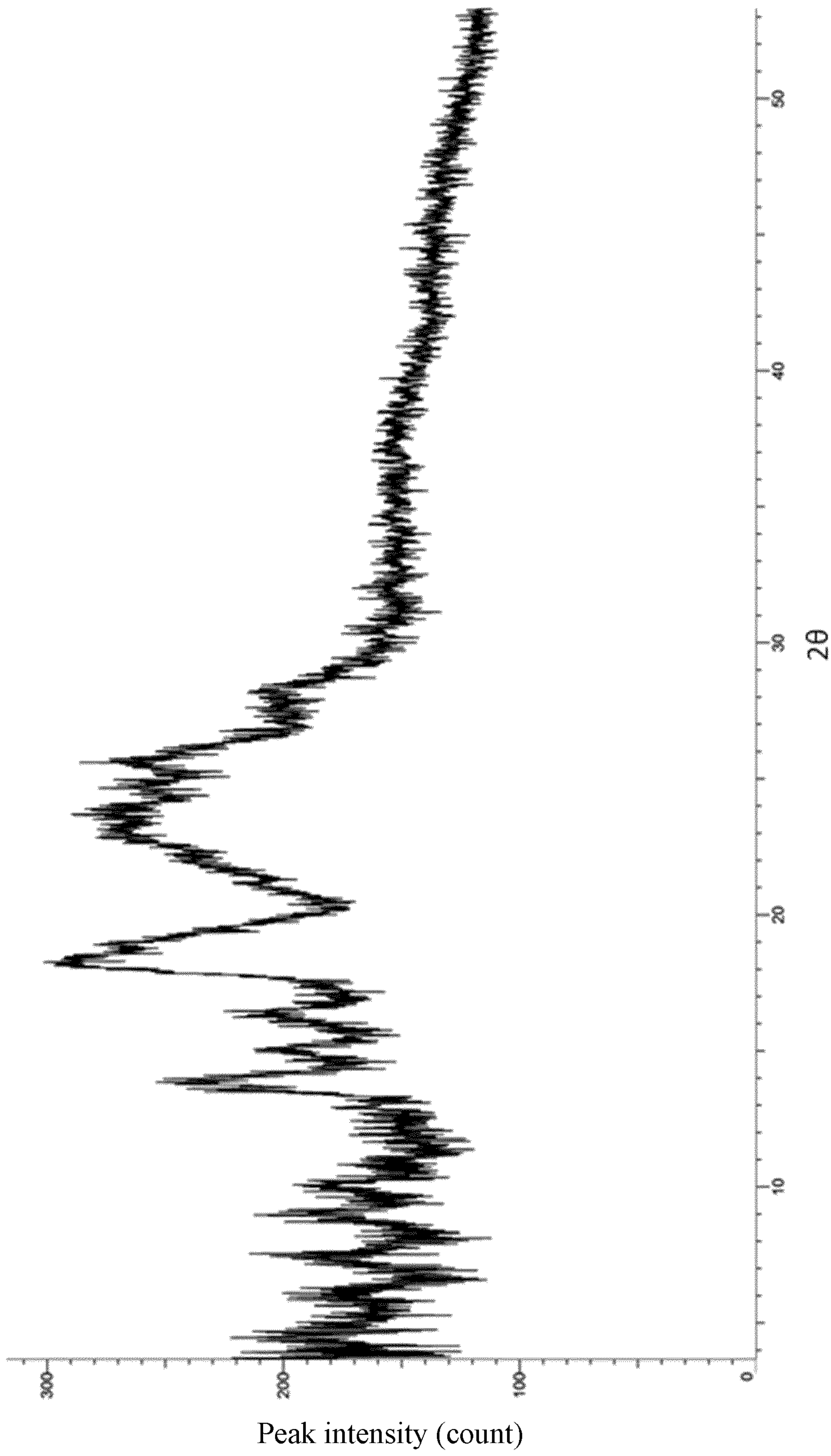


FIG. 16

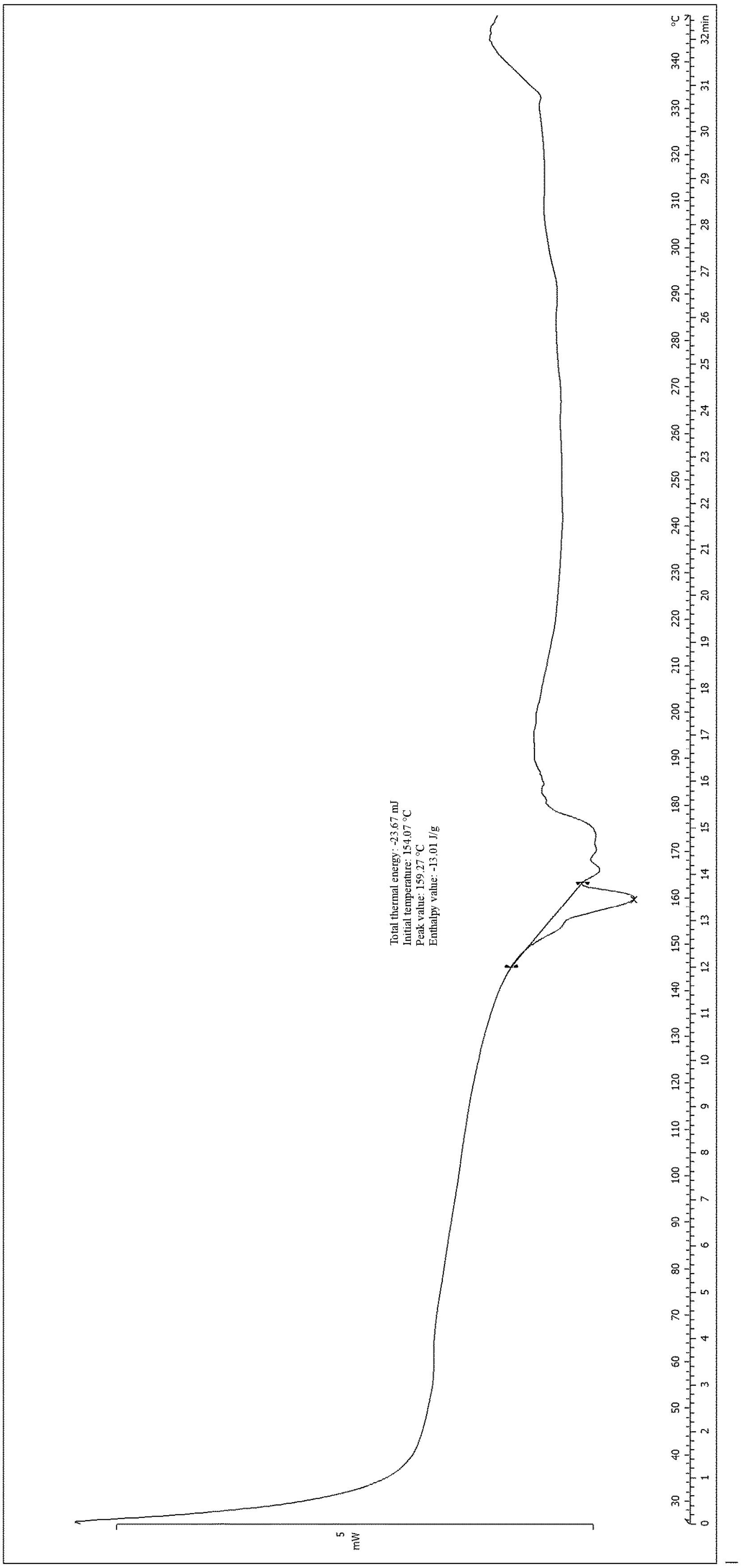
Total thermal energy: -23.67 mJ
Initial temperature: 154.07 °C
Peak value: 159.27 °C
Enthalpy value: -13.01 J/g
5
mW
°C
min

FIG. 17

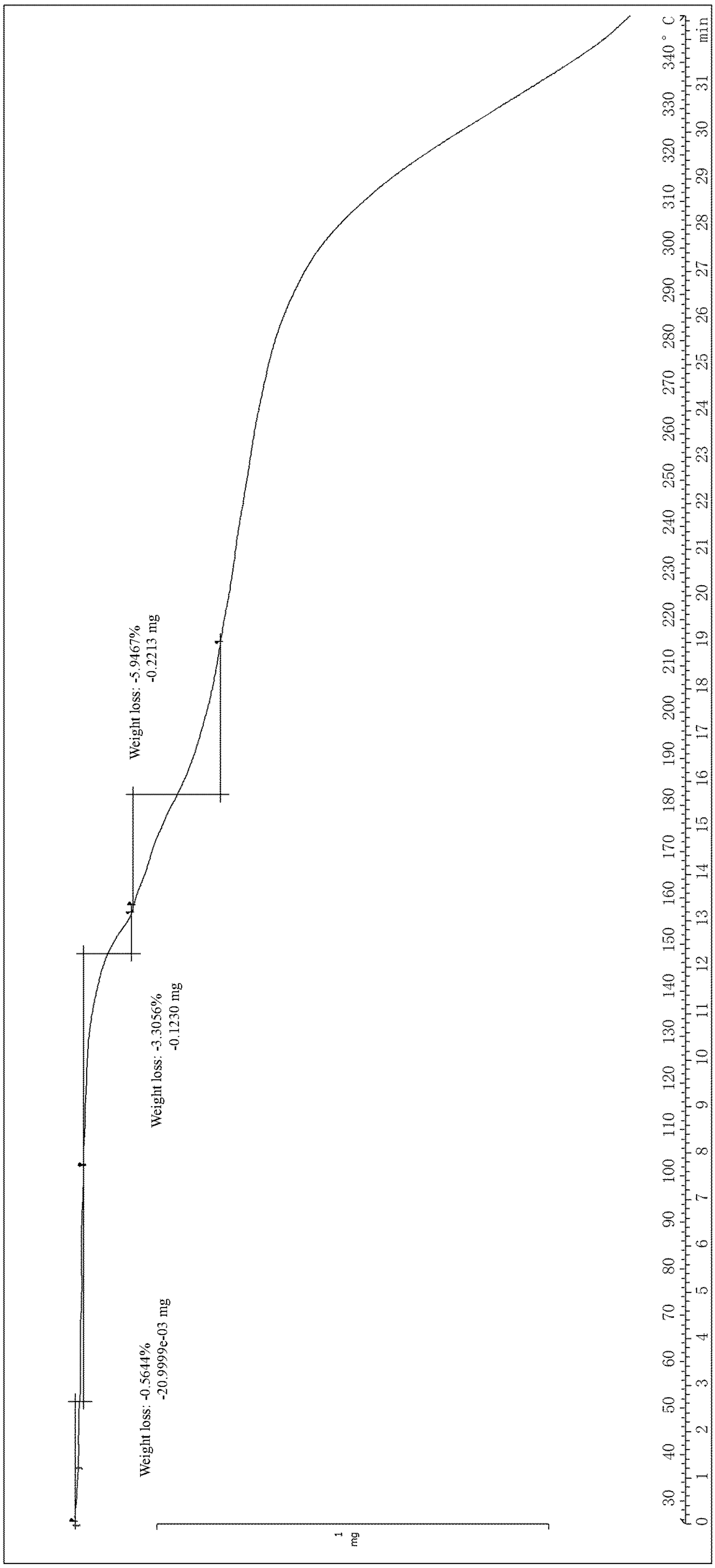
Weight loss: -0.5644%
-20.9999e-03 mg
Weight loss: -3.3056%
-0.1230 mg
Weight loss: -5.9467%
-0.2213 mg
1
mg
° C
min

FIG. 18

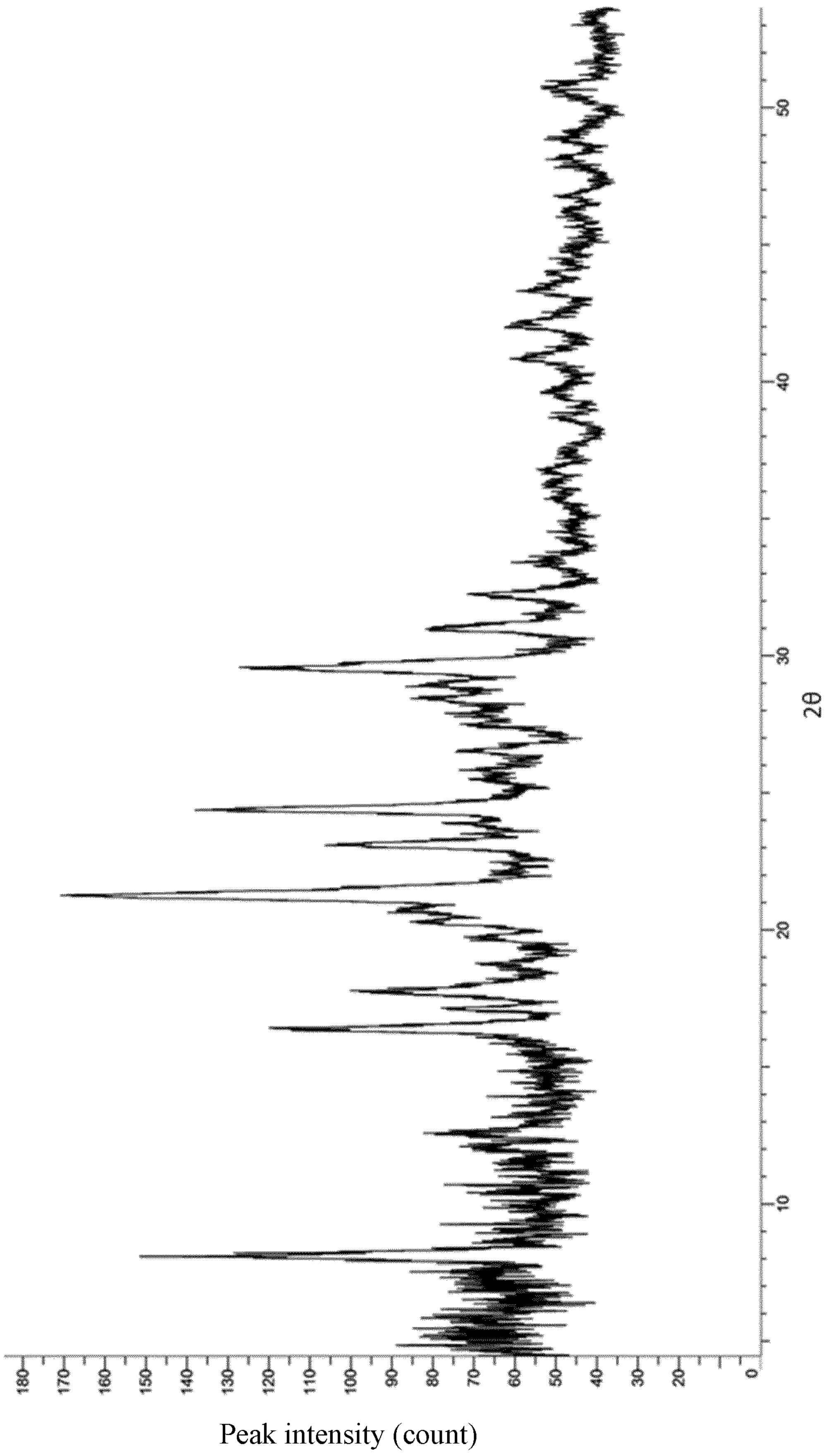
2θ
Peak intensity (count)
10
20
30
40
50
0
20
30
40
50
60
70
80
90
100
110
120
130
140
150
160
170
180

FIG. 19

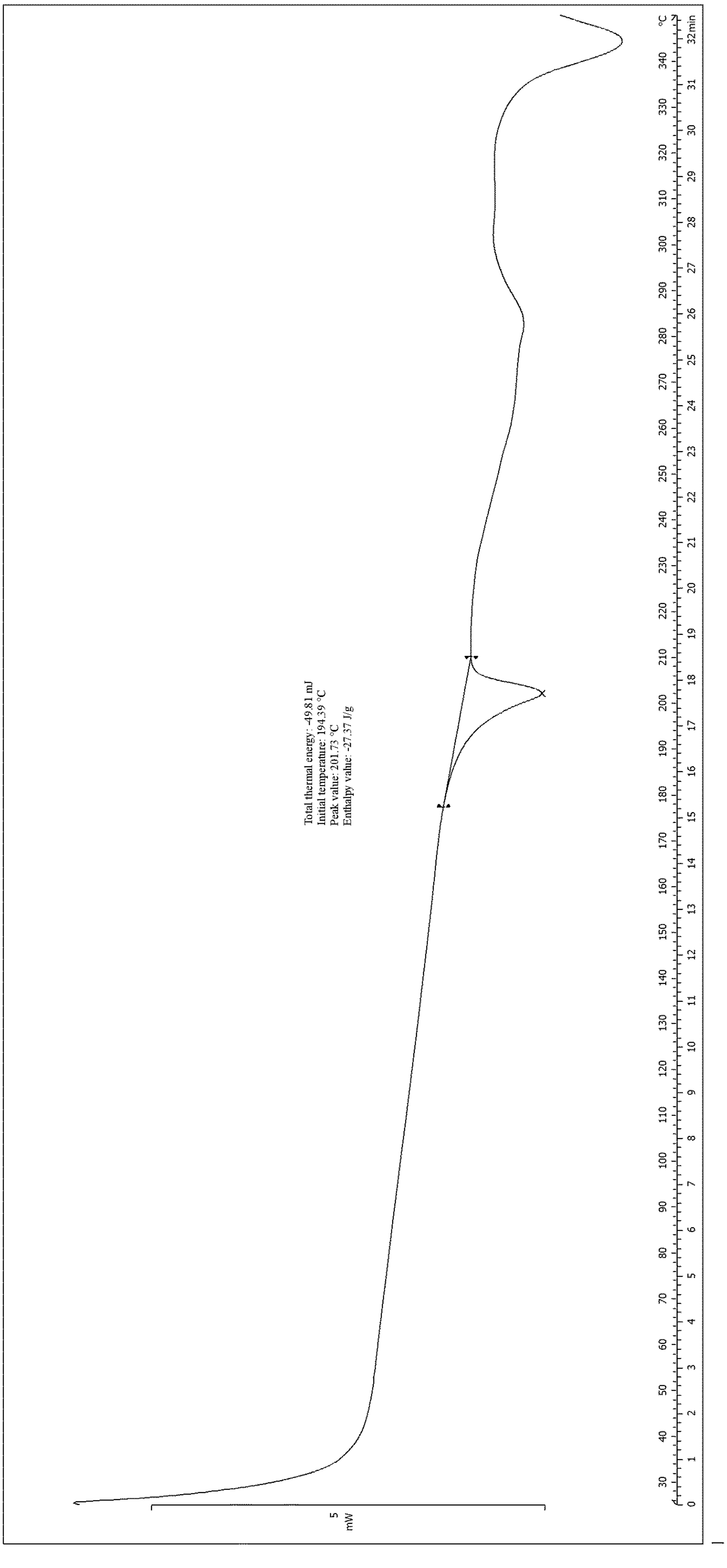
Total thermal energy: -49.81 mJ
Initial temperature: 194.39 °C
Peak value: 201.73 °C
Enthalpy value: -27.37 J/g
°C
min
5
mW

FIG. 20

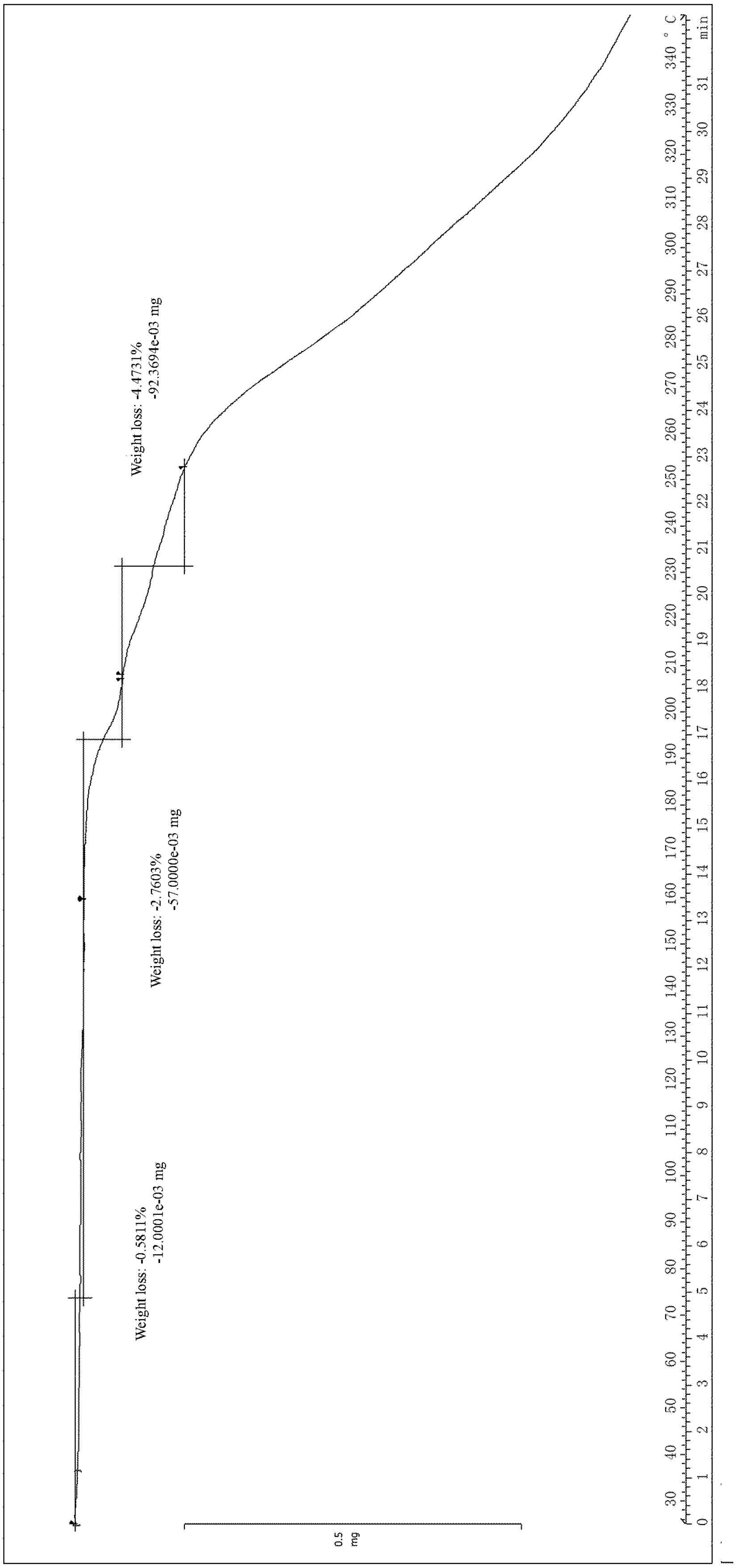
Weight loss: -0.5811%
-12.0001e-03 mg
Weight loss: -2.7603%
-57.0000e-03 mg
Weight loss: -4.4731%
-92.3694e-03 mg
0.5
mg
30 40 50 60 70 80 90 100 110 120 130 140 150 160 170 180 190 200 210 220 230 240 250 260 270 280 290 300 310 320 330 340 °C
0 1 2 3 4 5 6 7 8 9 10 11 12 13 14 15 16 17 18 19 20 21 22 23 24 25 26 27 28 29 30 31 min

FIG. 21

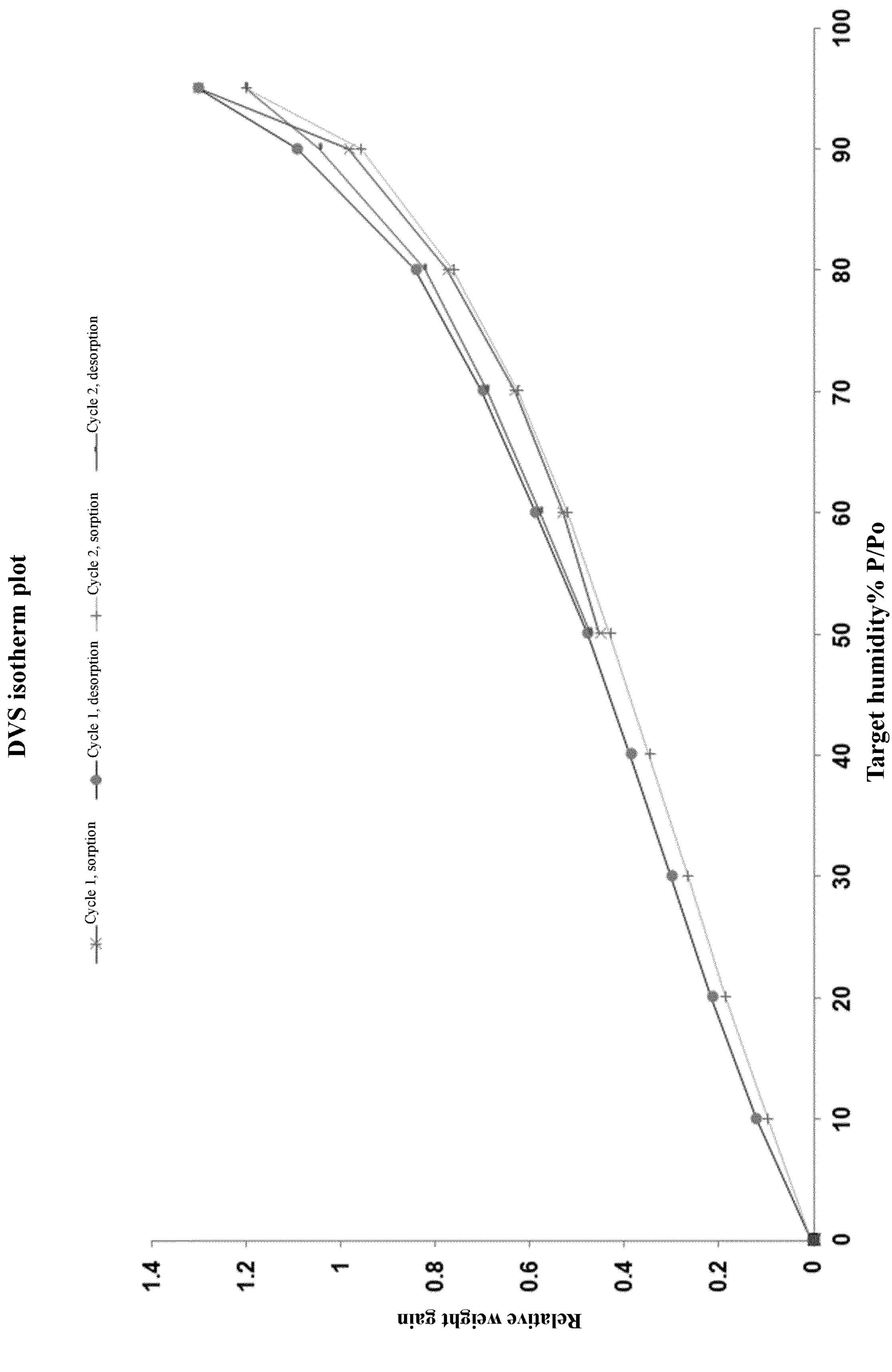
DVS isotherm plot
Cycle 1, sorption
Cycle 1, desorption
Cycle 2, sorption
Cycle 2, desorption
Relative weight gain
0
0.2
0.4
0.6
0.8
1
1.2
1.4
0
10
20
30
40
50
60
70
80
90
100
Target humidity% P/Po

FIG. 22

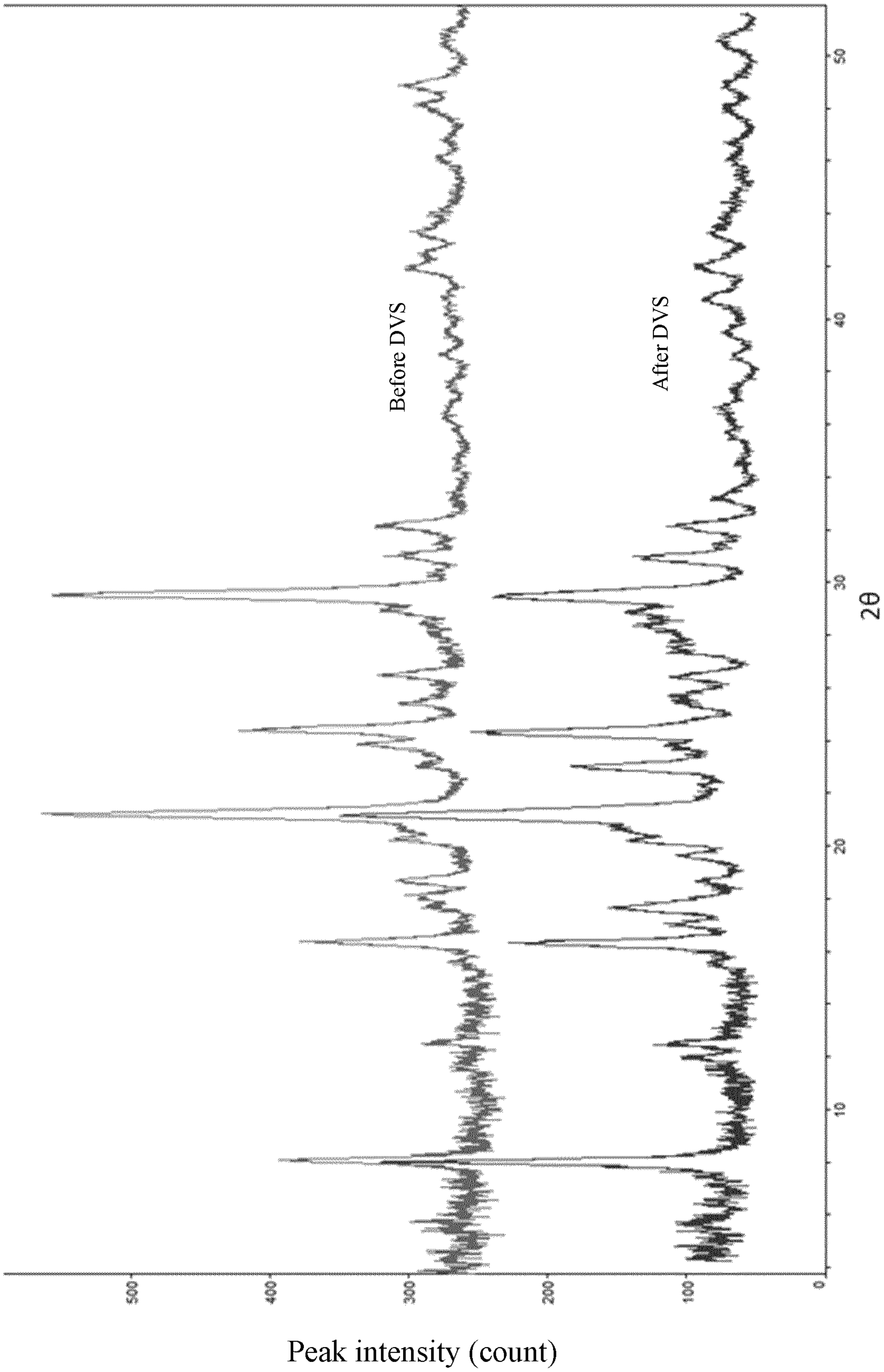
Before DVS
After DVS
2θ
Peak intensity (count)
0
100
200
300
400
500
10
20
30
40
50

FIG. 23

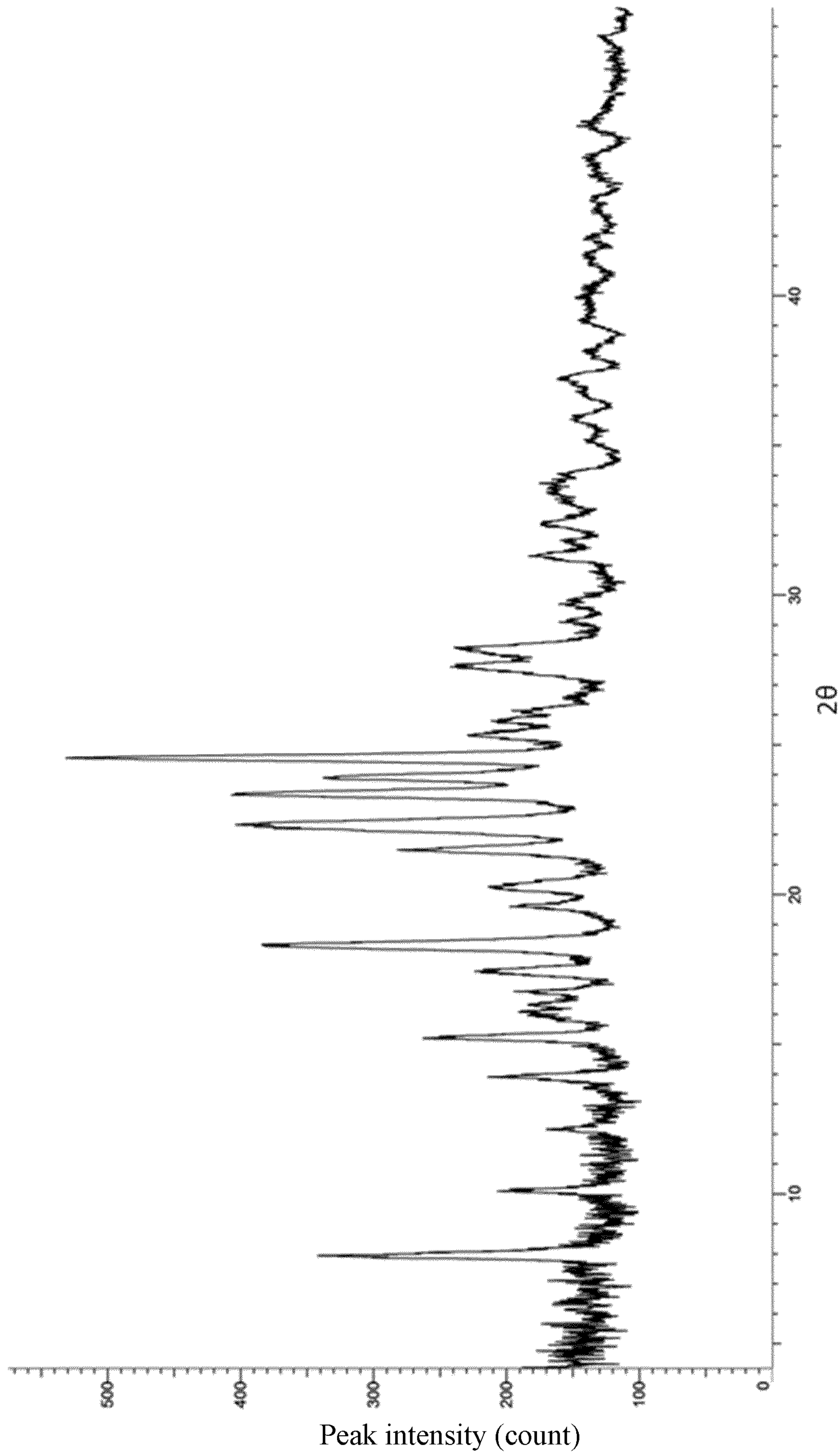


FIG. 24

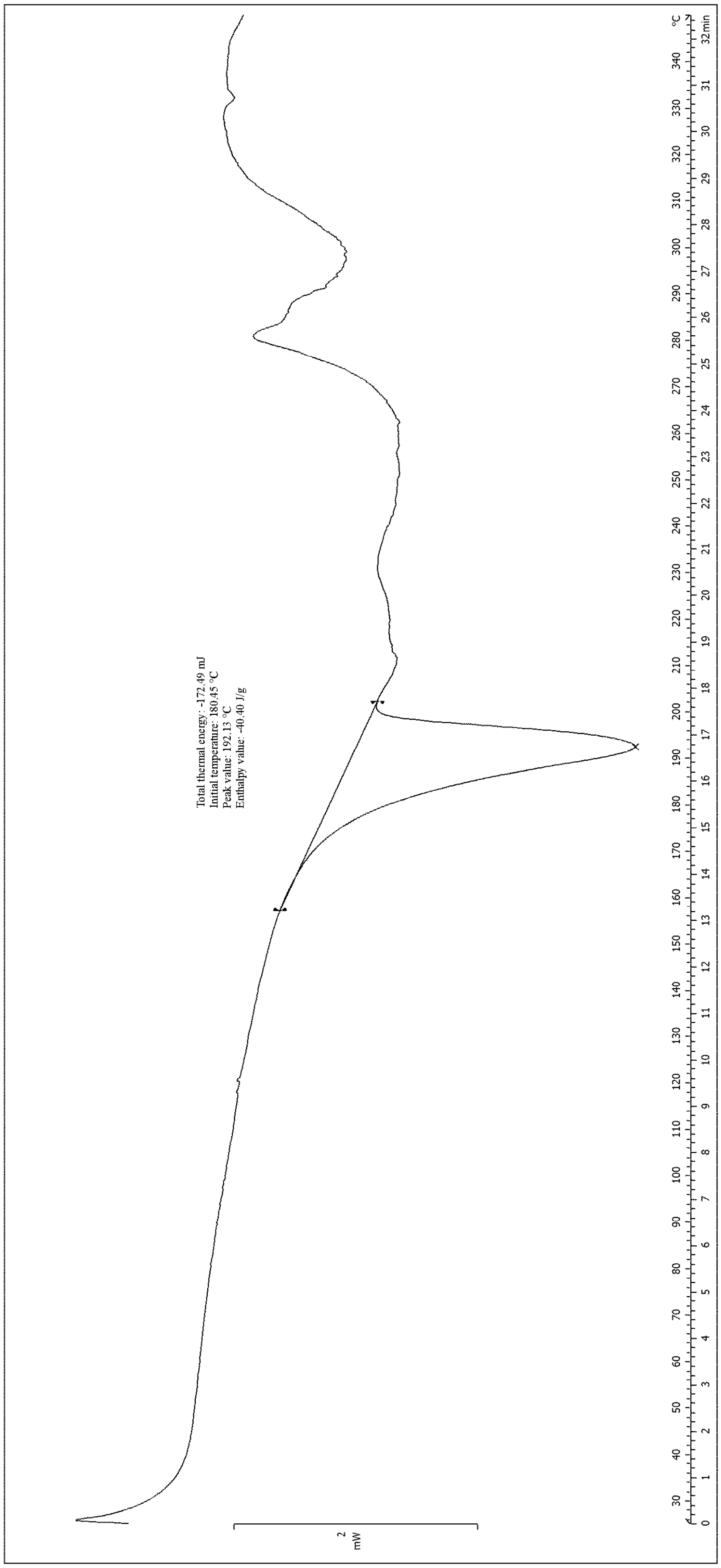
Total thermal energy: -172.49 mJ
Initial temperature: 180.45 °C
Peak value: 192.13 °C
Enthalpy value: -40.40 J/g
2
mW
°C
min

FIG. 25

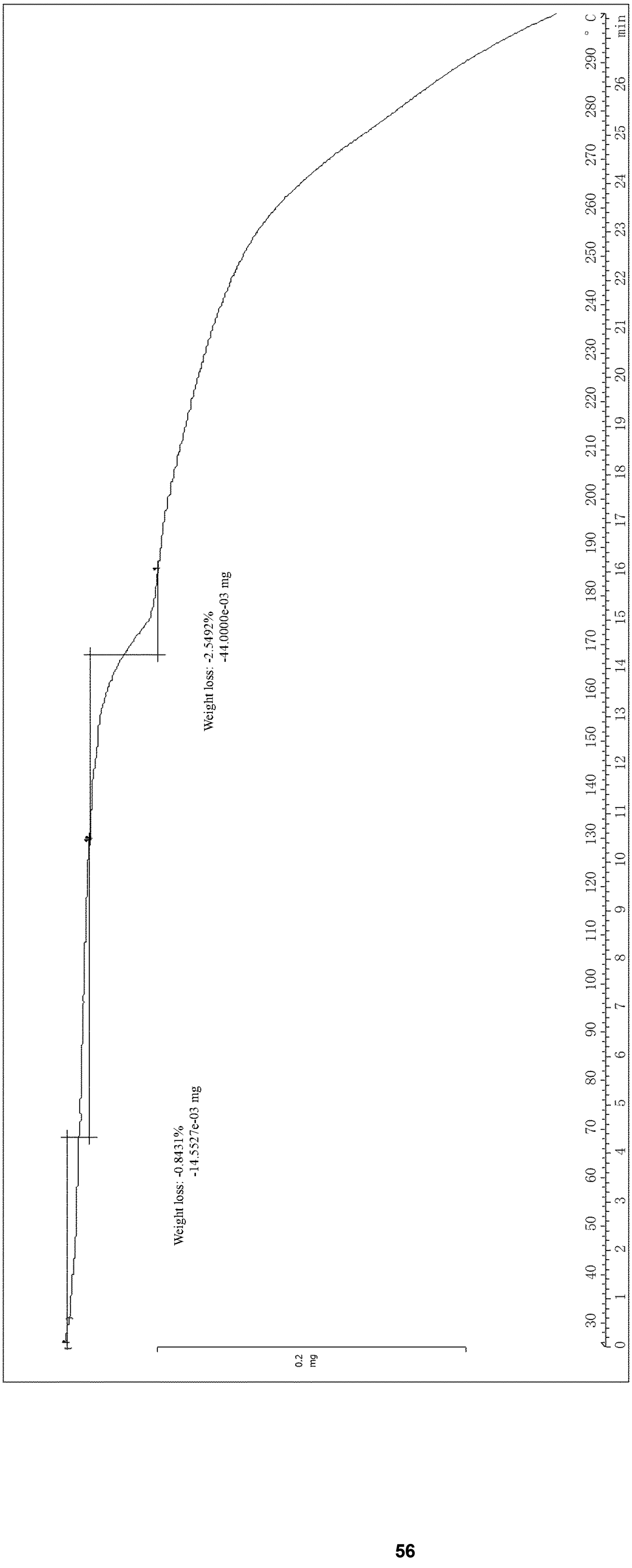
Weight loss: -0.8431%
-14.5527e-03 mg
Weight loss: -2.5492%
-44.0000e-03 mg
0.2
mg
° C
min
30 40 50 60 70 80 90 100 110 120 130 140 150 160 170 180 190 200 210 220 230 240 250 260 270 280 290
0 1 2 3 4 5 6 7 8 9 10 11 12 13 14 15 16 17 18 19 20 21 22 23 24 25 26

FIG. 26

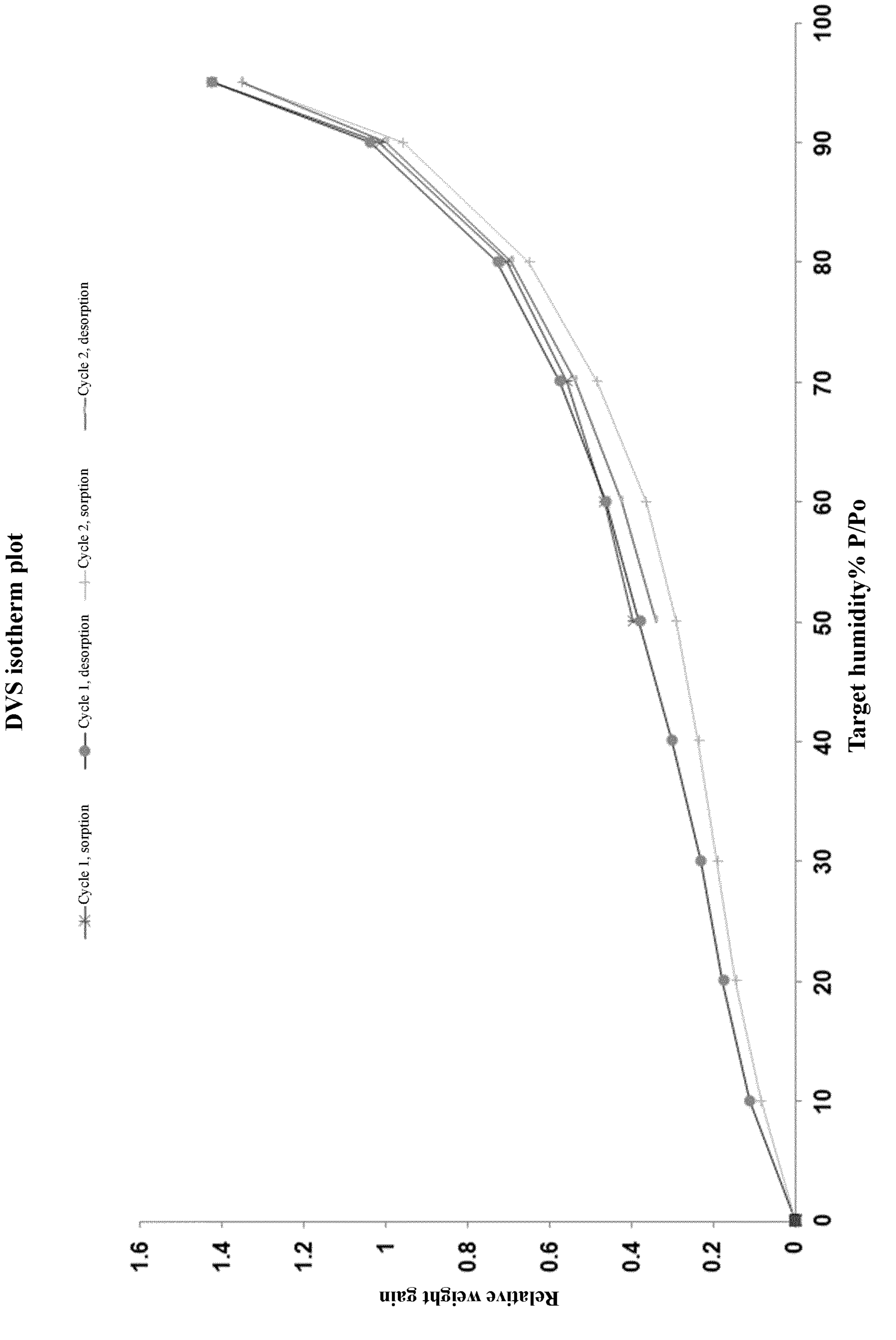
DVS isotherm plot
Cycle 1, sorption
Cycle 1, desorption
Cycle 2, sorption
Cycle 2, desorption
Relative weight gain
0
0.2
0.4
0.6
0.8
1
1.2
1.4
1.6
Target humidity% P/Po
0
10
20
30
40
50
60
70
80
90
100

FIG. 27

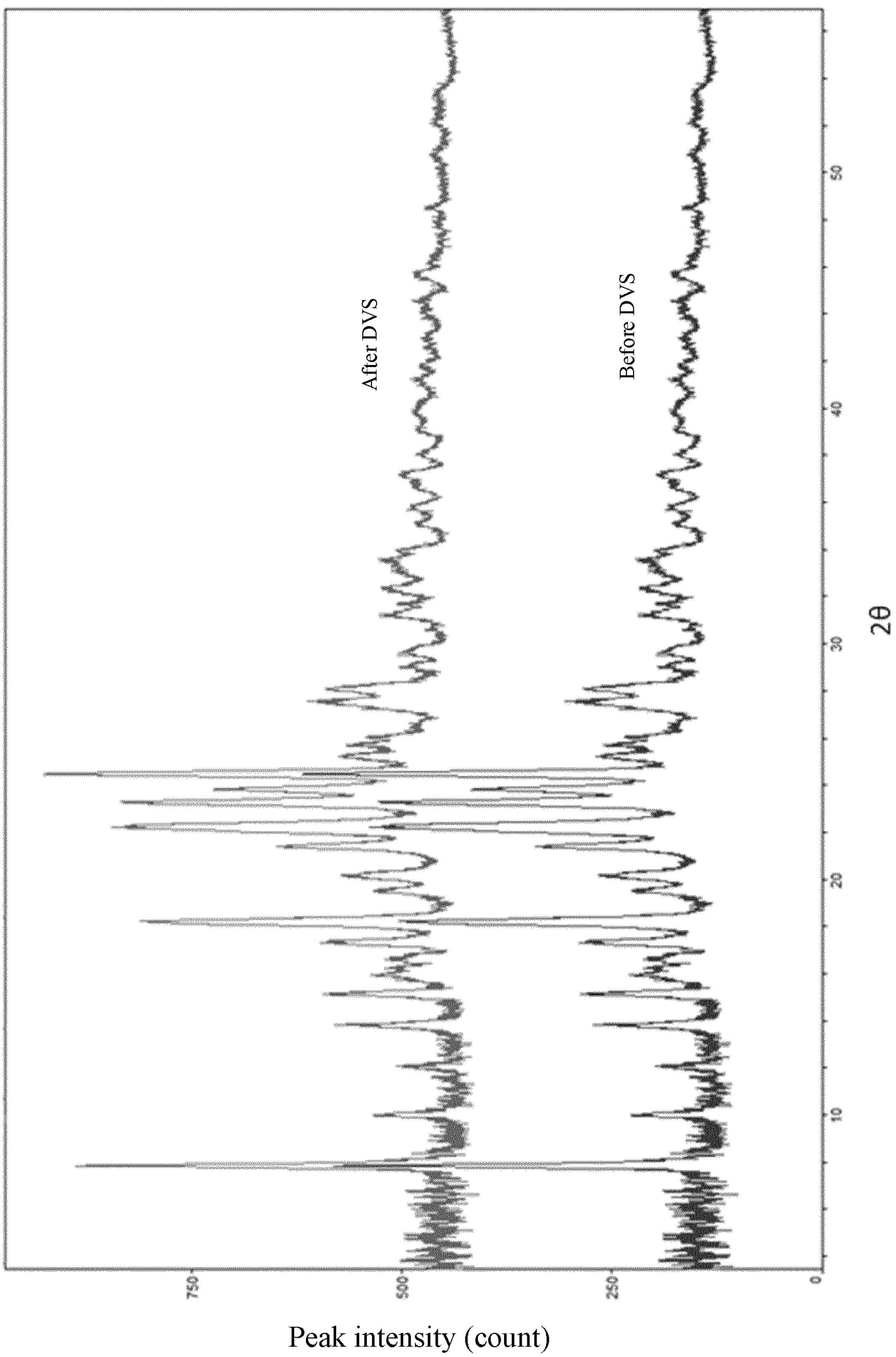
After DVS
Before DVS
2θ
Peak intensity (count)
0
250
500
750
10
20
30
40
50

FIG. 28

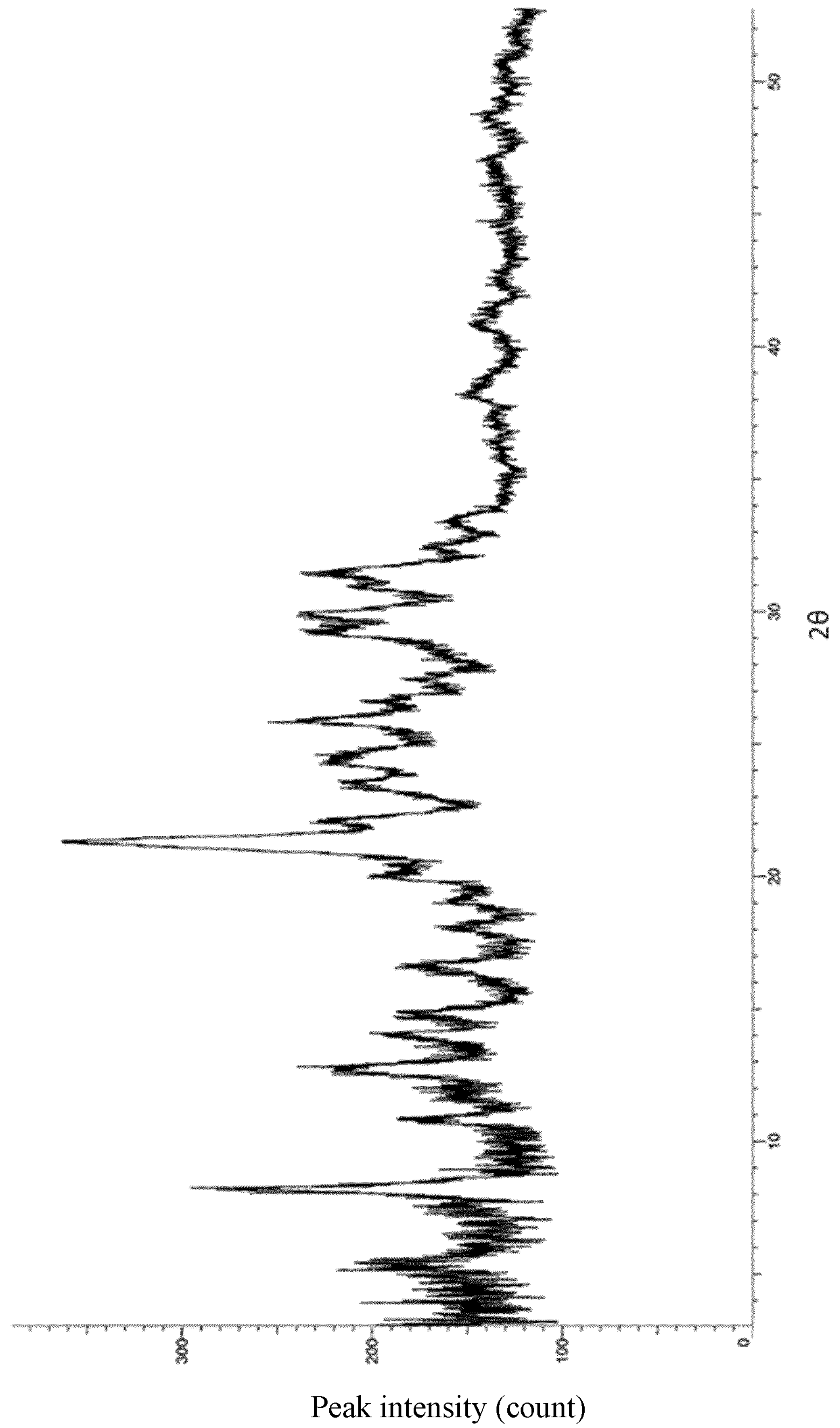


FIG. 29

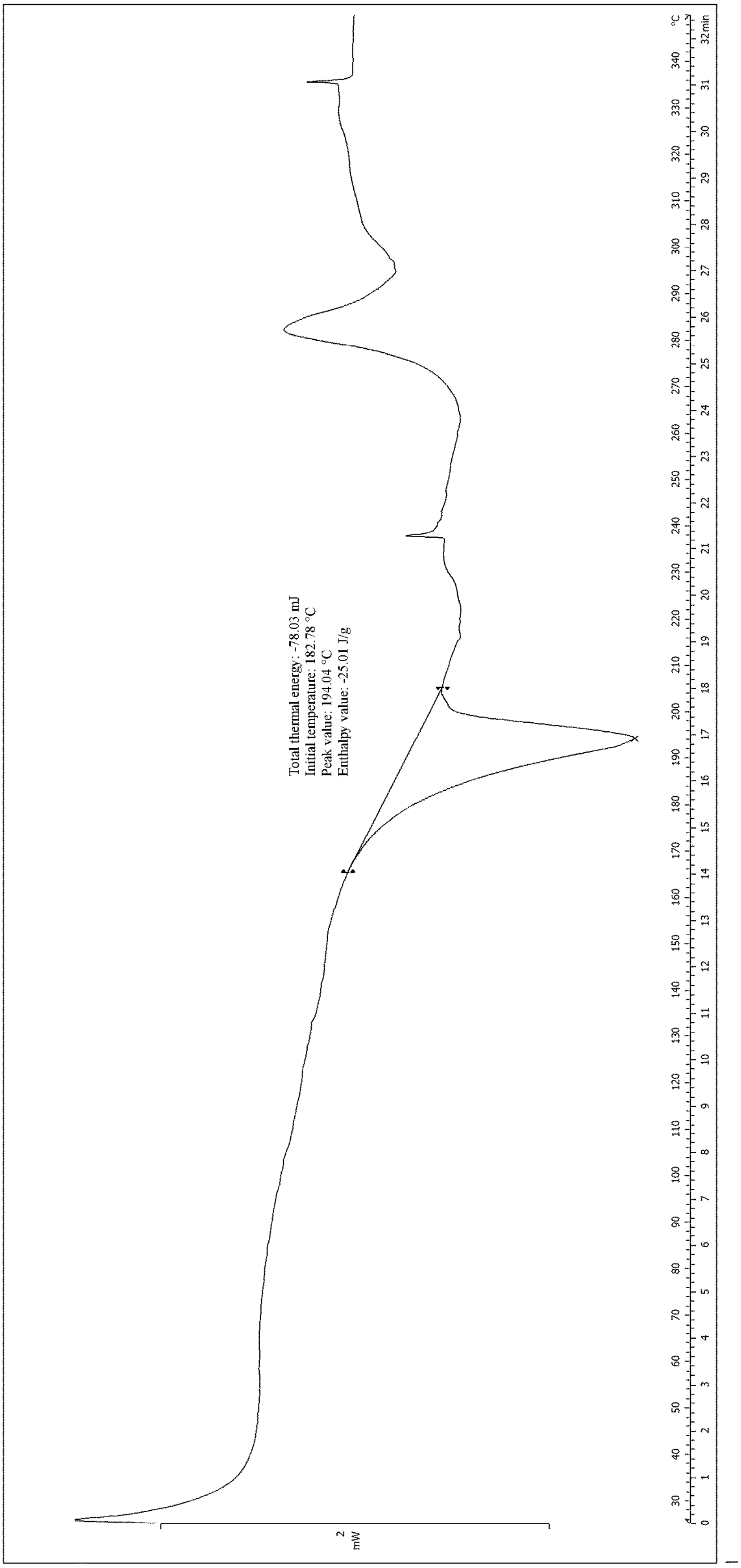
Total thermal energy: -78.03 mJ
Initial temperature: 182.78 °C
Peak value: 194.04 °C
Enthalpy value: -25.01 J/g
°C
min
2
mW

FIG. 30

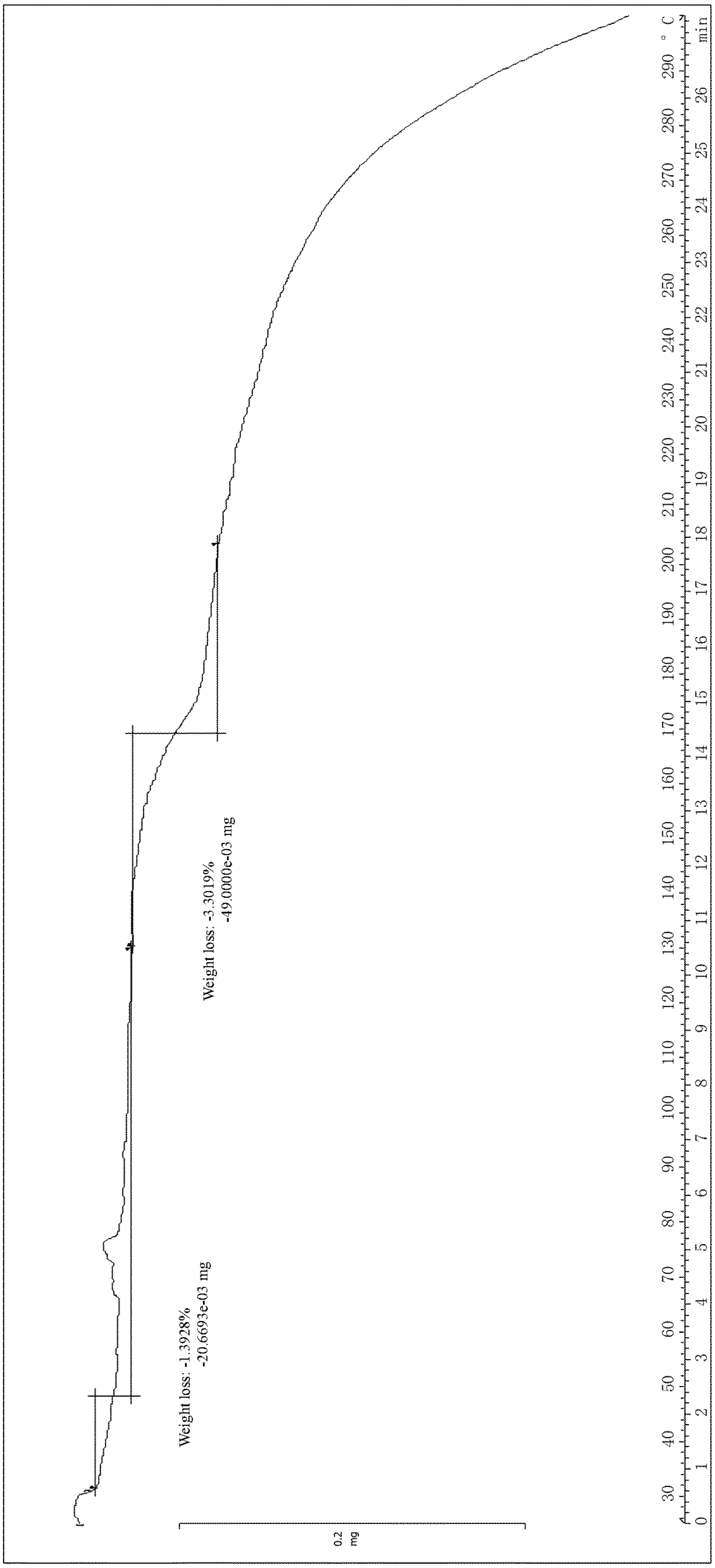
Weight loss: -1.3928%
-20.6693e-03 mg
Weight loss: -3.3019%
-49.0000e-03 mg
0.2
mg
° C
min

FIG. 31

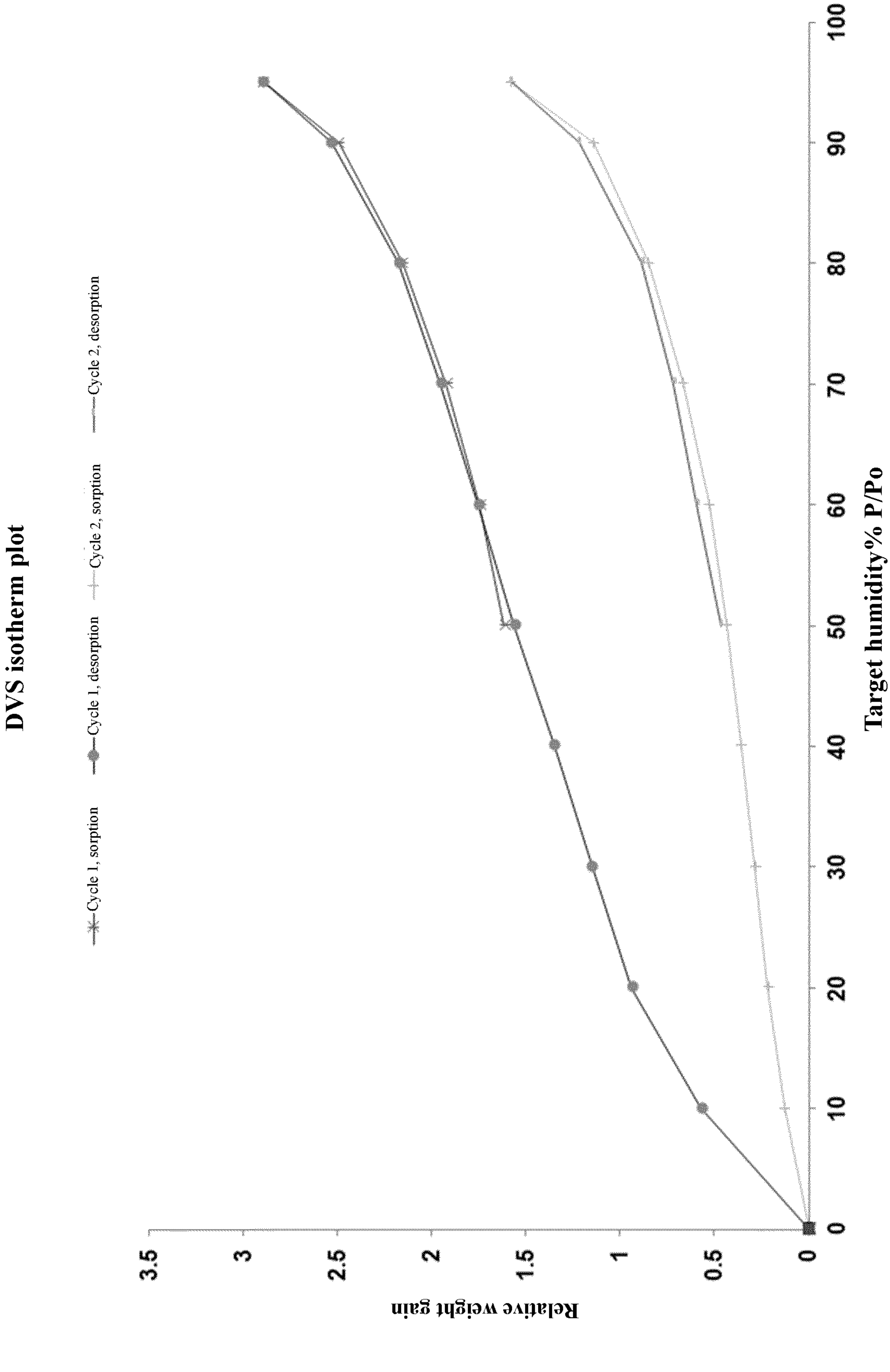
DVS isotherm plot
Cycle 1, sorption
Cycle 1, desorption
Cycle 2, sorption
Cycle 2, desorption
Relative weight gain
3.5
3
2.5
2
1.5
1
0.5
0
0
10
20
30
40
50
60
70
80
90
100
Target humidity% P/Po

FIG. 32

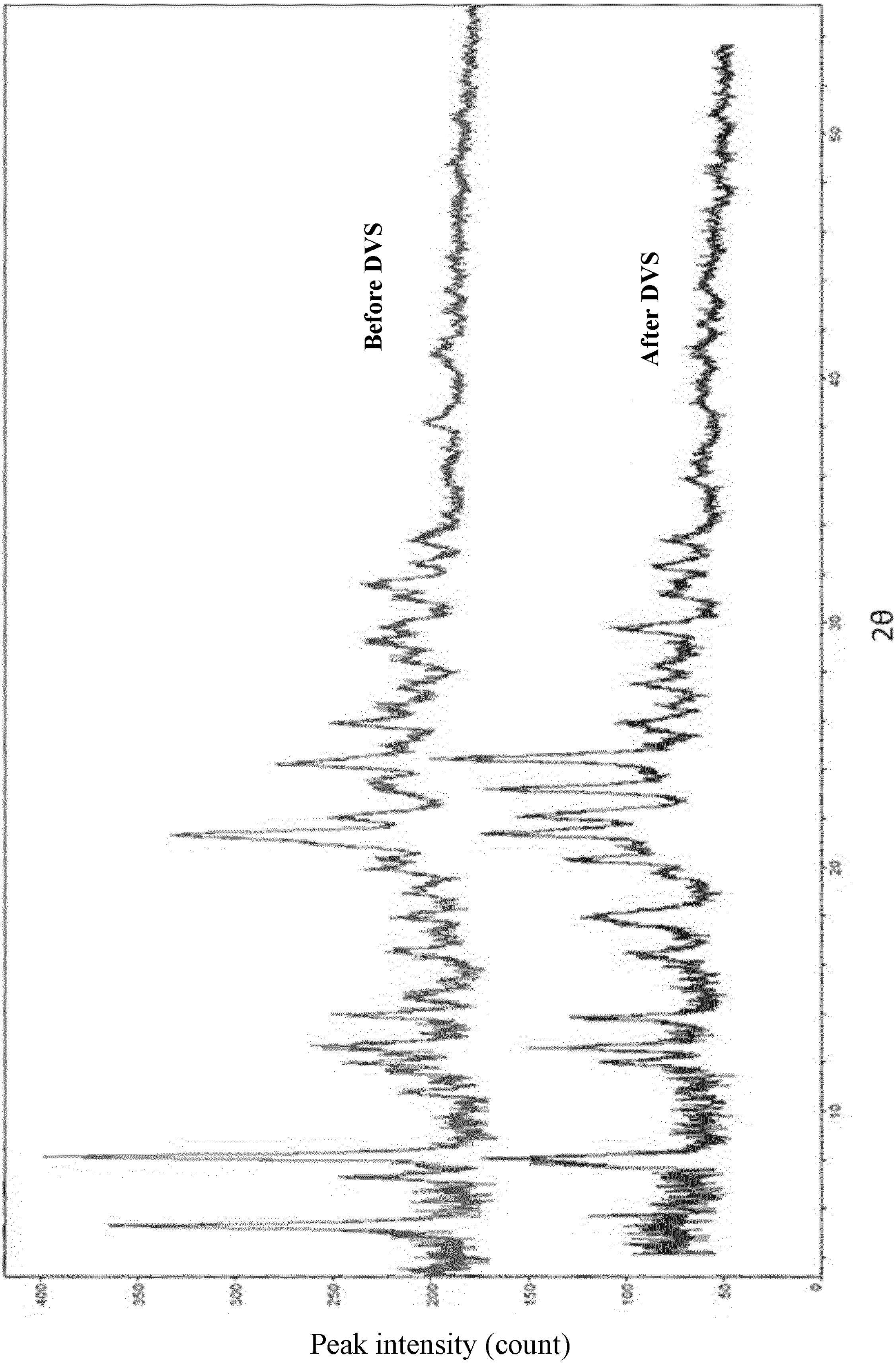
Before DVS
After DVS
2θ
Peak intensity (count)
0
50
100
150
200
250
300
350
400
10
20
30
40
50

FIG. 33

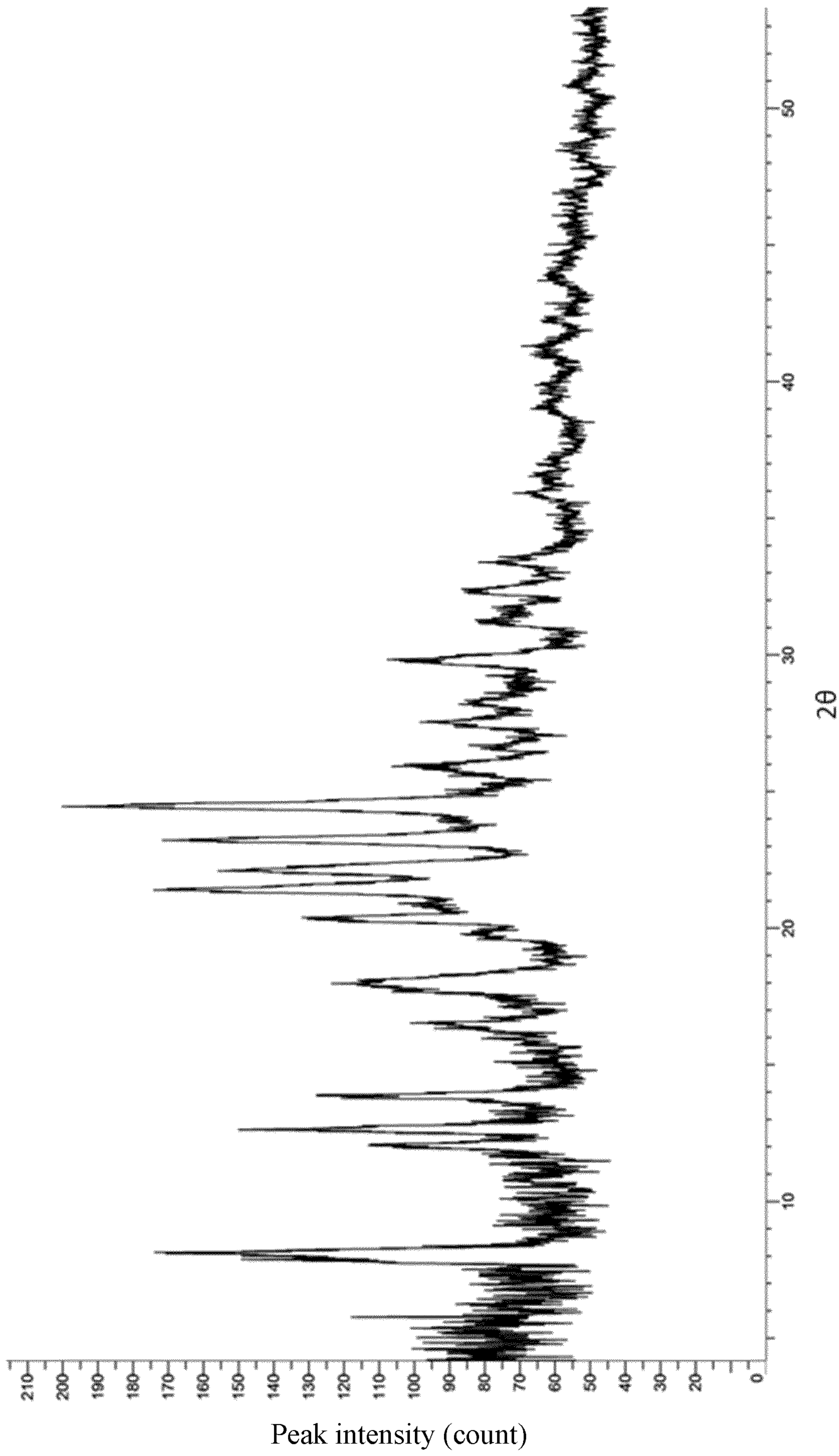


FIG. 34

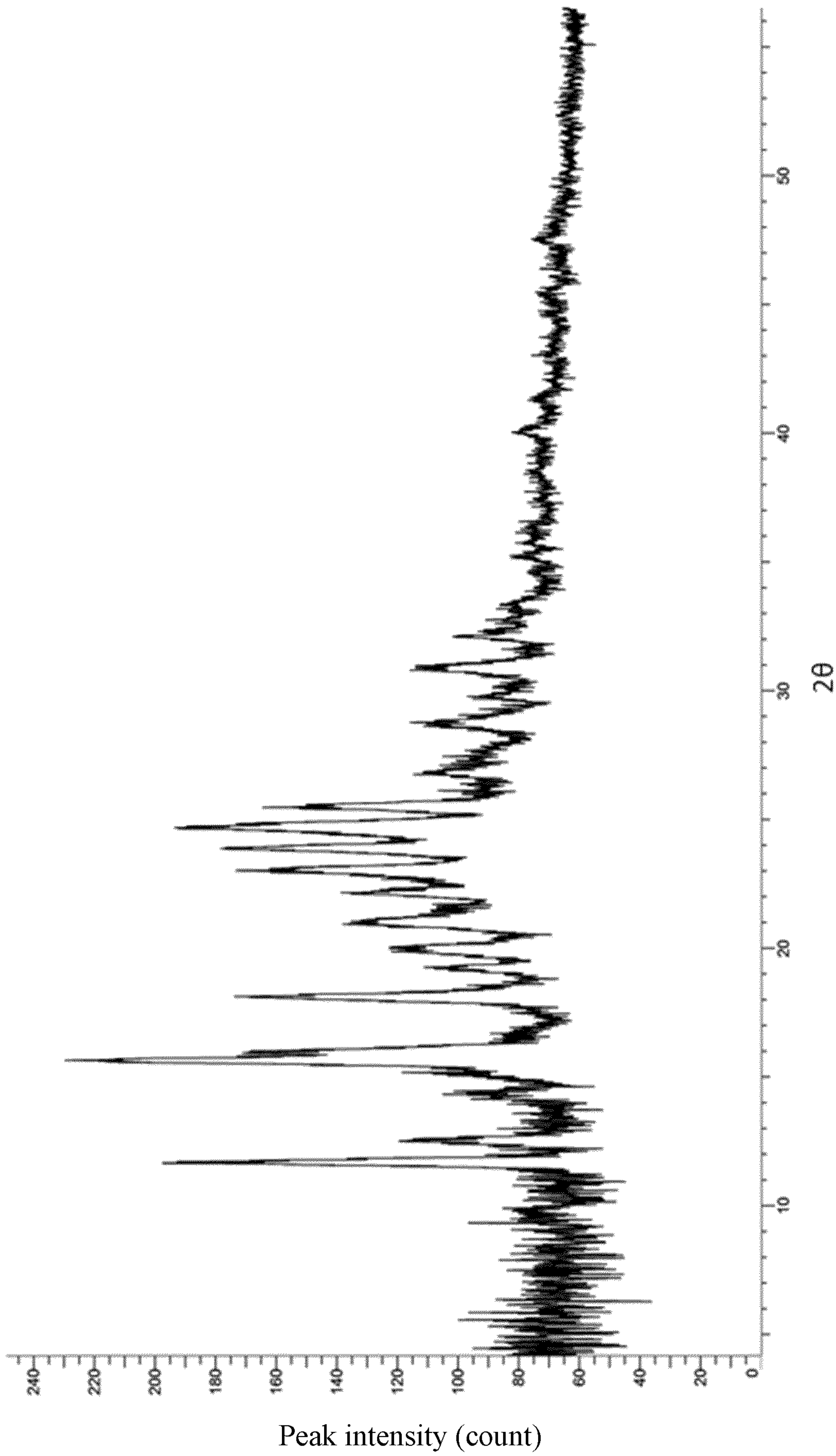
Peak intensity (count)
0
20
40
60
80
100
120
140
160
180
200
220
240
2θ
10
20
30
40
50

FIG. 35

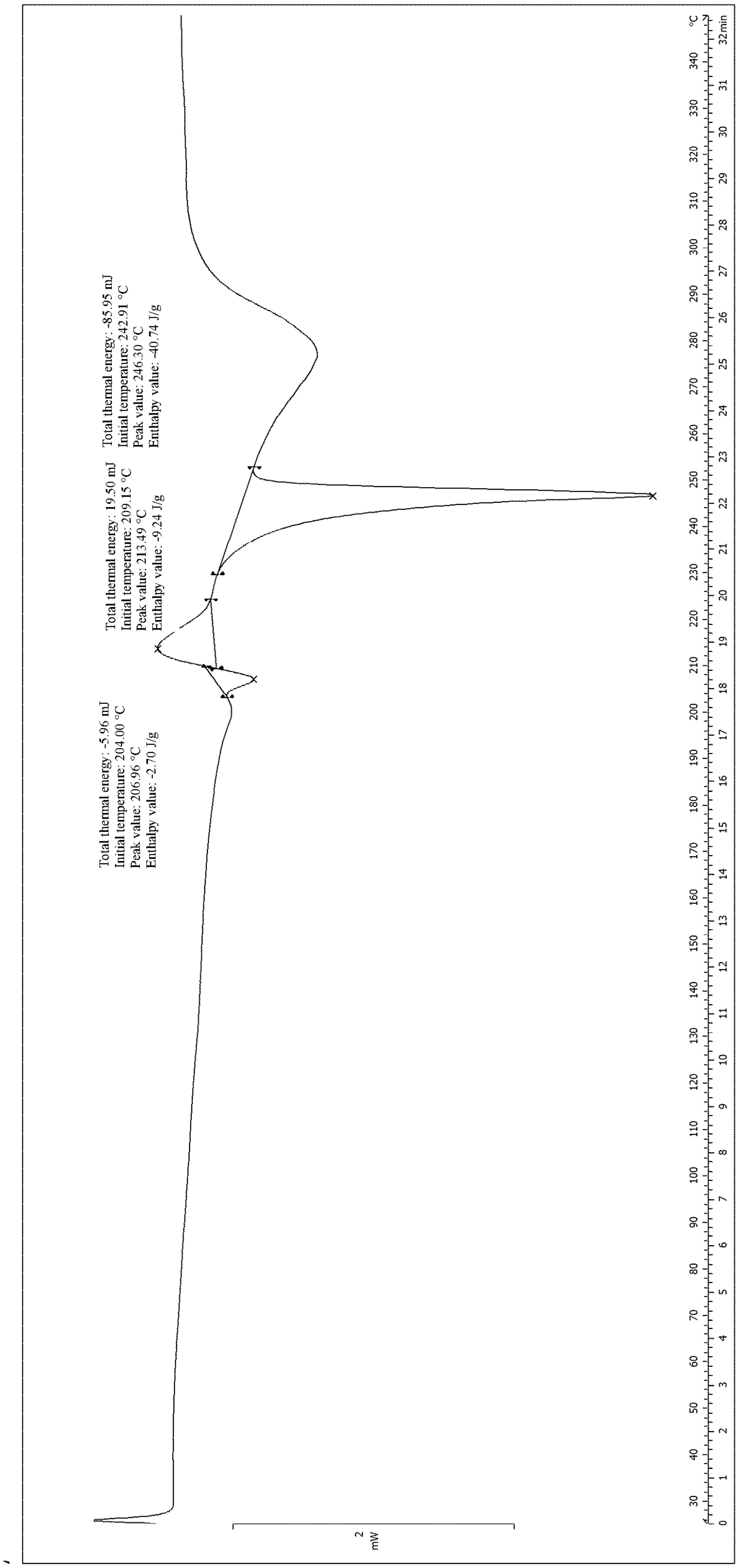
Total thermal energy: -5.96 mJ
Initial temperature: 204.00 °C
Peak value: 206.96 °C
Enthalpy value: -2.70 J/g
Total thermal energy: 19.50 mJ
Initial temperature: 209.15 °C
Peak value: 213.49 °C
Enthalpy value: -9.24 J/g
Total thermal energy: -85.95 mJ
Initial temperature: 242.91 °C
Peak value: 246.30 °C
Enthalpy value: -40.74 J/g
2 mW
°C
min

FIG. 36

**INTERNATIONAL SEARCH REPORT**

International application No.
**PCT/CN2020/125125**

**A. CLASSIFICATION OF SUBJECT MATTER**

C07D 235/04(2006.01)i; A61K 31/4184(2006.01)i

According to International Patent Classification (IPC) or to both national classification and IPC

**B. FIELDS SEARCHED**

Minimum documentation searched (classification system followed by classification symbols)

C07D; A61K

Documentation searched other than minimum documentation to the extent that such documents are included in the fields searched

Electronic data base consulted during the international search (name of data base and, where practicable, search terms used)

CNABS, CNTXT, SIPOABS, DWPI, CAPLUS(STN), REGISTRY(STN), CNKI, 百度学术: 恒瑞医药, 周先强, 杜振兴, 王捷, 王林, 二氟甲氧基, 苯并咪唑, 环丙基, 磺酰基, 酰胺, 盐, 溶剂化物, 苯甲酸, 草酸, 甲磺酸, 马来酸, 乙酸, 氢溴酸, 盐酸, 晶体, 晶型, 无定型, 类视黄醇相关的孤儿受体, Hengrui, difluoromethoxy, benzimidazole, cyclopropyl, sulfonyl, amide, salt, solvate, benzoic acid, oxalic acid, methanesulfonic acid, maleic acid, acetic acid, hydrobromic acid, hydrochloric acid, crystal, crystalline, amorphous, retinoid-related orphan receptors, ROR

**C. DOCUMENTS CONSIDERED TO BE RELEVANT**

| Category* | Citation of document, with indication, where appropriate, of the relevant passages | Relevant to claim No. |
|---|---|---|
| PX | WO 2019213470 A1 (ETERNITY BIOSCIENCE INC.) 07 November 2019 (2019-11-07)<br>page 50 lines 13-30, page 59 line 21- page 60 line 4 and embodiments 152 and 153 | 1-27 |
| E | CN 112135611 A (JIANGSU HENGRUI MEDICINE CO., LTD. et al.) 25 December 2020 (2020-12-25)<br>abstract, description paragraphs 0008, 0163, 0213-0215 and embodiments 152 and 153 | 1-2, 19, 22, 27 |
| A | CN 109071509 A (VITAE PHARMACEUTICALS, INC.) 21 December 2018 (2018-12-21)<br>abstract, claims 1-54, description paragraphs 0060, 0147-0172 and embodiments 1-20 | 1-27 |
| A | CN 109485595 A (JIANGSU HENGRUI MEDICINE CO., LTD. et al.) 19 March 2019 (2019-03-19)<br>entire document | 1-27 |
| A | CN 104603118 A (PHENEX PHARMACEUTICALS AG) 06 May 2015 (2015-05-06)<br>entire document | 1-27 |
| A | CN 105209453 A (JANSSEN PHARMACEUTICA NV) 30 December 2015 (2015-12-30)<br>entire document | 1-27 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 January 2021** | **28 January 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2020/125125**

**C. DOCUMENTS CONSIDERED TO BE RELEVANT**

| Category* | Citation of document, with indication, where appropriate, of the relevant passages | Relevant to claim No. |
|---|---|---|
| A | CN 105829316 A (MERCK PATENT GMBH) 03 August 2016 (2016-08-03)<br>entire document | 1-27 |

Form PCT/ISA/210 (second sheet) (January 2015)

**INTERNATIONAL SEARCH REPORT**
**Information on patent family members**

International application No.
**PCT/CN2020/125125**

| Patent document cited in search report | Publication date (day/month/year) | Patent family member(s) | Publication date (day/month/year) |
|---|---|---|---|
| WO 2019213470 A1 | 07 November 2019 | TW 202014185 A | 16 April 2020 |
| CN 112135611 A | 25 December 2020 | None | |
| CN 109071509 A | 21 December 2018 | TW 201734001 A | 01 October 2017 |
| | | US 2019352286 A1 | 21 November 2019 |
| | | JP 2019503385 A | 07 February 2019 |
| | | AU 2017212577 A1 | 02 August 2018 |
| | | RU 2018127360 A3 | 06 April 2020 |
| | | BR 112018015273 A2 | 18 December 2018 |
| | | UY 37098 A | 31 August 2017 |
| | | MX 2018009257 A | 09 November 2018 |
| | | CA 3011838 A1 | 03 August 2017 |
| | | KR 20180100697 A | 11 September 2018 |
| | | WO 2017132432 A8 | 31 August 2017 |
| | | EP 3408268 A1 | 05 December 2018 |
| | | RU 2018127360 A | 02 March 2020 |
| | | WO 2017132432 A1 | 03 August 2017 |
| CN 109485595 A | 19 March 2019 | None | |
| CN 104603118 A | 06 May 2015 | PE 20150229 A1 | 02 March 2015 |
| | | CO 7160113 A2 | 15 January 2015 |
| | | EA 201491943 A1 | 30 July 2015 |
| | | CR 20150003 A | 23 April 2015 |
| | | JP 2015521193 A | 27 July 2015 |
| | | TW I511960 B | 11 December 2015 |
| | | TW 201400465 A | 01 January 2014 |
| | | HK 1209117 A1 | 24 March 2016 |
| | | EP 2855440 A1 | 08 April 2015 |
| | | MX 2014014614 A | 12 February 2015 |
| | | UA 117913 C2 | 25 October 2018 |
| | | GT 201400275 A | 24 August 2017 |
| | | AU 2013270036 B2 | 01 December 2016 |
| | | KR 20150015031 A | 09 February 2015 |
| | | AR 091194 A1 | 21 January 2015 |
| | | SG 11201407919 W A | 30 December 2014 |
| | | BR 112014029868 A2 | 27 June 2017 |
| | | KR 101742954 B1 | 02 June 2017 |
| | | WO 2013178362 A1 | 05 December 2013 |
| | | EA 028330 B1 | 30 November 2017 |
| | | NZ 702539 A | 25 November 2016 |
| | | CN 104603118 B | 03 October 2017 |
| | | JP 6225178 B2 | 01 November 2017 |
| | | NO 20141395 A | 30 December 2014 |
| | | NO 20141395 A1 | 30 December 2014 |
| | | IL 235626 A | 29 November 2018 |
| | | EA 201791271 A1 | 31 January 2018 |
| | | CA 2873877 A1 | 05 December 2013 |
| | | CN 107007597 A | 04 August 2017 |
| | | AU 2013270036 A1 | 27 November 2014 |
| | | PH 12014502570 A1 | 21 January 2015 |
| | | CA 2873877 C | 07 August 2018 |
| | | US 10301272 B2 | 28 May 2019 |

Form PCT/ISA/210 (patent family annex) (January 2015)

**INTERNATIONAL SEARCH REPORT**
**Information on patent family members**

International application No.
**PCT/CN2020/125125**

| Patent document cited in search report | Publication date (day/month/year) | Patent family member(s) | Publication date (day/month/year) |
|---|---|---|---|
| | | US 2015175562 A1 | 25 June 2015 |
| | | IL 235626 D0 | 01 February 2015 |
| | | CL 2014003277 A1 | 10 July 2015 |
| | | UY 34832 A | 31 December 2013 |
| CN 105209453 A | 30 December 2015 | AU 2013331496 B2 | 27 July 2017 |
| | | IL 237866 A | 31 May 2018 |
| | | JP 2015536320 A | 21 December 2015 |
| | | HU E035335 T2 | 02 May 2018 |
| | | AR 093017 A1 | 13 May 2015 |
| | | NZ 706775 A | 26 October 2018 |
| | | RS 56283 B1 | 29 December 2017 |
| | | PT 2909192 T | 04 August 2017 |
| | | US 2014107094 A1 | 17 April 2014 |
| | | TW 201427969 A | 16 July 2014 |
| | | GT 201500093 A | 26 September 2017 |
| | | ES 2632269 T3 | 12 September 2017 |
| | | CL 2015000945 A1 | 28 August 2015 |
| | | PE 20150778 A1 | 23 May 2015 |
| | | CA 2888210 A1 | 24 April 2014 |
| | | PH 12015500817 A1 | 08 June 2015 |
| | | NI 201500053 A | 18 March 2020 |
| | | MX 358508 B | 22 August 2018 |
| | | SI 2909192 T1 | 31 August 2017 |
| | | EA 201590736 A1 | 30 September 2015 |
| | | EA 026415 B1 | 28 April 2017 |
| | | EP 2909192 A1 | 26 August 2015 |
| | | CR 20150193 A | 18 May 2015 |
| | | US 9290476 B2 | 22 March 2016 |
| | | LT 2909192 T | 10 July 2017 |
| | | WO 2014062658 A1 | 24 April 2014 |
| | | AU 2013331496 A1 | 09 April 2015 |
| | | CN 105209453 B | 20 June 2017 |
| | | HK 1213248 A1 | 30 June 2016 |
| | | HR P20171082 T1 | 06 October 2017 |
| | | PH 12015500817 B1 | 08 June 2015 |
| | | SG 11201502369 X A | 28 May 2015 |
| | | PL 2909192 T3 | 29 September 2017 |
| | | KR 20150070347 A | 24 June 2015 |
| | | JP 6466335 B2 | 06 February 2019 |
| | | TW I606045 B | 21 November 2017 |
| | | MX 2015004783 A | 14 August 2015 |
| | | CY 1119234 T1 | 14 February 2018 |
| | | BR 112015008308 A2 | 05 December 2017 |
| | | DK 2909192 T3 | 07 August 2017 |
| | | ME 02794 B | 20 January 2018 |
| | | EP 2909192 B1 | 17 May 2017 |
| | | ZA 201503418 B | 29 November 2017 |
| | | UY 35084 A | 30 April 2014 |
| | | WO 2014062658 A8 | 10 July 2014 |
| CN 105829316 A | 03 August 2016 | AU 2014365915 B2 | 09 August 2018 |

Form PCT/ISA/210 (patent family annex) (January 2015)

**INTERNATIONAL SEARCH REPORT**
**Information on patent family members**

International application No.
**PCT/CN2020/125125**

| Patent document cited in search report | Publication date (day/month/year) | Patent family member(s) | | | Publication date (day/month/year) |
|---|---|---|---|---|---|
| | | US | 2016311830 | A1 | 27 October 2016 |
| | | AU | 2014365915 | A1 | 28 July 2016 |
| | | EP | 3083624 | B1 | 20 December 2017 |
| | | ES | 2663498 | T3 | 13 April 2018 |
| | | AU | 2014365915 | C1 | 04 April 2019 |
| | | JP | 6463362 | B2 | 30 January 2019 |
| | | IL | 246297 | D0 | 02 August 2016 |
| | | US | 9688684 | B2 | 27 June 2017 |
| | | JP | 2016540031 | A | 22 December 2016 |
| | | EP | 3083624 | A1 | 26 October 2016 |
| | | WO | 2015090507 | A1 | 25 June 2015 |
| | | CN | 105829316 | B | 08 January 2019 |
| | | CA | 2934378 | A1 | 25 June 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201911049930
- WO 2012027965 A
- WO 2013029338 A
- US 2015291607 A
- US 1930526 W


### Non-patent literature cited in the description


- **BENOIT G et al.** *Pharmacological Reviews,* 2006, vol. 58 (4), 798-836
- **ZHANG, Y et al.** *Acta Pharmacogica Sinica,* 2015, vol. 36, 71-87
- **VILLEY I et al.** *Eur. J. Immunol.,* 1999, vol. 29 (12), 4072-80
- **LOUTEN et al.** *J. Allergy Clin. Immunol.,* 2009, vol. 123, 1004-1011
- **ANNUZIATO, F. et al.** *Nat. Rev. Rheumatol.,* 2009, vol. 5 (6), 325-331
- **LIZUKA, M. et al.** *J. Immunol.,* 2014, vol. 194, 56-67
- **CHEN, Y. et al.** *Mucosal. Immunol.,* 2014, vol. 7 (1), 38-45
- **JIA, S. et al.** *Exp. Immunol.,* 2010, vol. 162, 131-137
- **BOAVENTURA, VS et al.** *Eur. J. Immunol.,* 2010, vol. 40, 2830-2836
- **FIGUEROA-VEGA, N. et al.** *J. Clin. Endocrinol. Metab.,* 2010, vol. 95, 953-62
- **JUN R. HUH ; DAN R. LITTMAN.** *Eur. J. Immunol.,* 2012, vol. 42 (9), 2232-2237